(19) Europäisches Patentamt
European Patent Office
Office européen des brevets

(11) EP 3 314 265 B1

(12) **EUROPEAN PATENT SPECIFICATION**

(45) Date of publication and mention of the grant of the patent:
**24.07.2019 Bulletin 2019/30**

(21) Application number: **16733928.2**

(22) Date of filing: **28.06.2016**

(51) Int Cl.:
***G01N 33/574*** (2006.01) ***A61K 31/553*** (2006.01)
***C12Q 1/68*** (2018.01)

(86) International application number:
**PCT/EP2016/064920**

(87) International publication number:
**WO 2017/001362 (05.01.2017 Gazette 2017/01)**

# (54) METHODS OF TREATMENT WITH TASELISIB

VERFAHREN ZUR BEHANDLUNG MIT TASELISIB

MÉTHODES DE TRAITEMENT AVEC DU TASÉLISIB

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2015 US 201562186236 P**

(43) Date of publication of application:
**02.05.2018 Bulletin 2018/18**

(73) Proprietor: **F. Hoffmann-La Roche AG**
**4070 Basel (CH)**

(72) Inventors:
- **EDGAR, Kyle**
  **South San Francisco, California 94080 (US)**
- **FRIEDMAN, Lori**
  **South San Francisco, California 94080 (US)**
- **SAMPATH, Deepak**
  **South San Francisco, California 94080 (US)**
- **SONG, Kyung**
  **South San Francisco, California 94080 (US)**
- **WERTZ, Ingrid**
  **South San Francisco, California 94080 (US)**
- **WILSON, Timothy**
  **South San Francisco, California 94080 (US)**

(74) Representative: **Sauer, Frank**
**F. Hoffmann-La Roche AG**
**Patent Department**
**Grenzacherstrasse 124**
**4070 Basel (CH)**

(56) References cited:
**WO-A1-2013/182668**

- **KYLE A EDGAR ET AL: "Characterization of the enhanced potency of PI3K inhibitor taselisib (GDC-0032) in PI3K mutant cell lines and models", AACR 106TH ANNUAL MEETING 2015, 20 April 2015 (2015-04-20), XP055297624,**
- **E De Azambuja ET AL: "LORELEI: A Phase II Randomized, Double-Blind Study of Neoadjuvant Letrozole Plus Taselisib (GDC-0032) Versus Letrozole Plus Placebo in Postmenopausal Women with ER+/HER2-Early Stage Breast Cancer", , 19 May 2015 (2015-05-19), XP055297591, Retrieved from the Internet: URL:http://qfuse.com/client_downloads/ASCO 2015_deAzambuja_LORELEI_FINAL.pdf [retrieved on 2016-08-25]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

Sequence Listing

<110> Edgar, Kyle
Friedman, Lori
Sampath, Deepak
Song, Kyung
Wertz, Ingrid
Wilson, Timothy

<120> METHODS OF TREATMENT WITH TASELISIB

<130> P32910-US-1

<141> 2016-06-28

<150> US 62/186,236
<151> 2015-06-29

<160> 20

<210> 1
<211> 17
<212> DNA
<213> Artificial sequence

<220>
<223> sequence is synthesized

<400> 1
atgatgcaca tcatggt 17

<210> 2
<211> 24
<212> DNA
<213> Artificial sequence

<220>
<223> sequence is synthesized

<400> 2
ggctttggag tatttcatga aaca 24

<210> 3
<211> 25
<212> DNA
<213> Artificial sequence

<220>
<223> sequence is synthesized

<400> 3
gaagatccaa tccatttttg ttgtc 25

<210> 4
<211> 16
<212> DNA
<213> Artificial sequence

<220>
<223> sequence is synthesized

<400> 4
tgatgcacgt catggt 16

<210> 5
<211> 24
<212> DNA
<213> Artificial sequence

<220>
<223> sequence is synthesized

<400> 5
ggctttggag tatttcatga aaca 24

<210> 6
<211> 25
<212> DNA
<213> Artificial sequence

<220>
<223> sequence is synthesized

<400> 6
gaagatccaa tccatttttg ttgtc 25

<210> 7
<211> 13
<212> PRT
<213> Artificial sequence

<220>
<223> sequence is synthesized

<400> 7

```
Gln Met Asn Asp Ala His His Gly Gly Trp Thr Thr Lys
                5                   10
```

<210> 8

<211> 7
<212> PRT
<213> Artificial sequence

<220>
<223> sequence is synthesized

<400> 8

His Gly Gly Trp Thr Thr Lys
5

<210> 9
<211> 8
<212> PRT
<213> Artificial sequence

<220>
<223> sequence is synthesized

<400> 9

Glu Ala Thr Leu Ile Thr Ile Lys
5

<210> 10
<211> 8
<212> PRT
<213> Artificial sequence

<220>
<223> sequence is synthesized

<400> 10

Asp Leu Asn Ser Pro His Ser Arg
5

<210> 11
<211> 9
<212> PRT
<213> Artificial sequence

<220>
<223> sequence is synthesized

<400> 11

Leu Cys Val Leu Glu Tyr Gln Gly Lys
5

<210> 12
<211> 11
<212> PRT
<213> Artificial sequence

<220>
<223> sequence is synthesized

<400> 12

Val Cys Gly Cys Asp Glu Tyr Phe Leu Glu Lys
5 10

<210> 13
<211> 7
<212> PRT
<213> Artificial sequence

<220>
<223> sequence is synthesized

<400> 13

Val Pro Cys Ser Asn Pro Arg
5

<210> 14
<211> 14
<212> PRT
<213> Artificial sequence

<220>
<223> sequence is synthesized

<400> 14

Glu Ala Gly Phe Ser Tyr Ser His Ala Gly Leu Ser Asn Arg
5 10

<210> 15
<211> 11
<212> PRT
<213> Artificial sequence

<220>
<223> sequence is synthesized

<400> 15

Tyr Glu Gln Tyr Leu Asp Asn Leu Leu Val Arg
5 10

<210> 16
<211> 10
<212> PRT
<213> Artificial sequence

<220>
<223> sequence is synthesized

<400> 16

Phe Gly Leu Leu Leu Glu Ser Tyr Cys Arg
5 10

<210> 17

<211> 9
<212> PRT
<213> Artificial sequence

<220>
<223> sequence is synthesized

<400> 17

```
Leu Ile Asn Leu Thr Asp Ile Leu Lys
                5
```

<210> 18
<211> 6
<212> PRT
<213> Artificial sequence

<220>
<223> sequence is synthesized

<400> 18

```
Gln Met Asn Asp Ala Arg
                5
```

<210> 19
<211> 11
<212> PRT
<213> Artificial sequence

<220>
<223> sequence is synthesized

<400> 19

```
Asp Pro Leu Ser Glu Ile Thr Glu Gln Glu Lys
                5                   10
```

<210> 20
<211> 8
<212> PRT
<213> Artificial sequence

<220>
<223> sequence is synthesized

<400> 20

```
Asp Pro Leu Ser Glu Ile Thr Lys
                5
```

## Description

CROSS REFERENCE TO RELATED APPLICATIONS

This non-provisional application filed under 37 CFR §1.53(b), claims the benefit under 35 USC §119(e) of US Provisional Application Serial No. 62/186,236 filed on 29 June 2015.

FIELD OF THE INVENTION

The invention relates generally to treatment of cancer with PI3K inhibitor compound, taselisib (GDC-0032). The invention also relates to methods of using taselisib for *in vitro, in situ,* and *in vivo* diagnosis or treatment of mammalian cells, or associated pathological conditions.

BACKGROUND OF THE INVENTION

Upregulation of the phosphoinositide 3-kinase (PI3K)/Akt signaling pathway is a common feature in most cancers (Yuan and Cantley (2008) Oncogene 27:5497-510). Genetic deviations in the pathway have been detected in many human cancers (Osaka et al (2004) Apoptosis 9:667-76) and act primarily to stimulate cell proliferation, migration and survival. Activation of the pathway occurs following activating point mutations or amplifications of the *PIK3CA* gene encoding the p110a PI3K isoforms (Samuels et al (2004) Science 304:554; Hennessy et al (2005) Nat. Rev. Drug Discov. 4:988-1004). Genetic deletion or loss of function mutations within the tumor suppressor PTEN, a phosphatase with opposing function to PI3K, also increases PI3K pathway signaling (Zhang and Yu (2010) Clin. Cancer Res. 16:4325-30).

These aberrations lead to increased downstream signaling through kinases such as Akt and mTOR and increased activity of the PI3K pathway has been proposed as a hallmark of resistance to cancer treatment (Opel et al (2007) Cancer Res. 67:735-45; Razis et al (2011) Breast Cancer Res. Treat. 128:447-56).

The phosphatidylinositol 3-kinase (PI3K) signaling pathway is one of the most dysregulated pathways in hormone receptor (HR)-positive metastatic breast cancer (mBC) (Bachman KE, et al. Cancer Biol Ther. 2004; 3:772-775; Stemke-Hale K, et al. Cancer Res. 2008; 68:6084-6091; Koboldt DC, et al. Nature 2012; 490:61-70). Phosphatidylinositol-4,5-bisphosphate 3-kinase, catalytic subunit alpha (*PIK3CA*) encodes the PI3Kα isoform (p110) of the PI3K catalytic subunit,(Samuels Y, et al. Science 2004; 304:554) with mutations in this gene detected in ~40% of HR-positive BC (Arthur LM, et al. Breast Cancer Res Treat. 2014; 147:211).

Phosphatidylinositide 3-Kinase (PI3K) is a major signaling node for key survival and growth signals for lymphomas and is opposed by the activity of the phosphatase PTEN. The PI3K pathway is dysregulated in aggressive forms of lymphoma (Abubaker (2007) Leukemia 21:2368-2370). Eight percent of DLBCL (diffuse large B-cell lymphoma) cancers have PI3KCA (phosphatidylinositol-3 kinase catalytic subunit alpha) missense mutations and 37% are PTEN negative by immunohistochemistry test.

Phosphatidylinositol is one of a number of phospholipids found in cell membranes, and which participate in intracellular signal transduction. Cell signaling via 3'-phosphorylated phosphoinositides has been implicated in a variety of cellular processes, e.g., malignant transformation, growth factor signaling, inflammation, and immunity (Rameh et al (1999) J. Biol Chem. 274:8347-8350). The enzyme responsible for generating these phosphorylated signaling products, phosphatidylinositol 3-kinase (also referred to as PI 3-kinase or PI3K), was originally identified as an activity associated with viral oncoproteins and growth factor receptor tyrosine kinases that phosphorylate phosphatidylinositol (PI) and its phosphorylated derivatives at the 3'-hydroxyl of the inositol ring (Panayotou et al (1992) Trends Cell Biol 2:358-60). Phosphoinositide 3-kinases (PI3K) are lipid kinases that phosphorylate lipids at the 3-hydroxyl residue of an inositol ring (Whitman et al (1988) Nature, 332:664). The 3-phosphorylated phospholipids (PIP3s) generated by PI3-kinases act as second messengers recruiting kinases with lipid binding domains (including plekstrin homology (PH) regions), such as Akt and PDK1, phosphoinositide-dependent kinase-1 (Vivanco et al (2002) Nature Rev. Cancer 2:489; Phillips et al (1998) Cancer 83:41).

The PI3 kinase family comprises at least 15 different enzymes sub-classified by structural homology and are divided into 3 classes based on sequence homology and the product formed by enzyme catalysis. The class I PI3 kinases are composed of 2 subunits: a 110 kd catalytic subunit and an 85 kd regulatory subunit. The regulatory subunits contain SH2 domains and bind to tyrosine residues phosphorylated by growth factor receptors with a tyrosine kinase activity or oncogene products, thereby inducing the PI3K activity of the p110 catalytic subunit which phosphorylates its lipid substrate. Class I PI3 kinases are involved in important signal transduction events downstream of cytokines, integrins, growth factors and immunoreceptors, which suggests that control of this pathway may lead to important therapeutic effects such as modulating cell proliferation and carcinogenesis. Class I PI3Ks can phosphorylate phosphatidylinositol (PI), phosphatidylinositol-4-phosphate, and phosphatidylinositol-4,5-biphosphate (PIP2) to produce phosphatidylinositol-3-phosphate (PIP), phosphatidylinositol-3,4-biphosphate, and phosphatidylinositol-3,4,5-triphosphate, respectively.

Class II PI3Ks phosphorylate PI and phosphatidylinositol-4-phosphate. Class III PI3Ks can only phosphorylate PI. A key PI3-kinase isoform in cancer is the Class I PI3-kinase, p110α as indicated by recurrent oncogenic mutations in p110α (Samuels et al (2004) Science 304:554; US 5824492; US 5846824; US 6274327). Other isoforms may be important in cancer and are also implicated in cardiovascular and immune-inflammatory disease (Workman P (2004) Biochem Soc Trans 32:393-396; Patel et al (2004) Proc. Am. Assoc. of Cancer Res. (Abstract LB-247) 95th Annual Meeting, March 27-31, Orlando, Florida, USA; Ahmadi K and Waterfield MD (2004) "Phosphoinositide 3-Kinase: Function and Mechanisms" Encyclopedia of Biological Chemistry (Lennarz W J, Lane M D eds) Elsevier/Academic Press).

After Ras, PI3K is the second most mutated oncogene in cancer. Oncogenic mutations of p110 alpha have been found at a significant frequency in colon, breast, brain, liver, ovarian, gastric, lung, and head and neck solid tumors. About 35-40% of hormone receptor positive (HR+) breast cancer tumors harbor a *PIK3CA* mutation. PTEN abnormalities are also found in glioblastoma, melanoma, prostate, endometrial, ovarian, breast, lung, head and neck, hepatocellular, and thyroid cancers. Phosphatase and tensin homolog (PTEN) is a protein that, in humans, is encoded by the PTEN gene (Steck PA, et al (1997) Nat. Genet. 15 (4): 356-62). Mutations of this gene are a step in the development of many cancers. PTEN acts as a tumor suppressor gene through the action of its phosphatase protein product. This phosphatase is involved in the regulation of the cell cycle, preventing cells from growing and dividing too rapidly (Chu EC, et al (2004) Med. Sci. Monit. 10 (10): RA235-41).

PI3 kinase is a heterodimer consisting of p85 and p110 subunits (Otsu et al (1991) Cell 65:91-104; Hiles et al (1992) Cell 70:419-29). Four distinct Class I PI3K isoforms have been identified, designated PI3K α (alpha), β (beta), δ (delta), and γ (gamma), each consisting of a distinct 110 kDa catalytic subunit and a regulatory subunit. Three of the catalytic subunits, i.e., p110 alpha, p110 beta and p110 delta, each interact with the same regulatory subunit, p85; whereas p110 gamma interacts with a distinct regulatory subunit, p101. The patterns of expression of each of these PI3Ks in human cells and tissues are distinct. In each of the PI3K alpha, beta, and delta isoform subtypes, the p85 subunit acts to localize PI3 kinase to the plasma membrane by the interaction of its SH2 domain with phosphorylated tyrosine residues (present in an appropriate sequence context) in target proteins (Rameh et al (1995) Cell, 83:821-30; Volinia et al (1992) Oncogene, 7:789-93).

Measuring levels of biomarkers (e.g., expression levels or functional protein levels of secreted proteins in plasma) can be an effective means to identify patients and patient populations that will respond to specific therapies including, e.g., treatment with chemotherapeutic agents. There is a need for more effective means for determining which patients with hyperproliferative disorders such as cancer will respond to which treatment with chemotherapeutic agents, and for incorporating such determinations into more effective treatment regimens for patients, whether the chemotherapeutic agents are used as single agents or combined with other agents.

The phosphoinositide 3-kinase (PI3K) signaling cascade, a key mediator of cellular survival, growth, and metabolism, is frequently altered in human cancer. Activating mutations in *PIK3CA,* the gene which encodes the α-catalytic subunit of PI3K, occur in approximately 30% of breast cancers. These mutations result in constitutive activity of the enzyme and are oncogenic. Expression of mutant *PIK3CA* H1047R in the luminal mammary epithelium evokes heterogeneous tumors that express luminal and basal markers and are positive for the estrogen receptor. The *PIK3CA* H1047R oncogene targets a multipotent progenitor cells and recapitulates features of human breast tumors with *PIK3CA* H1047R (Meyer et al (2011). Cancer Res; 71(13):4344-51). Hyperactivation of PI3K can occur as a result of somatic mutations in *PIK3CA,* the gene encoding the p110α subunit of PI3K. The *HER2* oncogene is amplified in 25% of all breast cancers and some of these tumors also harbor *PIK3CA* mutations. PI3K can enhance transformation and confer resistance to HER2-directed therapies. PI3K mutations E545K and H1047R introduced in MCF10A human mammary epithelial cells that also overexpress HER2 conferred a gain of function to MCF10A/HER2 cells. Expression of H1047R PI3K but not E545K PI3K markedly upregulated the HER3/HER4 ligand heregulin (HRG) (Chakrabarty et al (2010) Oncogene 29(37):5193-5203).

The PI3 kinase/Akt/PTEN pathway is an attractive target for cancer drug development since such agents would be expected to inhibit cellular proliferation, repress signals from stromal cells that provide for survival and chemoresistance of cancer cells, reverse the repression of apoptosis, and surmount intrinsic resistance of cancer cells to cytotoxic agents. Certain thienopyrimidine compounds have p110 alpha binding, PI3 kinase inhibitory activity, and inhibit the growth of cancer cells (Wallin et al (2011) Mol. Can. Ther. 10(12):2426-2436; Sutherlin et al (2011) Jour. Med. Chem. 54:7579-7587; US 2008/0207611; US 7846929; US 7781433; US 2008/0076758; US 7888352; US 2008/0269210. GDC-0941 (pictilisib, CAS Reg. No. 957054-30-7, Genentech Inc.), is a selective, orally bioavailable inhibitor of PI3K with promising pharmacokinetic and pharmaceutical properties (Folkes et al (2008) Jour. of Med. Chem. 51(18):5522-5532; US 7781433; US 8324206; Belvin et al, American Association for Cancer Research Annual Meeting 2008, 99th:April 15, Abstract 4004; Folkes et al, American Association for Cancer Research Annual Meeting 2008, 99th:April 14, Abstract LB-146; Friedman et al, American Association for Cancer Research Annual Meeting 2008, 99th:April 14, Abstract LB-110; Wallin et al (2012) Clin. Cancer Res.18:3901-3911; Yuan et al (2013) Oncogene 32:318-326; O'Brien et al (2010) Clin Cancer Res. 16:3670-3683; Salphati et al (2010) Drug Metab. and Disp. 38(9):1436-1442; Edgar et al (2010) Cancer Res. 70:1164-1172) and shows synergistic activity *in vitro* and *in vivo* in combination with certain chemotherapeutic agents

against solid tumor cell lines (US 8247397; US 8604014; US 8536161).

Taselisib (GDC-0032, Genentech Inc., Roche RG7604, CAS Reg. No. 1282512-48-4), named as 2-(4-(2-(1-isopropyl-3-methyl-1H-1,2,4-triazol-5-yl)-5,6-dihydrobenzo[f]imidazo[1,2-d][1,4]oxazepin-9-yl)-1H-pyrazol-1-yl)-2-methylpropanamide, is a selective, potent, orally bioavailable inhibitor of PI3K alpha ($\alpha$) with a Ki = 0.2 nM, and with reduced inhibitory activity against PI3K beta ($\beta$) (Ndubaku et al (2013) Jour. Med. Chem. 56(11):4597-4610; Staben et al (2013) Bioorg. Med. Chem. Lett. 23 2606-2613; WO 2011/036280; US 8242104; US 8343955; US 8586574; US 2014/0044706). Taselisib is being studied in patients with locally advanced or metastatic solid tumors. This selectivity profile, and excellent pharmacokinetic and pharmaceutical properties, allowed for greater efficacy *in vivo* at the maximum tolerated dose relative to the pan Class I PI3K inhibitor, GDC-0941 (Genentech Inc., pictilisib) in PIK3CA mutant xenografts. Mutations in the phosphoinositide-3 kinase alpha isoform (PIK3CA) are frequent in breast cancer and activate the PI3K signaling pathway (Kang et al (2005) Cell Cycle 4(4):578-581. Mutations increase lipid binding where helical mutations activate by weakening inhibitory interactions with the p85 subunit (Zhao and Vogt (2008) Oncogene 27(41):5486-5496). Kinase domain mutations activate by changing protein conformation (Burke et al (2012) Proc. Natl. Acad. Sci. 109(38):15259-15264. Notably, GDC-0032 preferentially inhibits PIK3CA mutant cells relative to cells with wild-type PI3K. GDC-0032 potently inhibits signal transduction downstream of PI3K and induces apoptosis at low concentrations in breast cancer cell lines and xenograft models that harbor PIK3CA mutations. The mutant-bias of GDC-0032 is linked to unique properties of GDC-0032, including cellular potency against the mutant isoform and reduction of receptor tyrosine kinase (RTK) signaling.

Taselisib is a potent and selective inhibitor of class I PI3K$\alpha$, -$\delta$, and -$\gamma$ isoforms, and displays greater selectivity for mutant PI3K$\alpha$ isoforms than wild-type PI3K$\alpha$ (Olivero A, et al. American Association for Cancer Research annual meeting, Washington, DC, USA, April 6-10, 2013; Wallin J, et al. 36th San Antonio Breast Cancer Symposium, San Antonio, TX, USA, December 10-14, 2013). In PIK3CA-mutant breast cancer (BC) models, taselisib enhanced the efficacy of standard-of-care therapeutics, including the ER antagonist fulvestrant (Sampath D, et al, 36th San Antonio Breast Cancer Symposium (SABCS), San Antonio, TX, USA, Dec. 10-14, 2013). PIK3CA mutations are one of the most frequent genomic alterations in breast cancer (BC), being present in about 40% of estrogen receptor (ER)-positive, HER2-negative breast tumors. PIK3CA mutations promote growth and proliferation of tumors and mediate resistance to endocrine therapies in BC. Taselisib displays greater selectivity for mutant PI3K$\alpha$ than wild-type PI3K$\alpha$ (alpha). Taselisib has enhanced activity against PIK3CA-mutant breast cancer cell lines, and clinical data include confirmed partial responses in patients with PIK3CA-mutant BC treated with taselisib either as a single agent or in combination with fulvestrant.

## SUMMARY OF THE INVENTION

Taselisib (GDC-0032, Genentech Inc., Roche RG7604, CAS Reg. No. 1282512-48-4), named as 2-(4-(2-(1-isopropyl-3-methyl-1H-1,2,4-triazol-5-yl)-5,6-dihydrobenzo[f]imidazo[1,2-d][1,4]oxazepin-9-yl)-1H-pyrazol-1-yl)-2-methylpropanamide), induces the degradation of mutant-p110 alpha protein. The ability of PI3K inhibitors to degrade p110 is correlated with the clinical response. Depletion of p110 alpha protein may identify a patient who will respond to treatment with taselisib.

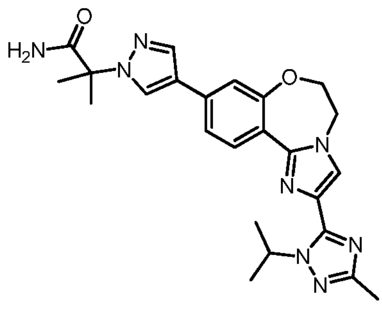

GDC-0032 (taselisib)

Taselisib specifically promotes the degradation of the mutant p110alpha subunit in a dose, time, ubiquitin and proteasome-dependent manner. This effect observed for all p110 mutations tested to date may be mediated through the destabilization of p110-p85 interaction.

Degradation of mutant, but not wildtype PI3K protein appears to prevent RTK-driven PI3K pathway reactivation, possibly enabling a wider therapeutic window for the use of PI3K inhibitors.

An aspect of the invention is a method of selecting a patient for treatment with taselisib comprising:

(a) treating a biological sample obtained from the patient with taselisib; and

(b) detecting the depletion of p110 alpha protein;

wherein the depletion of p110 alpha protein in the biological sample identifies a patient who will respond to treatment with taselisib. Depletion of p110 alpha protein indicates therapeutic responsiveness by a patient to the compound, and may be measured by binding to an anti-pl 10 alpha antibody. Binding of the anti-pl 10 alpha antibody to the p110 alpha protein in the sample is determined by western blot analysis, enzyme-linked immunosorbent assay (ELISA), radioimmunoassay (RIA), immunohistochemistry (IHC), fluorescence-activated cell sorting (FACS), or reverse-phase protein array.

An aspect is a method of treating a patient comprising:

(a) testing a biological sample obtained from the patient for PIK3CA mutation status, wherein the PIK3CA mutation status comprises a mutation selected from H1047R, C420R, H1047L, E542K, E545K and Q546R;

(b) contacting the biological sample from a patient with a PIK3CA mutation with taselisib and detecting depletion of p110 alpha isoform; and

(c) administering taselisib to the patient with a PIK3CA mutation. The biological sample may be a circulating tumor cell. In combination with taselisib, the patient may be administered a chemotherapeutic agent selected from 5-FU, docetaxel, eribulin, gemcitabine, GDC-0973, GDC-0623, paclitaxel, tamoxifen, fulvestrant, dexamethasone, pertuzumab, trastuzumab emtansine, trastuzumab and letrozole. The patient may have a HER2 expressing breast cancer or estrogen receptor positive (ER+) breast cancer. The cancer may be metastatic. Taselisib may be administered to a patient in an adjuvant setting.

An aspect of the invention is a method of selecting patients with a PIK3CA mutation for treatment with taselisib comprising:

(a) detecting a PIK3CA mutation in a biological sample obtained from the patient; and

(b) comparing the level of p110 alpha in a biological sample obtained from the patient prior to administration of taselisib with the level of p110 alpha in the biological sample obtained from the patient after administration of taselisib,

wherein a depletion in the level of p110 alpha in the biological sample obtained from the patient after administration of taselisib identifies a patient who will respond to treatment with taselisib.

An aspect is a method of treating cancer comprising:

(a) comparing the level of p110 alpha in a biological sample obtained from a patient with cancer prior to administration of taselisib with the level of p110 alpha in a biological sample obtained from the patient after administration of taselisib, and
(b) altering the dosage, the frequency of dosing, or the course of taselisib therapy administered to the patient.

An aspect of the invention is a method of monitoring therapeutic efficacy in patients with cancer comprising:

(a) administering taselisib to the patient;
(b) measuring p110 alpha in a biological sample obtained from the patient after administration of taselisib; and
(c) altering the dosage, the frequency of dosing, or the course of taselisib therapy administered to the patient.

An aspect of the invention is an in vitro method of selecting a treatment regimen for a patient diagnosed as having cancer, the method comprising contacting a cancer cell of the patient with an effective amount of taselisib, and detecting the level of p110 alpha in response to taselisib, wherein detection of depletion of p110 alpha indicates that the cancer is susceptible to treatment with taselisib, and wherein the treatment regimen comprises administering taselisib to the patient if the cancer is determined to be susceptible to treatment with taselisib.

The cancer cell may be a PIK3CA mutant cancer cell.

An aspect is a method of treating cancer comprising:

a) administering taselisib to a patient;
b) measuring a change in the level of p110 alpha or a biomarker correlated to the level of p110 alpha in a biological sample obtained from the patient; and
c) selecting a dosage, frequency of dosing, or the course of taselisib therapy to be administered to the patient which

shows depletion of p110 alpha in a biological sample obtained from the patient. The change in the level of p110 alpha may be depletion in the level of p110 alpha.

An aspect is a method of identifying a biomarker for monitoring responsiveness to taselisib in the treatment of cancer, the method comprising:

(a) detecting the expression, modulation, or activity of a biomarker correlated to the level of p110 alpha in a biological sample obtained from a patient who has received at least one dose of taselisib; and
(b) comparing the expression, modulation, or activity of the biomarker to the status of the biomarker in a reference sample wherein the reference sample is a biological sample obtained from the patient prior to administration of taselisib;

wherein the modulation of the biomarker changes by at least 2 fold lower or higher compared to the reference sample is identified as a biomarker useful for monitoring responsiveness to taselisib. The cancer may be HER2 expressing breast cancer.

An aspect is a method of treating cancer in a patient, comprising administering a therapeutically effective amount of taselisib to the patient, wherein treatment is based detecting a biomarker correlated to the level of p110 alpha in a biological sample obtained from the patient. The biological sample may be a tumor biopsy sample or a circulating tumor cell.

BRIEF DESCRIPTION OF THE DRAWINGS

Figure 1 shows Western blot analysis of p110a protein degradation by taselisib is dose-dependent, specific to PI3Ka mutant cells. This mechanism of action allows taselisib to diminish the impact of feedback through RTKs, which otherwise attenuates anti-tumor activity.

Figure 2 shows Western blot analysis of HCC1954 cells (PIK3CA H1047R) 24 hrs treated with taselisib, pictilisib (GDC-0941, Genentech), and alpelisib (BYL719, Novartis CAS#: 1217486-61-7). Other oral PI3K inhibitors, pictilisib and alpelisib in clinic development do not degrade mutant p110a protein.

Figure 3 shows Western blot analysis of PIK3CA wildtype HDQP1 breast cancer cells and mutant HCC1954 (PIK3CA H1047R) breast cancer cells treated with taselisib. Taselisib leads to p110a depletion in PIK3CA mutant cell line without effecting p85 level.

Figure 4 shows Western blot analysis of SW48 isogenic lines including SW48 parental PIK3CA wildtype, mutant isogenic SW48 E545K heterozygote (het), and PIK3CA mutant isogenic SW48 H1047R heterozygote cells with taselisib at various concentrations; 0.2 μM, 1μM, 5μM, plus control (DMSO vehicle).

Figure 5A shows a plot of p110 alph mRNA expression in cells measured by relative p110alpha mRNA expression versus 18S RNA levels. Drug does not change the mRNA expression of p110a.

Figure 5B shows Western blot analysis of CRISPR (clustered regularly interspaced short palindromic repeats) generated SW48 E545K hemizygous lines (two clones ran in duplicates). This western blot shows significantly reduced mutant p110a level compare to SW48 E545K heterozygous line which suggests mutant p110a may be less stable than WT p110a. The lanes from left to right are SW48 E545K hemizygous clone 1, SW48 E545K hemizygous clone2, SW48 parental, SW48 E545K heterozygous, SW48 E545K heterozygous.

Figure 6 shows Western blot analysis of PIK3CA mutant HCC1954 (PIK3CA H1047R) breast cancer cells treated with taselisib at 1μM and 5μM, plus control (DMSO vehicle). P110 alpha (p110a) is depleted in a time dependent manner.

Figure 7A shows real time QPCR results in measuring relative RNA levels versus 18S control in HCC1954 wildtype (left) and H1047R mutant (right) p110 alpha cells treated with taselisib (GDC0032).

Figure 7B shows real time QPCR results in measuring p110a mRNA expression relative to RPL19 control in HCC1954 p110 alpha wildtype (left) and p110 alpha H1047R mutant (right) p110 alpha cells treated with taselisib (GDC0032).

Figure 7C shows real time QPCR results in measuring p110a mRNA expression relative to RPL19 control in HCC1954 p110 alpha wildtype (left) and p110 alpha H1047R mutant (right) p110 alpha cells treated with alpelisib (BYL-719).

Figure 8A shows Western blot analysis of mutant HCC1954 (PIK3CA H1047R) breast cancer cells treated with taselisib at 1.6μM for the indicated time. At 4 hours prior to harvest, 10 μM MG132 was added (right lanes).

Figure 8B shows Western blot analysis of lysates of mutant HCC1954 (PIK3CA H1047R) breast cancer cells treated with taselisib at 1.6μM for the indicated time. At 4 hours prior to harvest, 10 μM MG132 was added (middle lanes) and 10 μM UAE1 inhibitor.

Figure 8C shows Western blot analysis of mutant HCC1954 (PIK3CA H1047R) breast cancer cells treated with taselisib at 1.6μM for the indicated time. At 4 hours prior to harvest, 10 μM MG132 was added (right lanes).

Figure 8D shows Western blot analysis of mutant HCC1954 (PIK3CA H1047R) breast cancer cells treated with taselisib at 1.6μM for the indicated time. At 4 hours prior to harvest, 10 μM MG132 was added (right lanes).

Figure 8E shows Western blot analysis of mutant HCC1954 (PIK3CA H1047R) breast cancer cells treated with taselisib at 1.6μM for the indicated time. At 4 hours prior to harvest, 10 μM MG132, chloroquine, or ammonium chloride was added (right lanes).

Figure 8F shows a pathway diagram to ubiquitination of p110 describing two different mechanism by which animal cells degrade proteins with pathway specific inhibitors.

Figure 9A shows Western blot analysis of PI3K wildtype, BRAF mutant SW982 cells treated with taselisib and alpelisib (BYL719). Taselisib does not degrade or deplete p110 delta.

Figure 9B shows Western blot analysis of PI3K wildtype, B cell lymphoma SU-DHL-10 cells treated with G-102 (Table 1, US8242104) and GDC-0032 (taselisib).

Figure 10 shows Western blot analysis of PIK3CA wildtype HDQP1 breast cancer cells at 1 hr and 24 hr with PI3K inhibition by a PI3K inhibitor at various concentrations; 100nM, 1μM, 5μM, plus control (DMSO vehicle).

Figure 11A shows Western blot analysis of MDA-MB 453 (H1047R) cells at 1 hr and 24 hr with PI3K inhibition by G-102 (Table 1) at various concentrations; 3 nM, 16 nM, 60 nM, 400 nM, 2 μM, plus control (DMSO vehicle).

Figure 11B shows Western blot analysis of MDA-MB 453 (H1047R) cells at 1 hr and 24 hr with PI3K inhibition by taselisib (GDC-0032) at various concentrations; 3 nM, 16 nM, 60 nM, 400 nM, 2 μM, plus control (DMSO vehicle).

Figure 12 shows Western blot analysis of SW48 H1047R cells at 1 hr and 24 hr with PI3K inhibition by taselisib (GDC-0032) and G-102 (Table 1) at various concentrations; 1 nM, 10 nM, 100 nM, plus control (DMSO vehicle).

Figure 13 shows Western blot analysis of SW48 PIK3CA H1047R cells with and without growth factor ligand NRG treated with PI3K inhibition by taselisib (GDC-0032) and G-102 (US 8242104) at various concentrations; 1 nM, 10 nM, 100 nM, plus control (DMSO vehicle).

Figure 14A shows *in vitro* cellular proliferation of pPRAS40 in isogenic mutant (E545K, H1047R) versus wildtype parental PI3K cells at 24 hours at various concentrations of G-181, to establish parental/mutant selectivity EC50 values.

Figure 14B shows *in vitro* cellular proliferation of pPRAS40 in isogenic mutant (E545K, H1047R) versus wildtype parental PI3K cells at 24 hours at various concentrations GDC-0032, to establish parental/mutant selectivity EC50 values.

Figure 14C shows *in vitro* cellular proliferation of pPRAS40 in isogenic mutant (E545K, H1047R) versus wildtype parental PI3K cells at 24 hours at various concentrations G-102, to establish parental/mutant selectivity EC50 values.

Figure 15 shows a plot of *in vitro* cellular proliferation data with SW48 isogenic wildtype and mutant (E545K, H1047R) cell lines and treatment with dose titrations of: taselisib and pan-PI3K inhibitor, pictilisib (GDC-0941).

Figure 16 shows plots of efficacy (IC50 micromolar) of pictilisib (GDC-0941), BKM120 (buparlisib, Novartis AG, CAS Reg. No. 944396-07-0), taselisib (GDC-0032) and BYL719 (alpelisib, Novartis CAS#: 1217486-61-7) in a 4 day cell proliferation (Cell-Titer GloR, Promega) assay against PIK3CA mutant cell lines. Each dot represents a different cancer cell line.

Figure 17 shows plots of *in vitro* cellular proliferation data with PIK3CA wildtype and mutant (E545K, H1047R) cell lines and treatment with dose titrations of taselisib and PI3K alpha selective inhibitors GDC-0326 (US 8242104), and BYL719 in a 72 hr study with Cell Death-Nucleosome ELISA detection.

Figure 18A shows the fitted tumor volume change over 21 days in cohorts of 8-10 immunocompromised mice bearing HCC1954.x1 breast tumor xenografts harboring PIK3CA H1047R (PI3K$\alpha$) mutation dosed once daily by 100 microliter (ul) PO (oral) administration with Vehicle (MCT; 0.5% methycellulose/0.2% Tween 80), 150 mg/kg pictilisib (GDC-0941), and 25 mg/kg taselisib (GDC-0032). The term uL means microliter.

Figure 18B shows the fitted tumor volume change over 21 days in cohorts of 8-10 immunocompromised mice bearing HCC1954.x1 breast tumor xenografts harboring PIK3CA H1047R (PI3K$\alpha$) mutation dosed once daily by 100 microliter (ul) PO (oral) administration with Vehicle (MCT; 0.5% methycellulose/0.2% Tween 80), 40 mg/kg alpelisib (BYL-719), and 15 mg/kg taselisib (GDC-0032).

Figure 18C shows the fitted tumor volume change over 28 days in cohorts of 8-10 immunocompromised mice bearing WHIM20 hormone receptor positive patient-derived breast tumor xenografts harboring PIK3CA E542K (PI3K$\alpha$) mutation dosed once daily by 100 microliter (ul) PO (oral) administration with Vehicle (MCT; 0.5% methycellulose/0.2% Tween 80) and 15 mg/kg taselisib (GDC-0032).

Figure 18D shows the fitted tumor volume change over 27 days in cohorts of 8-10 immunocompromised mice bearing HCI-003 hormone receptor positive patient-derived breast tumor xenografts harboring PIK3CA H1047R (PI3K$\alpha$) mutation dosed once daily by 100 microliter (ul) PO (oral) administration with Vehicle (MCT; 0.5% methycellulose/0.2% Tween 80), 40 mg/kg alpelisib (BYL-719) and 2.5, 5.0, 15 mg/kg taselisib (GDC-0032).

Figure 19 shows Western blot analysis of mutant HCC1954 (PIK3CA H1047R) breast cancer cells treated with taselisib at various concentrations; 16 nM, 80 nM, 400 nM, plus control (DMSO vehicle).

Figure 20 shows a model of a conformation of the kinase domain of H0147R mutant p110 alpha PI3K isoform.

Figure 21 shows a plot of GDC-0032 potency (IC50s) in a four day viability assay across a cell line panel harboring PIK3CA mutants. Data is shown according to the location of the mutation in PIK3CA.

Figure 22 shows Western blot (WB) analysis of p85 co-immunoprecipitation (Co-IP) with p110a and the level parallel with p110a suggesting that stable p110a is in complex with p85 and significant dose dependent p110a degradation induced by taselisib.

Figure 23 shows steady state p110a mRNA expression.

Figure 24A shows trypsin cleavage of wild-type PIK3CA HCC-1954 (top) and H1047R mutation expressing PIK3CA HCC-1954 (bottom), according to Example 7.

Figure 24B shows liquid chromatography-tandem mass spectrometry (LC-MS/MS) analysis on the wild-type PIK3CA HCC-1954 (left) and H1047R mutation expressing PIK3CA HCC-1954 (right) after digestion and p110alpha (PIK3CA) protein immunoprecipitation, according to Example 7.

DETAILED DESCRIPTION OF EXEMPLARY EMBODIMENTS

Reference will now be made in detail to certain embodiments of the invention, examples of which are illustrated in the accompanying structures and formulas. While the invention will be described in conjunction with the enumerated embodiments, it will be understood that they are not intended to limit the invention to those embodiments. On the contrary, the invention is intended to cover all alternatives, modifications, and equivalents which may be included within the scope of the present invention as defined by the claims. One skilled in the art will recognize many methods and materials similar or equivalent to those described herein, which could be used in the practice of the present invention. The present

invention is in no way limited to the methods and materials described. In the event that one or more of the incorporated literature, patents, and similar materials differs from or contradicts this application, including but not limited to defined terms, term usage, described techniques, or the like, this application controls.

DEFINITIONS

The words "comprise," "comprising," "include," "including," and "includes" when used in this specification and claims are intended to specify the presence of stated features, integers, components, or steps, but they do not preclude the presence or addition of one or more other features, integers, components, steps, or groups thereof.

The terms "treat" and "treatment" refer to both therapeutic treatment and prophylactic or preventative measures, wherein the object is to prevent or slow down (lessen) an undesired physiological change or disorder, such as the growth, development or spread of cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The phrase "therapeutically effective amount" means an amount of a compound of the present invention that (i) treats the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can be measured, for example, by assessing the time to disease progression (TTP) and/or determining the response rate (RR).

The term "biological sample" comprises all samples of tissue, cells and body fluid taken from an animal or a human being.

An "effective response" of a patient or a patient's "responsiveness" to treatment with a medicament and similar wording refers to the clinical or therapeutic benefit imparted to a patient at risk for, or suffering from, a disease or disorder, such as cancer. In one embodiment, such benefit includes any one or more of: extending survival (including overall survival and progression free survival); resulting in an objective response (including a complete response or a partial response); or improving signs or symptoms of cancer. In one embodiment, a biomarker (e.g., mutant p110 alpha, for example, as determined using IHC) is used to identify the patient who is predicted to have an increase likelihood of being responsive to treatment with drug, e.g. taselisib (GDC-0032), relative to a patient who does not express the biomarker. In one embodiment, the biomarker, as determined using IHC) is used to identify the patient who is predicted to have an increase likelihood of being responsive to treatment with a drug, relative to a patient who does not express the biomarker at the same level. In one embodiment, the presence of the biomarker is used to identify a patient who is more likely to respond to treatment with a drug, relative to a patient that does not have the presence of the biomarker. In another embodiment, the presence of the biomarker is used to determine that a patient will have an increase likelihood of benefit from treatment with a drug, relative to a patient that does not have the presence of the biomarker.

The "amount" or "level" of a biomarker associated with an increased clinical benefit to a cancer (e.g. breast or NSCLC) patient refers to a detectable level in a biological sample wherein the level of biomarker is associated with increased patient clinical benefit. These can be measured by methods known to the expert skilled in the art and also disclosed by this invention. The expression level or amount of biomarker assessed can be used to determine the response to the treatment. In some embodiments, the amount or level of biomarker is determined using IHC (e.g., of patient tumor sample from biopsy or blood). In some embodiments, amount or level of a biomarker associated with an increased clinical benefit in a cancer patient is an IHC score of 2, an IHC score of 3, or an IHC score of 2 or 3. In some embodiments, amount or level of a c-met biomarker associated with an increased clinical benefit in a cancer patient is 50% or more tumor cells with moderate staining intensity, combined moderate/high staining intensity or high staining intensity. In some embodiments, amount or level of a biomarker associated with an increased clinical benefit in a cancer patient is 50% or more of tumor cells with moderate or high staining intensity.

The term "detection" includes any means of detecting, including direct and indirect detection.

The term "diagnosis" is used herein to refer to the identification or classification of a molecular or pathological state, disease or condition. For example, "diagnosis" may refer to identification of a particular type of cancer, *e.g.,* a lung cancer. "Diagnosis" may also refer to the classification of a particular type of cancer, *e.g.,* by histology (*e.g.,* a non small cell lung carcinoma), by molecular features (*e.g.,* a lung cancer characterized by nucleotide and/or amino acid variation(s)

in a particular gene or protein), or both.

The term "prognosis" is used herein to refer to the prediction of the likelihood of cancer-attributable death or progression, including, for example, recurrence, metastatic spread, and drug resistance, of a neoplastic disease, such as cancer.

The term "prediction" (and variations such as predicting) is used herein to refer to the likelihood that a patient will respond either favorably or unfavorably to a drug or set of drugs. In one embodiment, the prediction relates to the extent of those responses. In another embodiment, the prediction relates to whether and/or the probability that a patient will survive following treatment, for example treatment with a particular therapeutic agent and/or surgical removal of the primary tumor, and/or chemotherapy for a certain period of time without cancer recurrence. The predictive methods of the invention can be used clinically to make treatment decisions by choosing the most appropriate treatment modalities for any particular patient. The predictive methods of the present invention are valuable tools in predicting if a patient is likely to respond favorably to a treatment regimen, such as a given therapeutic regimen, including for example, administration of a given therapeutic agent or combination, surgical intervention, chemotherapy, *etc.,* or whether long-term survival of the patient, following a therapeutic regimen is likely.

The term "increased resistance" to a particular therapeutic agent or treatment option, when used in accordance with the invention, means decreased response to a standard dose of the drug or to a standard treatment protocol.

The term "decreased sensitivity" to a particular therapeutic agent or treatment option, when used in accordance with the invention, means decreased response to a standard dose of the agent or to a standard treatment protocol, where decreased response can be compensated for (at least partially) by increasing the dose of agent, or the intensity 5 of treatment.

"Patient response" can be assessed using any endpoint indicating a benefit to the patient, including, without limitation, (1) inhibition, to some extent, of tumor growth, including slowing down or complete growth arrest; (2) reduction in the number of tumor cells; (3) reduction in tumor size; (4) inhibition (*e.g.,* reduction, slowing down or complete stopping) of tumor cell infiltration into adjacent peripheral organs and/or tissues; (5) inhibition (*e.g.,* reduction, slowing down or complete stopping) of metastasis; (6) enhancement of anti-tumor immune response, which may, but does not have to, result in the regression or rejection of the tumor; (7) relief, to some extent, of one or more symptoms associated with the tumor; (8) increase in the length of survival following treatment; and/or (9) decreased mortality at a given point of time following treatment.

A "biomarker" is a characteristic that is objectively measured and evaluated as an indicator of normal biological processes, pathogenic processes, or pharmacological responses to a therapeutic intervention. Biomarkers may be of several types: predictive, prognostic, or pharmacodynamics (PD). Predictive biomarkers predict which patients are likely to respond or benefit from a particular therapy. Prognostic biomarkers predict the likely course of the patient's disease and may guide treatment. Pharmacodynamic biomarkers confirm drug activity, and enables optimization of dose and administration schedule.

"Change" or "modulation" of the status of a biomarker, including a PIK3CA mutation or set of PIK3CA mutations, as it occurs *in vitro* or *in vivo* is detected by analysis of a biological sample using one or more methods commonly employed in establishing pharmacodynamics (PD), including: (1) sequencing the genomic DNA or reverse-transcribed PCR products of the biological sample, whereby one or more mutations are detected; (2) evaluating gene expression levels by quantitation of message level or assessment of copy number; and (3) analysis of proteins by immunohistochemistry, immunocytochemistry, ELISA, or mass spectrometry whereby degradation, stabilization, or post-translational modifications of the proteins such as phosphorylation or ubiquitination is detected.

The terms "cancer" and "cancerous" refer to or describe the physiological condition in mammals that is typically characterized by unregulated cell growth. A "tumor" comprises one or more cancerous cells. Examples of cancer include, but are not limited to, carcinoma, lymphoma, blastoma, sarcoma, and leukemia or lymphoid malignancies. More particular examples of such cancers include squamous cell cancer (*e.g.,* epithelial squamous cell cancer), lung cancer including small- cell lung cancer, non-small cell lung cancer ("NSCLC"), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer. Gastric cancer, as used herein, includes stomach cancer, which can develop in any part of the stomach and may spread throughout the stomach and to other organs; particularly the esophagus, lungs, lymph nodes, and the liver.

The term "hematopoietic malignancy" refers to a cancer or hyperproliferative disorder generated during hematopoiesis involving cells such as leukocytes, lymphocytes, natural killer cells, plasma cells, and myeloid cells such as neutrophils and monocytes. Hematopoietic malignancies include non-Hodgkin's lymphoma, diffuse large hematopoietic lymphoma, follicular lymphoma, mantle cell lymphoma, chronic lymphocytic leukemia, multiple myeloma, acute myelogenous leukemia, and myeloid cell leukemia. Lymphocytic leukemia (or "lymphoblastic") includes Acute lymphoblastic

leukemia (ALL) and Chronic lymphocytic leukemia (CLL). Myelogenous leukemia (also "myeloid" or "nonlymphocytic") includes Acute myelogenous (or Myeloblastic) leukemia (AML) and Chronic myelogenous leukemia (CML).

A "chemotherapeutic agent" is a biological (large molecule) or chemical (small molecule) compound useful in the treatment of cancer, regardless of mechanism of action.

The term "mammal" includes, but is not limited to, humans, mice, rats, guinea pigs, monkeys, dogs, cats, horses, cows, pigs and sheep.

The term "package insert" is used to refer to instructions customarily included in commercial packages of therapeutic products, that contain information about the indications, usage, dosage, administration, contraindications and/or warnings concerning the use of such therapeutic products.

The phrase "pharmaceutically acceptable salt" as used herein, refers to pharmaceutically acceptable organic or inorganic salts of a compound of the invention. Exemplary salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate "mesylate", ethanesulfonate, benzenesulfonate, *p*-toluenesulfonate, and pamoate (i.e., 1,1'-methylene-bis-(2-hydroxy-3-naphthoate)) salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

The desired pharmaceutically acceptable salt may be prepared by any suitable method available in the art. For example, treatment of the free base with an inorganic acid, such as hydrochloric acid, hydrobromic acid, sulfuric acid, nitric acid, methanesulfonic acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like. Acids which are generally considered suitable for the formation of pharmaceutically useful or acceptable salts from basic pharmaceutical compounds are discussed, for example, by P. Stahl et al, Camille G. (eds.) Handbook of Pharmaceutical Salts. Properties, Selection and Use. (2002) Zurich: Wiley-VCH; S. Berge et al, Journal of Pharmaceutical Sciences (1977) 66(1) 1 19; P. Gould, International J. of Pharmaceutics (1986) 33 201 217; Anderson et al, The Practice of Medicinal Chemistry (1996), Academic Press, New York; Remington's Pharmaceutical Sciences, 18th ed., (1995) Mack Publishing Co., Easton PA; and in The Orange Book (Food & Drug Administration, Washington, D.C. on their website).

The phrase "pharmaceutically acceptable" indicates that the substance or composition must be compatible chemically and/or toxicologically, with the other ingredients comprising a formulation, and/or the mammal being treated therewith.

The term "synergistic" as used herein refers to a therapeutic combination which is more effective than the additive effects of the two or more single agents. A determination of a synergistic interaction between a mutant selective, PI3K-binding compound, or a pharmaceutically acceptable salt thereof, and one or more chemotherapeutic agent may be based on the results obtained from the assays described herein. The results of these assays can be analyzed using the Chou and Talalay combination method and Dose-Effect Analysis with CalcuSyn software in order to obtain a Combination Index (Chou and Talalay, 1984, Adv. Enzyme Regul. 22:27-55). The combination therapy may provide "synergy" and prove "synergistic", i.e., the effect achieved when the active ingredients used together is greater than the sum of the effects that results from using the compounds separately. A synergistic effect may be attained when the active ingredients are: (1) co-formulated and administered or delivered simultaneously in a combined, unit dosage formulation; (2) delivered by alternation or in parallel as separate formulations; or (3) by some other regimen. When delivered in alternation therapy, a synergistic effect may be attained when the compounds are administered or delivered sequentially, e.g., by different injections in separate syringes or in separate pills or tablets. In general, during alternation therapy, an effective dosage of each active ingredient is administered sequentially, i.e., serially, whereas in combination therapy, effective dosages of two or more active ingredients are administered together. Combination effects may also be evaluated using the BLISS independence model and the highest single agent (HSA) model (Lehár et al. 2007, Molecular Systems Biology 3:80).

"ELISA" (Enzyme-linked immunosorbent assay) is a popular format of a "wet-lab" type analytic biochemistry assay that uses one sub-type of heterogeneous, solid-phase enzyme immunoassay (EIA) to detect the presence of a substance in a liquid sample or wet sample (Engvall E, Perlman P (1971). "Enzyme-linked immunosorbent assay (ELISA). Quantitative assay of immunoglobulin G". Immunochemistry 8 (9): 871-4; Van Weemen BK, Schuurs AH (1971). "Immunoassay using antigen-enzyme conjugates". FEBS Letters 15 (3): 232-236). ELISA can perform other forms of ligand binding assays instead of strictly "immuno" assays, though the name carried the original "immuno" because of the

common use and history of development of this method. The technique essentially requires any ligating reagent that can be immobilized on the solid phase along with a detection reagent that will bind specifically and use an enzyme to generate a signal that can be properly quantified. In between the washes only the ligand and its specific binding counterparts remain specifically bound or "immunosorbed" by antigen-antibody interactions to the solid phase, while the nonspecific or unbound components are washed away. Unlike other spectrophotometric wet lab assay formats where the same reaction well (e.g. a cuvette) can be reused after washing, the ELISA plates have the reaction products immunosorbed on the solid phase which is part of the plate and thus are not easily reusable. Performing an ELISA involves at least one antibody with specificity for a particular antigen. The sample with an unknown amount of antigen is immobilized on a solid support (usually a polystyrene microtiter plate) either non-specifically (via adsorption to the surface) or specifically (via capture by another antibody specific to the same antigen, in a "sandwich" ELISA). After the antigen is immobilized, the detection antibody is added, forming a complex with the antigen. The detection antibody can be covalently linked to an enzyme, or can itself be detected by a secondary antibody that is linked to an enzyme through bioconjugation. Between each step, the plate is typically washed with a mild detergent solution to remove any proteins or antibodies that are not specifically bound. After the final wash step, the plate is developed by adding an enzymatic substrate to produce a visible signal, which indicates the quantity of antigen in the sample.

"Immunohistochemistry" (IHC) refers to the process of detecting antigens (e.g., proteins) in cells of a tissue section by exploiting the principle of antibodies binding specifically to antigens in biological tissues. Immunohistochemical staining is widely used in the diagnosis of abnormal cells such as those found in cancerous tumors. Specific molecular markers are characteristic of particular cellular events such as proliferation or cell death (apoptosis). IHC is also widely used to understand the distribution and localization of biomarkers and differentially expressed proteins in different parts of a biological tissue. Visualising an antibody-antigen interaction can be accomplished in a number of ways. In the most common instance, an antibody is conjugated to an enzyme, such as peroxidase, that can catalyze a color-producing reaction (see immunoperoxidase staining). Alternatively, the antibody can also be tagged to a fluorophore, such as fluorescein or rhodamine (see immunofluorescence).

"Immunocytochemistry" (ICC) is a common laboratory technique that uses antibodies that target specific peptides or protein antigens in the cell via specific epitopes. These bound antibodies can then be detected using several different methods. ICC can evaluate whether or not cells in a particular sample express the antigen in question. In cases where an immunopositive signal is found, ICC also determines which sub-cellular compartments are expressing the antigen.

"Isogenic" cell lines and human disease models are a family of cells that are selected or engineered to accurately model the genetics of a specific patient population, in vitro ("in the lab", in an artificial environment). They are provided with a genetically matched 'normal cell' to provide an isogenic system to research disease biology and novel therapeutic agents (Bardelli A, et al (2003) Science 300 (5621): 949). Isogenic cell lines can be used to model any disease with a genetic foundation. Cancer is one such disease for which isogenic human disease models have been widely used. Isogenic cell lines are created via a process called homologous gene-targeting. Targeting vectors that utilize homologous recombination are the tools or techniques that are used to knock-in or knock-out the desired disease causing mutation or SNP (single nucleotide polymorphism) to be studied. Although disease mutations can be harvested directly from cancer patients, these cells usually contain many background mutations in addition to the specific mutation of interest, and a matched normal cell line is typically not obtained. Subsequently, targeting vectors are used to 'knock-in' or 'knock out' gene mutations enabling a switch in both directions; from a normal to cancer genotype; or vice versa; in characterized human cancer cell lines.

The terms "adjuvant" and "adjuvant setting" refer to care or treatment that is given in addition to the primary, main or initial treatment. The surgeries and complex treatment regimens used in cancer therapy have led the term to be used mainly to describe adjuvant cancer treatments. An example of adjuvant therapy is the additional treatment usually given after surgery where all detectable disease has been removed, but where there remains a statistical risk of relapse due to occult disease. If known disease is left behind following surgery, then further treatment is not technically adjuvant. For example, radiotherapy or systemic therapy is commonly given as adjuvant treatment after surgery for breast cancer. Systemic therapy consists of chemotherapy, immunotherapy or biological response modifiers or hormone therapy. Oncologists use statistical evidence to assess the risk of disease relapse before deciding on the specific adjuvant therapy. The aim of adjuvant treatment is to improve disease-specific symptoms and overall survival. Because the treatment is essentially for a risk, rather than for provable disease, it is accepted that a proportion of patients who receive adjuvant therapy will already have been cured by their primary surgery. Adjuvant systemic therapy and radiotherapy are often given following surgery for many types of cancer.

The term "wild type PI3K p110 alpha isoform" means that no mutation exists in the PI3K p110 alpha gene.

The term "mutant PI3K p110 alpha isoform" means that one or more activating mutations lie within an allele of PI3K p110 alpha.

The parameter "$IC_{50}$" means the half maximal inhibitory concentration and is a measure of the effectiveness of a substance in inhibiting a specific biological or biochemical function. This quantitative measure indicates how much

of a particular drug or other substance (inhibitor) is needed to inhibit a given biological process (or component of a process, i.e. an enzyme, cell, cell receptor or microorganism) by half. It is commonly used as a measure of antagonist drug potency in pharmacological research. $IC_{50}$ represents the concentration of a drug that is required for 50% inhibition *in vitro* and is comparable to an $EC_{50}$ for agonist drugs. $EC_{50}$ also represents the plasma concentration required for obtaining 50% of a maximum effect *in vivo.* The parameter Ki is correlated with IC50 (Cer RZ et al (2009) Nucl. Acids Res. 37:W441-W445). Whereas $K_i$ is the binding affinity of the inhibitor, $IC_{50}$ is the functional strength of the inhibitor.

p110 ALPHA DEPLETION BY TASELISIB

Degradation of p110alpha (p110a) by taselisib occurs in a dose-dependent, time-dependent, proteasome dependent, and ubiquitin dependent manner. Degradation by taselisib is specific to p110a in PIK3CA mutant cells; no degradation of p85, p110 delta isoform, or p110a in wildtype cells is observed. The surprising and unexpected benefits of pathway suppression in the face of feedback is measured by pAKT and pPRAS40 levels at 1 and 24 hours. These correlative observations may widen the therapeutic window (increased therapeutic index) for treatment options with taselisib.

Degradation of p110a by taselisib can be tested by immunoprecipitation (IP) of p85/western blot for p110, or vice-versa, in the presence of increasing doses of taselisib. Since negligible p110 degradation occurs at 2 hours of treatment this is a reasonable window in which to evaluate p110/p85 dissociation without significant p110 degradation to complicate interpretation, and thus provides a diagnostic opportunity to predict cancer patients that will respond to treatment with taselisib.

Quantification of mutant versus wildtype p110 alpha degradation may be performed by proteomic techniques, including establishing the half-life of wildtype and mutant proteins, off-rates of taselisib in mutant and wildtype cell lines, and identification of ubiquitination sites and cellular machinery. Degradation of p110a may thus be measured as a depletion of p110a.

Taselisib is more effective than other PI3K inhibitors in mutant cells, because it uniquely degrades mutant-pl 10a protein. Figure 1 shows Western blot analysis of p110a protein degradation by taselisib is dose-dependent, specific to PI3Ka mutant cells. While the invention is not limited by any particular mechanism of action, this rationale for activity allows taselisib to diminish the impact of feedback through RTKs, which otherwise attenuates anti-tumor activity. Figure 2 shows Western blot analysis of HCC1954 cells (PIK3CA H1047R) 24 hrs treated with taselisib, pictilisib (GDC-0941, Genentech), and alpelisib (BYL719, Novartis CAS#: 1217486-61-7). Other oral PI3K inhibitors, pictilisib and alpelisib in clinic development do not degrade mutant p110a protein.

Taselisib leads to p110a depletion in PIK3CA mutant cell lines without effecting p85 level, consistent with a mechanism of dissociation of p110a/p85 that results in p110a monomer degradation (Figure 3). When p110 alpha is dissociated from p85, as a monomer p110 alpha is unstable and rapidly turned over (Yu et al (1998) Mol. Cell Bio. 18:1379-1387; Wu et al (2009) Proc. Natl. Acad. Sci. 106(48):20258-20263). The p110a half-life is approximately 1 hr, whereas the p110a/p85 dimer is significantly more stable, with a half-life of approximately 5 hr.

Mutant p110a is more susceptible than wild type to degradation by taselisib. Figure 4 shows Western blot analysis of SW48 isogenic lines including SW48 parental PIK3CA wildtype, mutant isogenic SW48 E545K heterozygote (het), and PIK3CA mutant isogenic SW48 H1047R heterozygote cells with taselisib at various concentrations; 0.2 μM, 1μM, 5μM, plus control (DMSO vehicle).

The p110a E545K mutant protein appears to be less stable than wildtype protein (Figures 5A and 5B). Mutant p110 alpha RNA expression is unchanged in the E545K engineered cells. Figure 5A shows a plot of p110 alpha mRNA expression in cells measured by relative p110alpha mRNA expression versus 18S RNA levels. Drug does not change the mRNA expression of p110a. Figure 5B shows Western blot analysis of CRISPR (clustered regularly interspaced short palindromic repeats) generated SW48 E545K hemizygous lines (two clones ran in duplicates). This western blot shows significantly reduced mutant p110a level compare to SW48 E545K heterozygous line which suggests mutant p110a may be less stable than WT p110a. The lanes from left to right are SW48 E545K hemizygous clone 1, SW48 E545K hemizygous clone2, SW48 parental, SW48 E545K heterozygous, SW48 E545K heterozygous.

P110 alpha is depleted in a time dependent manner. Figure 6 shows Western blot analysis of mutant HCC1954 (PIK3CA H1047R) breast cancer cells treated with taselisib at 1μM and 5μM. Since taselisib has a long clinical pharmacokinetic half-life of about 40 hours, as measured from patient samples, mutant p110a degradation should be occurring in tumors.

Taselisib does not decrease p110a RNA, although protein is decreased. Figure 7A shows real time QPCR results in measuring relative RNA levels versus 18S control in HCC1954 wildtype and mutant p110 alpha cells. No difference in p110a mRNA levels for DMSO vs. GDC-0032 treated cells was detected. There was approximately 8-fold higher expression of mutant allele. The DNA copy number PIK3CA is 4-5, with 1 WT and 3-4mutant alleles (exome seq). The Ratio of mutant to wildtype RNA predicts amount of drug-induced p110a degradation. Reduction of p110a does not occur at the transcriptional stage. Figure 7B shows real time QPCR results in measuring p110a mRNA expression

relative to RPL19 control in HCC1954 p110 alpha wildtype (left) and p110 alpha H1047R mutant (right) p110 alpha cells treated with taselisib (GDC0032). Figure 7C shows real time QPCR results in measuring p110a mRNA expression relative to RPL19 control in HCC1954 p110 alpha wildtype (left) and p110 alpha H1047R mutant (right) p110 alpha cells treated with alpelisib (BYL-719). The ratio of mRNA levels in wild type and mutant cells confirm that reduction of p110a does not occur at the transcriptional stage.

Assay Name: PIK3CA.H1047R.WT

| | | |
|---|---|---|
| FAM probe sequence: | ATGATGCACATCATGGT | (SEQ ID NO.:1) |
| Forward Primer Sequence: | GGCTTTGGAGTATTTCATGAAACA | (SEQ ID NO.:2) |
| Reverse Primer Sequence: | GAAGATCCAATCCATTTTTGTTGTC | (SEQ ID NO.:3) |

Assay Name: PIK3CA.H1047R.Mutant

| | | |
|---|---|---|
| FAM probe sequence: | TGATGCACGTCATGGT | (SEQ ID NO.:4) |
| Forward Primer Sequence: | GGCTTTGGAGTATTTCATGAAACA | (SEQ ID NO.:5) |
| Reverse Primer Sequence: | GAAGATCCAATCCATTTTTGTTGTC | (SEQ ID NO.:6) |

Depletion of p110a by taselisib is proteasome mediated (Figures 8A-8E) and requires E1 ubiquitin-activating enzyme, illustrated in Figure 8F. Figure 8A shows Western blot analysis of mutant HCC1954 (PIK3CA H1047R) breast cancer cells treated with taselisib at 1.6μM for the indicated time. At 4 hours prior to harvest, 10 μM of proteasome inhibitor MG132 (N-(benzyloxycarbonyl)leucinylleucinylleucinal Z-Leu-Leu-Leu-al, CAS Reg. No. 133407-82-6) was added (right lanes). MG-132 proteasome inhibitor rescues degradation of p110a by taselisib (GDC-0032). Adding proteasome inhibitor at 24 hrs is too late to protect from drug-induced degradation.

P110 alpha depletion is proteasome mediated and require E1 ubiquitin-activating enzyme. Figure 8B shows Western blot analysis of lysates of mutant HCC1954 (PIK3CA H1047R) breast cancer cells treated with taselisib at 1.6μM for the indicated time. At 4 hours prior to harvest, 10 μM MG132 was added (middle lanes) and 10 μM UAE1 inhibitor, ((2R,3S,4R,5R)-5-(6-(((S)-2,3-dihydro-1H-inden-1-yl)amino)-9H-purin-9-yl)-3,4-dihydroxytetrahydrofuran-2-yl)methyl sulfamate, CAS Reg. No. 905578-77-0, having the structure:

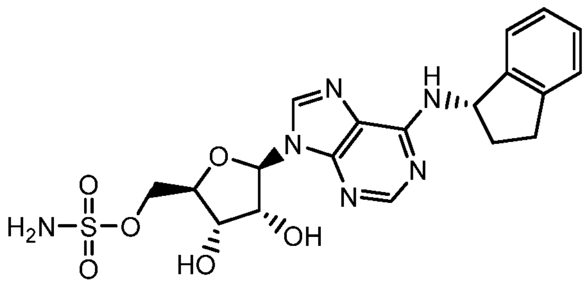

Figure 8C shows Western blot analysis of mutant HCC1954 (PIK3CA H1047R) breast cancer cells treated with taselisib at 1.6μM for the indicated time. At 4 hours prior to harvest, 10 μM MG132 was added (right lanes). Taselisib mediates p110a poly-ubiquitination and poly-ubiquitinated p110a accumulates with MG132. Treatment with the E1 inhibitor (UAE1 inhibitor), see Figure 8E, collapsed high molecular weight bands in the autoradiogram, confirming specificity of antibody.

Figure 8D also shows Western blot analysis of mutant HCC1954 (PIK3CA H1047R) breast cancer cells treated with taselisib at 1.6μM for the indicated time. At 4 hours prior to harvest, 10 μM MG132 was added (right lanes). Comparison of measurements conducted from the cell membrane and cytosol demonstrated that ubiquitination of p110a occurs primarily at the membrane. Membrane associated p110a is more efficiently ubiquitinated by taselisib than cytosolic p110a. Taselisib rapidly mediates degradation of mutant p110a at the plasma membrane. The degradation rate of membrane-associated p110a is much faster than cytosolic p110a degradation. Short term treatment of cells with taselisib mediates dose dependent degradation of membrane but not cytosolic p110a. In comparison, alpelisib (BYL-719) only has a weak effect at membrane and no effect in total lysate. Alpelisib showed a weak initial response in membrane but did not cause degradation of p110a over time. Taselisib is a superior degrader of p110a than alpelisib.

Figure 8E shows Western blot analysis of mutant HCC1954 (PIK3CA H1047R) breast cancer cells treated with taselisib at 1.6μM for the indicated time. At 4 hours prior to harvest, 10 μM MG132, chloroquine, or ammonium chloride

was added (right lanes). Taselisib mediated p110a depletion is not affected by lysosome inhibitors, suggesting that p110a degradation is not endosome/lysosome mediated, where proteasome inhibitor MG132 is a positive control.

Figure 8F shows a pathway diagram to ubiquitination of p110 alpha describing two different mechanism by which animal cells degrade proteins with pathway specific inhibitors (Jadhav, T. et al (2009) "Defining an Embedded Code for Protein Ubiquitination" J. Proteomics Bioinform, Vol 2(7):316-333; Wang, G. et al (2012) ("K63-linked ubiquitination in kinase activation and cancer" Frontiers in Oncology, Vol 2(5): 1-13). Because proteasome inhibitor MG132 but not lysosome inhibitors, chloroquine or ammonium chloride $NH_4Cl$, was able to rescue p110a degradation, GDC-0032 mediated p110a degradation is a ubiquitin proteasome dependent degradation pathway rather than dependent on a lysosome degradation machinery.

Thus, taselisib-mediated p110a protein degradation is time-dependent, dose-dependent, non-transcriptional, specific to mutant cells, ubiquitin dependent and mediated by the proteasome, and rapid for membrane-associated p110a.

Neither taselisib or alpelisib affect liver p110a at the membrane.

Taselisib does not degrade or deplete p110 delta (Figures 9A and 9B). Figure 9A shows Western blot analysis of PI3K wildtype, BRAF mutant SW982 cells treated with taselisib and alpelisib (BYL719, Novartis, CAS#: 1217486-61-7, (S)-N1-(4-methyl-5-(2-(1,1,1-trifluoro-2-methylpropan-2-yl)pyridin-4-yl)thiazol-2-yl)pyrrolidine-1,2-dicarboxamide). Taselisib does not degrade or deplete p110 delta. Figure 9B shows Western blot analysis of PI3K wildtype, B cell lymphoma SU-DHL-10 cells treated with taselisib and G-102 (Table 1, US8242104).

In the PI3K signaling pathway, PI3K inhibitors relieve negative feedback and prime the pathway for reactivation. When negative feedback is blocked, the phospho-RTK (ErbB2, EGFR, ErbB3) activity increases, effectiveness of PI3K inhibition is reduced, and pAKT is increased. Figure 10 shows Western blot analysis of PIK3CA wildtype HDQP1 cells at 1 hr and 24 hr with PI3K inhibition with a PI3K inhibitor at various concentrations; 100nM, 1μM, 5μM, plus control (DMSO vehicle).

Taselisib is better than another PI3K inhibitor G-102 (US 8242104) at maintaining signaling suppression at late timepoint. Figure 11A shows Western blot analysis of MDA-MB 453 (H1047R) cells at 1 hr and 24 hr with PI3K inhibition by G-102 at various concentrations; 3 nM, 16 nM, 60 nM, 400 nM, 2 μM, plus control (DMSO vehicle). Figure 11B shows Western blot analysis of MDA-MB 453 (H1047R) cells at 1 hr and 24 hr with PI3K inhibition by taselisib (GDC-0032) at various concentrations; 3 nM, 16 nM, 60 nM, 400 nM, 2 μM, plus control (DMSO vehicle).

Taselisib protects against RTK-driven pathway reactivation. At 1 hr the pAkt knockdown is approximately equivalent for both PI3K inhibitors. At 24 hrs there is increased pRTK. Taselisib is better at suppressing signaling at 24 hrs than G-102, a non-degrader PI3K inhibitor. Figure 12 shows Western blot analysis of SW48 H1047R cells at 1 hr and 24 hr with PI3K inhibition by taselisib (GDC-0032) and G-102 at various concentrations; 1 nM, 10 nM, 100 nM, plus control (DMSO vehicle).

In cells stimulated with growth factor ligand, taselisib is better than non-degrader G-102 at suppressing signaling. A rationale for this effect is mutant PI3Ka is more susceptible to degradation when pRTK is increased. pRTK binding is thought to shift p85 relative to p110α, leading to more active p110α (alpha). Hotspot p110α mutations can occur on the interface between p110α and p85, and may loosen p85/p110 interaction. Figure 13 shows Western blot analysis of SW48 PIK3CA H1047R cells with and without growth factor ligand NRG treated with PI3K inhibition by taselisib (GDC-0032) and G-102 at various concentrations; 1 nM, 10 nM, 100 nM, plus control (DMSO vehicle).

Measuring *in vitro* cellular proliferation, pPRAS40 in isogenic mutant vs wildtype cells at 24 hrs is useful to select PI3K inhibitor compounds in a degradation assay. Figures 14A-C shows *in vitro* cellular proliferation of pPRAS40 in isogenic mutant (E545K, H1047R) versus wildtype parental PI3K cells at 24 hours at various concentrations of G-181, GDC-0032 and G-102 to establish parental/mutant selectivity EC50 values.

Increased cellular potency of taselisib is observed relative to pan-PI3K inhibitor, pictilisib (GDC-0941) in mutant PI3Ka knock-in cells. Figure 15 shows a plot of *in vitro* cellular proliferation data with SW48 isogenic wildtype and mutant (E545K, H1047R) cell lines and treatment with dose titrations of taselisib and pictilisib.

Taselisib has enhanced potency in PIK3CA mutant cancer lines. Figure 16 shows plots of efficacy (IC50 micromolar) of pictilisib (GDC-0941), BKM120 (buparlisib, Novartis AG, CAS Reg. No. 944396-07-0, 5-(2,6-dimorpholinopyrimidin-4-yl)-4-(trifluoromethyl)pyridin-2-amine), taselisib (GDC-0032) and BYL719 (alpelisib, Novartis CAS#: 1217486-61-7) in a 4 day cell proliferation (Cell-Titer GloR, Promega) assay against PIK3CA mutant cell lines. Each dot represents a different cancer cell line. Pictilisib, BKM120 (buparlisib, Novartis AG, CAS Reg. No. 944396-07-0), and BYL719 are non-degraders of PI3K.

Taselisib has enhanced potency in an apoptosis assay. Figure 17 shows plots of *in vitro* cellular proliferation data with PIK3CA wildtype and mutant (E545K, H1047R) cell lines and treatment with dose titrations of taselisib and PI3K alpha selective inhibitor GDC-0326 (US 8242104), and BYL719 in a 72 hr study with Cell Death-Nucleosome ELISA detection.

Taselisib has greater maximal *in vivo* efficacy than other non-degrader drugs, in a PI3Kα mutant xenograft in mice. At the maximum tolerable dose (MTD), taselisib can cause tumor shrinkage. Figure 18A shows the fitted tumor volume change over 21 days in cohorts of 8-10 immunocompromised mice bearing HCC1954.x1 breast tumor xenografts

harboring PIK3CA H1047R (PI3Kα) mutation dosed once daily by 100 microliter (ul) PO (oral) administration with Vehicle (MCT; 0.5% methycellulose/0.2% Tween 80), 150 mg/kg pictilisib (GDC-0941), and 25 mg/kg taselisib (GDC-0032). The term uL means microliter. At maximum tolerated doses of both drugs, GDC-0032 is more efficacious than GDC-0941 and induces tumor regressions. Thus, GDC-0032 is more efficacious than a non-mutant selective PI3Kα inhibitor in a PI3K mutant xenograft model. Figure 18B shows the fitted tumor volume change over 21 days in cohorts of 8-10 immunocompromised mice bearing HCC1954.x1 breast tumor xenografts harboring PIK3CA H1047R (PI3Kα) mutation dosed once daily by 100 microliter (ul) PO (oral) administration with Vehicle (MCT; 0.5% methycellulose/0.2% Tween 80), 40 mg/kg alpelisib (BYL-719), and 15 mg/kg taselisib (GDC-0032). Oral and daily dosing of GDC-0032 for 21 days resulted in tumor regressions over the treatment (Rx) period. Alternatively, oral and daily dosing of the non-mutant selective PI3Kα inhibitor, BYL-719, over 21 days induced tumor stasis. Thus, GDC-0032 is more efficacious than a non-mutant selective PI3Ka inhibitor in a PI3K mutant xenograft model. Treatment with GDC-0032 and BYL-719 was well tolerated based on minimal changes in mouse body weight when compared to vehicle controls or from the initiation of the study.

The compound known as alpelisib (BYL719, Novartis, CAS#: 1217486-61-7) is an oral, selective inhibitor of the PI3K alpha isoform, and is in clinical trials for the potential treatment of a variety of tumor types, including a phase III study in combination with fulvestrant for second-line hormone receptor-positive, HER2- advanced metastatic breast cancer (Furet, P. et al (2013) Bioorg. Med. Chem. Lett. 23:3741-3748; US 8227462; US 8476268; US 8710085). Alpelisib is named as (S)-N1-(4-methyl-5-(2-(1,1,1-trifluoro-2-methylpropan-2-yl)pyridin-4-yl)thiazol-2-yl)pyrrolidine-1,2-dicarboxamide) and has the structure:

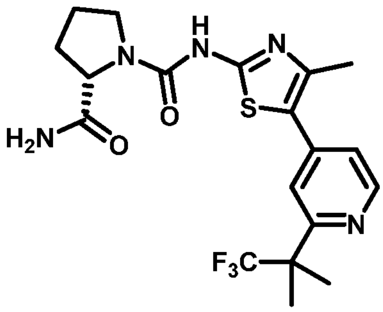

alpelisib (BYL-719)

Figure 18C shows the fitted tumor volume change over 28 days in cohorts of 8-10 immunocompromised mice bearing WHIM20 hormone receptor positive patient-derived breast tumor xenografts harboring PIK3CA E542K (PI3Kα) mutation dosed once daily by 100 microliter (ul) PO (oral) administration with Vehicle (MCT; 0.5% methylcellulose/0.2% Tween 80) and 15 mg/kg taselisib (GDC-0032). Oral and daily dosing of GDC-0032 for 28 days resulted in tumor regressions over the treatment period that was sustained after dosing had ended. Treatment with GDC-0032 was well tolerated based on minimal changes in mouse body weight when compared to vehicle controls or from the initiation of the study.

Figure 18D shows the fitted tumor volume change over 27 days in cohorts of 8-10 immunocompromised mice bearing HCI-003 hormone receptor positive patient-derived breast tumor xenografts harboring PIK3CA H1047R (PI3Kα) mutation dosed once daily by 100 microliter (ul) PO (oral) administration with Vehicle (MCT; 0.5% methylcellulose/0.2% Tween 80), 40 mg/kg alpelisib (BYL-719) and 2.5, 5.0, 15 mg/kg taselisib (GDC-0032). Oral and daily dosing of GDC-0032 for 27 days resulted in a dose-dependent increase in tumor regressions over the treatment (Rx) period. Alternatively, oral and daily dosing of the non-mutant selective PI3Kα inhibitor, BYL-719, over 27 days induced tumor stasis. Thus, GDC-0032 is more efficacious than a non-mutant selective PI3Ka inhibitor in a PI3K mutant xenograft model. Treatment with GDC-0032 and BYL-719 was well tolerated based on minimal changes in mouse body weight when compared to vehicle controls or from the initiation of the study.

Efficacy of GDC-0032 was evaluated in the WHIM20 hormone receptor positive patient-derived breast cancer xenograft model that harbors the PIK3CA E542K hot-spot mutation. Oral and daily dosing of GDC-0032 for 28 days resulted in tumor regressions over the treatment (Rx) period that was sustained after dosing had ended. Treatment with GDC-0032 was well tolerated based on minimal changes in mouse body weight when compared to vehicle controls or from the initiation of the study.

Efficacy of GDC-0032 was evaluated in the HCI-003 hormone receptor positive patient-derived breast cancer xenograft model that harbors the PIK3CA H1047R hot-spot mutation. Oral and daily dosing of GDC-0032 for 27 days resulted in a dose-dependent increase in tumor regressions over the treatment (Rx) period. Alternatively, oral and daily dosing of the non-mutant selective PI3Ka inhibitor, BYL-719, over 27 days induced tumor stasis. Thus, GDC-0032 is more efficacious than a non-mutant selective PI3Ka inhibitor in a PI3K mutant xenograft model. Treatment with GDC-0032 and BYL-719 was well tolerated based on minimal changes in mouse body weight when compared to vehicle

controls or from the initiation of the study.

Signaling rapidly returns to normal after taselisib washout, and p110a level begins to return at 8-24 hrs later in mutant HCC1954 (PIK3CA H1047R) breast cancer cells treated with taselisib. Figure 19 shows Western blot analysis of mutant HCC1954 (PIK3CA H1047R) breast cancer cells treated with taselisib at various concentrations; 16 nM, 80 nM, 400 nM, plus control (DMSO vehicle).

In one embodiment, depletion of p110 alpha protein can be measured by cellular proliferation, cell signaling, or apoptosis levels.

In one embodiment, depletion of p110 alpha protein is correlated with a measurable biomarker from a biological sample obtained from a patient. Depletion of p110 alpha protein may be detected in a clinical setting by a Protein Simple IEF (isoelectric focusing) technology. With adequate available patient tissue, western blot analysis, or mass spectroscopy could measure p110alpha protein levels. Mass spectrometry may allow interrogation of the proteome of single cells, including detection of ubiquitination. NMR (nuclear magnetic resonance) spectroscopy is another biophysical tool to detect and measure p110alpha and p85 dissociation or p110alpha degradation.

Alternatively a specific anti-pl 10alpha antibody may be useful in immunohistochemistry (IHC) or immunofluorescence (IF) based tests.

Immunoprecipitation (IP) and protein localization of PI3K proteins may detect changes in dissociation of p85 and p110alpha or when p110alpha is degraded before and after treatment by taselisib may identify and predict patient responders.

The activity of taselisib in a mutant isogenic PI3K cell line is greater than its activity in a wild type isogenic PI3K cell line. Isogenic PI3K mutant cell line may have a mutation selected from H1047R, C420R, H1047L, E542K, E545K and Q546R.

Taselisib differentially affects wild type p85/p110alpha complex relative to ATP-Km of mutant p85/p110alpha complex, as measured or detected by ATP-Km, PIP2-Km, the rate or extent of conversion of PIP2 to PIP3, membrane localization, lipid membrane affinity, and receptor tyrosine kinase binding.

Taselisib differentially induces conformational changes of wild type p85/p110alpha complex relative to mutant p85/p110alpha complex. Conformational changes include a binding interaction with mutant p85/p110alpha complex which is not present between taselisib with wild type p85/p110alpha complex.

In one embodiment, taselisib selectively binds a mutant form of isolated PI3K alpha and the IC50 for binding to mutant-PI3K alpha is less than the IC50 for binding to wild type-PI3K alpha.

In one embodiment, taselisib is more active in inhibiting a mutant-PI3K alpha isogenic cancer cell line than inhibiting a wild type form of the PI3K alpha isogenic cancer cell line.

In one embodiment, taselisib is selective for binding to the alpha-subunit of mutant-PI3K wherein the PI3K mutations are selected from H1047R, C420R, H1047L, E542K, E545K and Q546R, and has inhibitory activity, as measured by $IC_{50}$, in a mutant PI3K p110 alpha isoform cell line which is lower than the $IC_{50}$ inhibitory activity of taselisib in a wild type PI3K p110 alpha isoform cell line. The mutant PI3K p110 alpha isoform is selected from H1047R, C420R, H1047L, E542K, E545K and Q546R.

TRYPSIN CLEAVAGE AND MASS SPECTROSCOPY DETECTION OF P110 ALPHA DEPLETION

In addition to the methods above to detect and measure depletion of p110 alpha in biological samples treated with taselisib, direct detection can be achieved by mass spectrometry. HCC1954 breast cancer cells, expressing a mixture of wild type and H1047R mutant p110a protein, were treated with 500 nM taselisib (GDC-0032) in DMSO for 24 hours. Immunoprecipitation of p110 alpha protein was performed and captured protein separated by SDS-PAGE and Coomassie stained. Gel bands containing p110 alpha were subjected to in gel trypsin digestion to generate tryptic peptides for analysis. Tryptic peptides from cells treated with DMSO (control) or taselisib showed equivalent levels of the QM(ox)NDAHHGGWTTK peptide sequence (SEQ ID NO.:7), based on quantification of the corresponding 749.8282 m/z (+/- 10 ppm) ion.. Tryptic peptides from taselisib treated cells showed loss of mutant-specific peptide HGGWTTK (SEQ ID NO.:8), characterized by an ion of 393.6983 m/z (+/-10 ppm), relative to DMSO treated cells. Thus, neo-tryptic peptides specific to mutant tumors, such as p110a H1047R, can be used to demonstrate depletion of mutant p110a relative to wild type form.

| | | |
|---|---|---|
| wild type tryptic peptide | QM(ox)NDAHHGGWTTK | (SEQ ID NO.:7) |
| mutant tryptic peptide | HGGWTTK | (SEQ ID NO.:8) |

Figure 24A shows trypsin cleavage of wild-type PIK3CA HCC-1954 (top) and H1047R mutation expressing PIK3CA HCC-1954 (bottom), according to Example 7.

Figure 24B shows liquid chromatography-tandem mass spectrometry (LC-MS/MS) analysis on the wild-type

PIK3CA HCC-1954 (left) and H1047R mutation expressing PIK3CA HCC-1954 (right) after digestion and p110alpha (PIK3CA) protein immunoprecipitation, according to Example 7.

MUTANT SELECTIVITY

Table 1 compiles biological properties for several PI3K-binding compounds. GDC-0032 (Ndubaku et al (2013) Jour. Med. Chem. 56(11):4597-4610; Staben et al (2013) Bioorg. Med. Chem. Lett. 23 2606-2613; WO 2011/036280; US 8242104; US 8343955) is more potent against PI3K alpha mutant cancer cells relative to the pan-PI3K inhibitor compound GDC-0941 (Folkes et al (2008) Jour. of Med. Chem. 51(18):5522-5532; US 7781433; US 8324206). The four PI3K inhibitors of Table 1 differ in biochemical potency (Ki values) in binding to PI3K alpha and relative potency against the four wild type Class 1 isoforms of PI3K. GDC-0326 is an alpha selective PI3K inhibitor compound and binds weakly to the beta, delta, and gamma isoforms (US 8242104). GDC-0941 is a "pan" inhibitor, binding relatively well to all four isoforms. GDC-0032 is "beta sparing", binding well to the alpha, delta, and gamma isoforms and weakly to beta.

Table 1 PI3K binding compound activity

| Compound | wt-PI3Kα $K_i$ nM | mut-p110αH 1047R $K_i$ nM | mut-p110αE 545K $K_i$ nM | PI3K β $K_i$ nM (β/α) | PI3K δ $K_i$ nM (δ/α) | PI3K γ $K_i$ nM (γ/α) | Wild type-PI3K α/ Mut-PI3K α | MCF 7 $IC_{50}$ μM | PC3 $IC_{50}$ μM |
|---|---|---|---|---|---|---|---|---|---|
| G-102 | 0.14 | | | 39.7 (282) | 3.7 (27) | 16 (111) | | 0.056 | 1.6 |
| GDC-0326 | 0.23 | | | 31 (133) | 4.8 (20) | 12 (51) | 1 | | |
| GDC-0032 | 0.29 | 0.11 | 0.14 | 9.1 (31) | 0.092 (0.36) | 0.89 (3.5) | 2.6 | 0.025 (Mut E545 K) | not active |
| GDC-0941 | 3 | | | 33 (11) | 3 (1) | 75 (25) | | | |

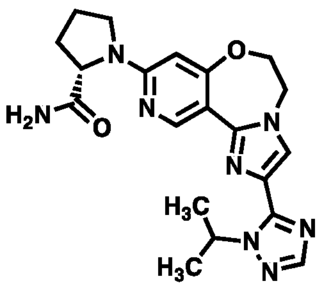

G-102

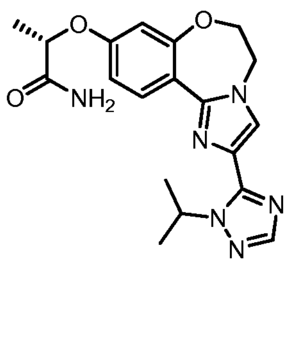

GDC-0326

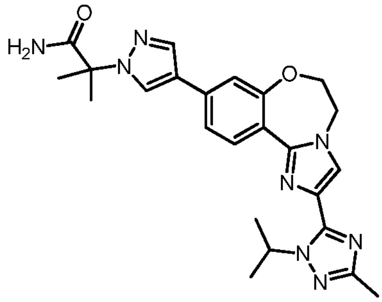

GDC-0032 (taselisib)

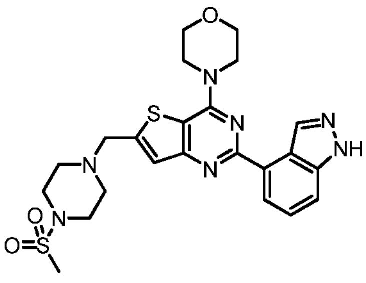

GDC-0941 (pictilisib)

Knock-in of mutant PI3K alpha increases cellular potency for taselisib (GDC-0032) as shown in SW48 isogenic lines, PI3K alpha wild-type (parental) and helical domain mutant E545K and kinase domain mutant H1047R, whereas GDC-0941 shows no such mutant selectivity effect. It can be deduced that GDC-0032 interacts with the mutant protein differently than GDC-0941 does. This unexpected result implies a unique mechanism or mode of binding of certain potent PI3K inhibitors, but not others. The mutant selectivity property of GDC-0032, lacking in GDC-0941, gives GDC-0032 greater maximal efficacy than GDC-0941 in a PI3K alpha mutant xenograft tumor model, HCC1954 breast cancer with kinase domain H1047R mutation. Following daily oral dosing for 21 days, at the maximum tolerated dose of 25 mg/kg, GDC-0032 induced tumor regressions whereas at the maximum tolerated dose of 150 mg/kg, GDC-0941 caused tumor growth inhibition.

*In vitro* tumor cell proliferation was measured in cancer cell lines treated with GDC-0032, GDC-0326 (US 8242104) or GDC-0941. GDC-0032 induces apoptosis in PI3K mutant cells at low concentrations. Increased mutant potency of GDC-0032 is not correlated with biochemical binding potency against wild-type PI3K alpha. Increased potency against PI3K mutant cell lines and tumors by PI3K inhibitor compounds may be affected by: physicochemical or permeability properties, intracellular levels, isoform selectivity, and absolute potency against wild type p110 alpha or wild type p110 delta. In a cell viability assay, p110 alpha-selective inhibitor GDC-0326 (Table 1) was 6X less potent against the H1047R p110 alpha cell line, HCC1954 than GDC-0032. GDC-0032 is less alpha selective against the beta, gamma, and delta isoforms than GDC-0326 (US 8242104). Knock-in of mutant PI3K alpha increases cellular potency for mutant selective GDC-0032, but not for PI3K alpha-specific inhibitors, such as G-102. A similar cell viability assay determined that inhibition of p110 delta does not decrease viability in PI3K mutant cell lines. Also, comparable intracellular levels of GDC-0032 were measured (pmol/mg) in various wild type and PI3K mutant cells. Results indicate intracellular accumulation does not explain increased mutant potency of GDC-0032.

Autoradiography of gel electrophoresis of radiolabelled lysates from mutant isogenic SW48 PI3K alpha H1047R cell line and wild type SW48 parental cells treated with GDC-0032, GDC-0326 or GDC-0941 measured cleaved PARP, pS6($^{S235/236}$), pAKT$^{T308}$, pAKT$^{S473}$, beta-Actin and GAPDH by Western blot analysis. PI3K pathway knockdown correlates with the induction of apoptosis in a dose-dependent manner. Taselisib induces apoptosis in cells harboring PI3K mutations at very low compound concentrations. Similar effects were seen in isogenic cells from MCF10A breast cell line and HCC1954, PI3K alpha H1047R breast cancer cell line. Despite comparable compound properties, pathway knockdown is stronger with mutant selective GDC-0032 than non-mutant selective GDC-0326. Increased mutant potency of GDC-0032 is not explained by biochemical potency against wild type PI3K alpha.

By these assays, compound can be assessed to examine the impact of structural changes on PI3K alpha mutant selectivity. Changes in size and hydrogen bonding capability in a specific region may correlate with improved selectivity.

Preliminary clinical trial data showed that taselisib achieved partial responses in five out of ten patients with PIK3CA mutant tumors, and four out of five patients with PIK3CA mutant breast tumors (Olivero and Juric (2013) AACR).

INTERACTIONS OF TASELISIB WITH PI3K

Taselisib (GDC-0032) is more selective for PI3K alpha mutant isoforms than PI3K wild type isoforms due to key interactions with PI3K alpha mutant isoforms that differ from interactions with the PI3K wild type isoform, and may include a precise positioning and arrangement of atoms and functional groups to interact with key mutant-specific features of PI3K alpha mutant isoforms. Such interactions may be achieved by functional groups acting as hydrogen bond-donors, hydrogen bond-acceptors and/or Van der Waals force partners with the PI3K alpha mutant isoform protein (Staben et al (2013) Bioorg. Med. Chem. Lett. 23:2606-2613; Ndubaku et al (2013) Jour. Med. Chem. 56(11):4597-4610). Taselisib may adopt binding topologies in low energy conformations and make efficient polar and van der Waals interactions in the ligand binding site.

It has been established that PIK3CA mutations increase lipid binding and PI3K basal activity (Burke et al (2012) Proc. Natl. Acad. Sci. 109:15259-15264). Mutations destabilize the closed, cytosolic inactive form of PI3K alpha, promoting increased lipid binding. The mutant selective, PI3K-binding compounds of the invention increase stabilization of the closed form of PI3K alpha, preventing conformational changes that increase lipid binding. Hotspot mutations induce regions of the kinase domain to be more deuterated in hydrogen-deuterium exchange of the H1047R mutant indicating they become more dynamic (destabilized) and available for exchange. Those changes were accompanied by increased affinity for lipid membrane and may account for increased activation and downstream signaling. This dynamic region, residues 848-859 (Figure 20), may provide key binding interactions for the compounds of the invention.

Figure 21 shows a plot of binding potency of GDC-0032 viability in cell lines with PIK3CA mutants, according to the location of the mutation in PIK3CA. GDC-0032 is potent against cell lines harboring PI3K mutations regardless of the location of the mutation. Some of the cell lines have additional mutations such as B-Raf and Ras as resistance markers.

Figure 22 shows Western blot (WB) analysis of p85 co-immunoprecipitation (Co-IP) with p110a and the level parallel with p110a suggesting that stable p110a is in complex with p85 and significant dose dependent p110a degradation induced by taselisib. Cells were treated with GDC-0032 for 24h, a point at which p110a is clearly being degraded. Alternative experiments may employ treatment of cells with sampling at shorter time points (2 hr, 4 hr, etc) where p110alpha is not yet degraded but is dissociated from p85. Detection of dissociation of mutant but not wild type p110alpha from p85 after treatment with taselisib may be a predictive biomarker for patients with mutant PI3K tumors likely to respond to treatment with taselisib.

Figure 23 shows steady state p110a mRNA expression. Cell lines most sensitive to p110a degradation by GDC0032 harbor more H1047R mutation than WT p110a. The ratio of Mutant to WT p110a allele may determine sensitivity to GDC-0032 mediated p110a degradation.

X-ray structures of PI3K beta (Zhang, X. et al (2011) Mol. Cell 41:567-5789), PI3K alpha (Huang, C.-H. et al (2007) Science 318:1744), PI3K delta (Berndt, A. et al (2010) Nat. Chem. Biol. 6:11), and PI3K gamma ("Structural determinants of phosphoinositide 3-kinase inhibition by wortmannin, LY294002, quercetin, myricetin, and staurosporine", Walker, E.H. et al (2000) Mol.Cell 6:909) have been reported. One difference of note is the conformation of a tryptophan residue present in all isoforms (alpha-Trp780, beta-Trp781, delta-Trp760, gamma-TRP812). This is the same tryptophan residue that is thought to be a crucial for obtaining isoform specificity for PI3K delta as the result of an 'induced fit' movement of adjacent residues or specific interaction by inhibitors. The conformation of the indole of this Trp residue is unique in PI3K beta and may provide basis for design of "beta-sparing" PI3K inhibitors with low, weak binding to PI3K beta. Rotation along $C\alpha$-$C\beta$ of beta-Trp781 presents an alternate orientation. Differences in second shell residues promote a unique orientation of this Trp in PI3K beta. First, the indole N-H donates an H-bond to acidic residues in both PI3K alpha (Glu798) and PI3K gamma (Glu814) that occupy a similar region of the binding site but are not present in PI3K beta or delta. This interaction could favor the observed orientation of the indole in these isoforms. In place of Glu814, PI3K beta and delta possess neutral residues with nonpolar side chains (beta-Val783 and delta-Met762 respectively) and thus the indole can occupy other energetically favorable orientations. It is possible that the branched valine residue of PI3K beta disfavors orientations similar to those observed in alpha, delta and gamma; the C gamma methyl instead approaches van der Waals distance to the indole 4/5 position. Under this interpretation of structure-activity relationship (SAR), in this unique conformation the Trp sidechain is more easily insulted by steric bulk of the inhibitor. Also of note is the differential angle and atoms presented for pi-stacking by the indole in PI3K alpha, delta and gamma relative to beta. For PDB analysis of pi-interaction between tryptophan rings and aromatic amino acid side chains (Phe, Tyr, His) see: Samanta, U. et al (1999) Biol. Crystallogr., D55:1421. This observation could partially explain why non-aromatic substitution in the same region results in molecules with lower overall selectivity over PI3Kb (Staben et al (2013) Bioorg. Med. Chem. Lett. 23:2606-2613).

BIOMARKER DETECTION OF MUTANT P110 ALPHA

In certain embodiments, the presence and/or expression level/amount of biomarker proteins in a sample is

examined using immunohistochemistry (IHC) and staining protocols. IHC staining of tissue sections has been shown to be a reliable method of determining or detecting presence of proteins in a sample. In some embodiments of any of the methods, assays and/or kits, a relevant biomarker is mutant p110 alpha protein. In some embodiments, mutant p110 alpha is detected by immunohistochemistry. In some embodiments, elevated expression of a mutant p110 alpha biomarker in a sample from an individual is elevated protein expression and, in further embodiments, is determined using IHC. In one embodiment, expression level of biomarker is determined using a method comprising: (a) performing IHC analysis of a sample (such as a subject cancer sample) with an antibody; and b) determining expression level of a biomarker in the sample. In some embodiments, IHC staining intensity is determined relative to a reference. In some embodiments, the reference is a reference value. In some embodiments, the reference is a reference sample (e.g., control cell line staining sample or tissue sample from non-cancerous patient).

IHC may be performed in combination with additional techniques such as morphological staining and/or fluorescence in-situ hybridization. Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody. The primary and/or secondary antibody used for IHC typically will be labeled with a detectable moiety. Numerous labels are available which can be generally grouped into the following categories: (a) Radioisotopes, such as 35S, 14C, 1251, 3H, and 1311; (b) colloidal gold particles; (c) fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above; (d) various enzyme-substrate labels are available (US 4275149; US 4318980). Examples of enzymatic labels include luciferases (e.g., firefly luciferase and bacterial luciferase (US 4737456), luciferin, 2,3-dihydrophthalazinediones, malate dehydrogenase, urease, peroxidase such as horseradish peroxidase (HRPO), alkaline phosphatase, .beta.-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Examples of enzyme-substrate combinations include, for example, horseradish peroxidase (HRPO) with hydrogen peroxidase as a substrate; alkaline phosphatase (AP) with para-Nitrophenyl phosphate as chromogenic substrate; and .beta.-D-galactosidase (beta-D-Gal) with a chromogenic substrate (e.g., p-nitrophenyl-.beta.-D-galactosidase) or fluorogenic substrate (e.g., 4-methylumbelliferyl-.beta.-D-galactosidase). In some embodiments of any of the methods, a biomarker such as mutant p110 alpha protein is detected by immunohistochemistry using an anti-mutant p110 alpha diagnostic antibody (i.e., primary antibody). In some embodiments, the anti-mutant p110 alpha diagnostic antibody specifically binds human mutant p110 alpha. In some embodiments, the anti-mutant p110 alpha antibody is a nonhuman antibody, such as a rat, mouse, or rabbit antibody. In some embodiments, anti-mutant p110 alpha diagnostic antibody is a monoclonal antibody. In some embodiments, the anti-mutant p110 alpha diagnostic antibody is directly labeled.

Specimens thus prepared may be mounted and coverslipped. Slide evaluation is then determined, e.g., using a microscope, and staining intensity criteria, routinely used in the art, may be employed. In one embodiment, it is understood that when cells and/or tissue from a tumor is examined using IHC, staining is generally determined or assessed in tumor cell and/or tissue (as opposed to stromal or surrounding tissue that may be present in the sample). In some embodiments, it is understood that when cells and/or tissue from a tumor is examined using IHC, staining includes determining or assessing in tumor infiltrating immune cells, including intratumoral or peritumoral immune cells. In some embodiments, the presence of a mutant p110 alpha biomarker is detected by IHC in >0% of the sample, in at least 1% of the sample, in at least 5% of the sample, in at least 10% of the sample. In some embodiments, the mutant p110 alpha biomarker is detected by IHC in tumor cells.

In alternative methods, the biological sample from a patient, e.g. tumor biopsy or circulating tumor cell, may be contacted with an antibody specific for a biomarker under conditions sufficient for an antibody-biomarker complex to form, and then detecting said complex. The presence of the biomarker may be detected in a number of ways, such as by Western blotting and ELISA procedures for assaying a wide variety of tissues and samples, including plasma or serum. A wide range of immunoassay techniques using such an assay format are available (US 4016043; US 4424279; US 4018653), including single-site and two-site or "sandwich" assays of the non-competitive types, as well as in the traditional competitive binding assays. These assays also include direct binding of a labeled antibody to a target biomarker. Presence and/or expression level/amount of a selected biomarker in a tissue or cell sample may also be examined by way of functional or activity-based assays. For instance, if the biomarker is an enzyme, one may conduct assays known in the art to determine or detect the presence of the given enzymatic activity in the tissue or cell sample.

The anti-mutant p110 alpha antibody or antigen binding fragment thereof, may be made using methods known

in the art, for example, by a process comprising culturing a host cell containing nucleic acid encoding any of the previously described anti- mutant p110 alpha antibodies or antigen-binding fragment in a form suitable for expression, under conditions suitable to produce such antibody or fragment, and recovering the antibody or fragment.

METHODS OF TREATMENT

Taselisib (GDC-0032) is useful in treating hyperproliferative disorders including cancer. In one example, a patient with a mutant PI3K tumor is treated with taselisib. The mutant PI3K tumor may be a breast tumor, a lung tumor, or a tumor found in other organs.

Disclosed is a method for the treatment of cancer comprising administering taselisib to a patient, wherein the activity of taselisib in a mutant isogenic PI3K cell line is greater than the activity of taselisib in a wild type isogenic PI3K cell line.

Further disclosed is a method for the treatment cancer comprising administering taselisib to a patient, wherein the IC50 binding activity of taselisib to a mutant PI3K p110 alpha isoform is lower than the IC50 binding activity of taselisib to wild type PI3K p110 alpha isoform.

In one example, the PI3K p110 alpha isoform mutant is selected from H1047R, H1047L, E542K, E545K and Q546R.

In one example, a biological sample obtained from the patient prior to administration of taselisib has been tested for PIK3CA or PTEN mutation status, and wherein PIK3CA or PTEN mutation status is indicative of therapeutic responsiveness by the patient to taselisib.

In one example, the mutation status includes detecting mutants selected from H1047R, H1047L, C420R, E542K, E545K or Q546R.

In one example, the hyperproliferative disorder is HER2 expressing breast cancer or estrogen receptor positive (ER+) breast cancer, where the breast cancer may be metastatic.

In one example, taselisib is administered to a patient in an adjuvant setting.

In one example, the patient has been previously treated with tamoxifen, fulvestrant, or letrozole.

The methods of the disclosure also include:

- methods of diagnosis based on the identification of a biomarker;
- methods of determining whether a patient will respond to taselisib, or a combination of taselisib and a chemotherapeutic agent;
- methods of optimizing therapeutic efficacy by monitoring clearance of taselisib, or a combination of taselisib and a chemotherapeutic agent;
- methods of optimizing a therapeutic regime of taselisib, or a combination of taselisib and a chemotherapeutic agent, by monitoring the development of therapeutic resistance mutations; and
- methods for identifying which patients will most benefit from treatment taselisib or a combination of taselisib and a chemotherapeutic agent therapies and monitoring patients for their sensitivity and responsiveness to treatment with taselisib or a combination of taselisib and a chemotherapeutic agent therapies.

The methods are useful for inhibiting abnormal cell growth or treating a hyperproliferative disorder such as cancer in a mammal (e.g., human patient). For example, the methods are useful for diagnosing, monitoring, and treating multiple myeloma, lymphoma, leukemias, prostate cancer, breast cancer, hepatocellular carcinoma, pancreatic cancer, and/or colorectal cancer in a mammal (e.g., human).

Therapeutic combinations of: (1) taselisib and (2) a chemotherapeutic agent are useful for treating diseases, conditions and/or disorders including, but not limited to, those characterized by activation of the PI3 kinase pathway. Accordingly, another aspect includes methods of treating diseases or conditions that can be treated by inhibiting lipid kinases, including PI3K. In one example, a method for the treatment of a solid tumor or hematopoietic malignancy comprises administering a therapeutic combination including taselisib as a combined formulation or by alternation to a mammal, wherein the therapeutic combination comprises a therapeutically effective amount of taselisib, and a therapeutically effective amount of one or more chemotherapeutic agents selected from 5-FU, docetaxel, eribulin, gemcitabine, cobimetinib (GDC-0973, XL-518, CAS Reg. No. 934660-93-2), GDC-0623 (CAS Reg. No. 1168091-68-6), paclitaxel, tamoxifen, fulvestrant, dexamethasone, pertuzumab, trastuzumab emtansine, trastuzumab and letrozole. Therapeutic combinations of: (1) taselisib and (2) a chemotherapeutic agent may be employed for the treatment of a hyperproliferative disease or disorder, including hematopoietic malignancy, tumors, cancers, and neoplastic tissue, along with pre-malignant and non-neoplastic or non-malignant hyperproliferative disorders. In one example, a human patient is treated with a therapeutic combination and a pharmaceutically acceptable carrier, adjuvant, or vehicle, wherein taselisib, or metabolite thereof, of said therapeutic combination is present in an amount to detectably inhibit PI3 kinase activity.

Hematopoietic malignancies include non-Hodgkin's lymphoma, diffuse large hematopoietic lymphoma, follicular lymphoma, mantle cell lymphoma, chronic lymphocytic leukemia, multiple myeloma, AML, and MCL.

Another aspect provides a pharmaceutical composition or therapeutic combination for use in the treatment of the diseases or conditions described herein in a mammal, for example, a human, suffering from such disease or condition. Also provided is the use of a pharmaceutical composition in the preparation of a medicament for the treatment of the diseases and conditions described herein in a warm-blooded animal, such as a mammal, for example a human, suffering from such disorder.

Another aspect provides taselisib for use in the treatment of cancer wherein the patients to be treated have a PIK3CA mutation, wherein the mutation comprises a mutation selected from H1047R, C420R, H1047L, E542K, E545K and Q546R.

Another aspect provides taselisib for use in the treatment of cancer wherein the patients to be treated have a PIK3CA mutation with depletion of p110 alpha isoform after contacting taselisib with a biological sample with PIK3CA mutation status, wherein the mutation comprises a mutation selected from H1047R, C420R, H1047L, E542K, E545K and Q546R.

Another aspect provides the use of taselisib in the manufacture of a medicament for the treatment of cancer, wherein the subject to be treated have a PIK3CA mutation.

Another aspect provides the use of taselisib in the manufacture of a medicament for the treatment of cancer wherein the patients to be treated have a PIK3CA mutation with depletion of p110 alpha isoform after contacting taselisib with a biological sample with PIK3CA mutation status, wherein the mutation comprises a mutation selected from H1047R, C420R, H1047L, E542K, E545K and Q546R.

Another aspect of this invention provides taselisib for use in the treatment of cancer, wherein the patients have a depletion of p110 alpha isoform after contacting taselisib.

Another aspect of this invention provides the method of determining the responsiveness to taselisib comprising the steps:

a) administering taselisib; and

b) measuring a change in the level of p110 alpha or a biomarker correlated to the level of p110 alpha in a biological sample obtained from the patient.

PHARMACEUTICAL COMPOSITIONS AND FORMULATIONS

Pharmaceutical compositions or formulations of the compounds of the invention comprise taselisib and one or more of a pharmaceutically acceptable carrier, a glidant, a diluent, and an excipient.

Pharmaceutical compositions or formulations of the compounds of the invention further comprise a second chemotherapeutic agent.

Mutant selective, PI3K-binding compounds and chemotherapeutic agents of the present invention may exist in unsolvated as well as solvated forms with pharmaceutically acceptable solvents such as water, ethanol, and the like, and it is intended that the invention embrace both solvated and unsolvated forms.

The compounds of the present invention may also exist in different tautomeric forms, and all such forms are embraced within the scope of the invention. The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

Pharmaceutical compositions encompass both the bulk composition and individual dosage units comprised of more than one (e.g., two) pharmaceutically active agents including a mutant selective, PI3K-binding compound and a chemotherapeutic agent selected from the lists of the additional agents described herein, along with any pharmaceutically inactive excipients, diluents, carriers, or glidants. The bulk composition and each individual dosage unit can contain fixed amounts of the aforesaid pharmaceutically active agents. The bulk composition is material that has not yet been formed into individual dosage units. An illustrative dosage unit is an oral dosage unit such as tablets, pills, capsules, and the like. Similarly, the methods of treating a patient by administering a pharmaceutical composition is also intended to encompass the administration of the bulk composition and individual dosage units.

Pharmaceutical compositions also embrace isotopically-labeled compounds of the present invention which are identical to those recited herein, but for the fact that one or more atoms are replaced by an atom having an atomic mass or mass number different from the atomic mass or mass number usually found in nature. All isotopes of any particular

atom or element as specified are contemplated within the scope of the compounds of the invention, and their uses. Exemplary isotopes that can be incorporated into compounds of the invention include isotopes of hydrogen, carbon, nitrogen, oxygen, phosphorus, sulfur, fluorine, chlorine and iodine, such as $^{2}H$, $^{3}H$, $^{11}C$, $^{13}C$, $^{14}C$, $^{13}N$, $^{15}N$, $^{15}O$, $^{17}O$, $^{18}O$, $^{32}P$, $^{33}P$, $^{35}S$, $^{18}F$, $^{36}Cl$, $^{123}I$ and $^{125}I$. Certain isotopically-labeled compounds of the present invention (e.g., those labeled with $^{3}H$ and $^{14}C$) are useful in compound and/or substrate tissue distribution assays. Tritiated ($^{3}H$) and carbon-14 ($^{14}C$) isotopes are useful for their ease of preparation and detectability. Further, substitution with heavier isotopes such as deuterium ($^{2}H$) may afford certain therapeutic advantages resulting from greater metabolic stability (e.g., increased in vivo half-life or reduced dosage requirements) and hence may be preferred in some circumstances. Positron emitting isotopes such as $^{15}O$, $^{13}N$, $^{11}C$ and $^{18}F$ are useful for positron emission tomography (PET) studies to examine substrate receptor occupancy. Isotopically labeled compounds of the present invention can generally be prepared by following procedures analogous to those disclosed in the Examples herein below, by substituting an isotopically labeled reagent for a non-isotopically labeled reagent.

Taselisib is formulated in accordance with standard pharmaceutical practice for use in a therapeutic combination for therapeutic treatment (including prophylactic treatment) of hyperproliferative disorders in mammals including humans. The invention provides a pharmaceutical composition comprising a mutant selective, PI3K-binding compound in association with one or more pharmaceutically acceptable carrier, glidant, diluent, additive, or excipient.

Suitable carriers, diluents, additives, and excipients are well known to those skilled in the art and include materials such as carbohydrates, waxes, water soluble and/or swellable polymers, hydrophilic or hydrophobic materials, gelatin, oils, solvents, water and the like. The particular carrier, diluent or excipient used will depend upon the means and purpose for which the compound of the present invention is being applied. Solvents are generally selected based on solvents recognized by persons skilled in the art as safe (GRAS) to be administered to a mammal. In general, safe solvents are non-toxic aqueous solvents such as water and other non-toxic solvents that are soluble or miscible in water. Suitable aqueous solvents include water, ethanol, propylene glycol, polyethylene glycols (e.g., PEG 400, PEG 300), dimethylsulfoxide (DMSO), cremophor (e.g. CREMOPHOR EL®, BASF), and mixtures thereof. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents and other known additives to provide an elegant presentation of the drug (i.e., a compound of the present invention or pharmaceutical composition thereof) or aid in the manufacturing of the pharmaceutical product (i.e., medicament).

Pharmaceutical formulations of the compounds of the present invention may be prepared for various routes and types of administration. For example, a mutant selective, PI3K-binding compound having the desired degree of purity may optionally be mixed with pharmaceutically acceptable diluents, carriers, excipients or stabilizers (Remington's Pharmaceutical Sciences (1995) 18th edition, Mack Publ. Co., Easton, PA), in the form of a lyophilized formulation, milled powder, or an aqueous solution.

The pharmaceutical formulations of the invention will be dosed and administered in a fashion, i.e., amounts, concentrations, schedules, course, vehicles and route of administration, consistent with good medical practice.

The initial pharmaceutically effective amount of taselisib administered orally or parenterally per dose will be in the range of about 0.01-1000 mg/kg, namely about 0.1 to 20 mg/kg of patient body weight per day, with the typical initial range of compound used being 0.3 to 15 mg/kg/day. The dose of taselisib and the dose of the chemotherapeutic agent to be administered may range for each from about 1 mg to about 1000 mg per unit dosage form, or from about 10 mg to about 100 mg per unit dosage form. The doses of taselisib and the chemotherapeutic agent may be administered in a ratio of about 1:50 to about 50:1 by weight, or in a ratio of about 1:10 to about 10:1 by weight.

Acceptable diluents, carriers, excipients and stabilizers are nontoxic to recipients at the dosages and concentrations employed, and include buffers such as phosphate, citrate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, CREMOPHOR EL®, PLURONICS™ or polyethylene glycol (PEG). The active pharmaceutical ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 18th edition, (1995) Mack Publ. Co., Easton, PA.

Sustained-release preparations of taselisib and chemotherapeutic compounds may be prepared. Suitable examples of sustained-release preparations include semipermeable matrices of solid hydrophobic polymers containing taselisib, which matrices are in the form of shaped articles, e.g., films, or microcapsules. Examples of sustained-release matrices include polyesters, hydrogels (for example, poly(2-hydroxyethyl-methacrylate), or poly(vinyl alcohol)), polylactides (US 3773919), copolymers of L-glutamic acid and gamma-ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate) and poly-D (-) 3-hydroxybutyric acid.

The pharmaceutical formulations include those suitable for the administration routes detailed herein. The formulations may conveniently be presented in unit dosage form and may be prepared by any of the methods well known in the art of pharmacy. Techniques and formulations generally are found in Remington's Pharmaceutical Sciences 18th Ed. (1995) Mack Publishing Co., Easton, PA. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

Formulations of taselisib and/or chemotherapeutic agent suitable for oral administration may be prepared as discrete units such as pills, hard or soft e.g., gelatin capsules, cachets, troches, lozenges, aqueous or oil suspensions, dispersible powders or granules, emulsions, syrups or elixirs each containing a predetermined amount of taselisib and/or a chemotherapeutic agent. The amount of taselisib and the amount of chemotherapeutic agent may be formulated in a pill, capsule, solution or suspension as a combined formulation. Alternatively, taselisib and the chemotherapeutic agent may be formulated separately in a pill, capsule, solution or suspension for administration by alternation.

Formulations may be prepared according to any method known to the art for the manufacture of pharmaceutical compositions and such compositions may contain one or more agents including sweetening agents, flavoring agents, coloring agents and preserving agents, in order to provide a palatable preparation. Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom.

Tablet excipients of a pharmaceutical formulation of the invention may include: Filler (or diluent) to increase the bulk volume of the powdered drug making up the tablet; Disintegrants to encourage the tablet to break down into small fragments, ideally individual drug particles, when it is ingested and promote the rapid dissolution and absorption of drug; Binder to ensure that granules and tablets can be formed with the required mechanical strength and hold a tablet together after it has been compressed, preventing it from breaking down into its component powders during packaging, shipping and routine handling; Glidant to improve the flowability of the powder making up the tablet during production; Lubricant to ensure that the tabletting powder does not adhere to the equipment used to press the tablet during manufacture. They improve the flow of the powder mixes through the presses and minimize friction and breakage as the finished tablets are ejected from the equipment; Antiadherent with function similar to that of the glidant, reducing adhesion between the powder making up the tablet and the machine that is used to punch out the shape of the tablet during manufacture; flavor incorporated into tablets to give them a more pleasant taste or to mask an unpleasant one, and colorant to aid identification and patient compliance.

Tablets containing the active ingredient in admixture with non-toxic pharmaceutically acceptable excipient which are suitable for manufacture of tablets are acceptable. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

Pharmaceutical compositions may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may be a solution or a suspension in a non-toxic parenterally acceptable diluent or solvent, such as a solution in 1,3-butanediol or prepared from a lyophilized powder. Among the acceptable vehicles and solvents that may be employed are water, Ringer's solution and isotonic sodium chloride solution. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables.

The formulations may be packaged in unit-dose or multi-dose containers, for example sealed ampoules and vials, and may be stored in a freeze-dried (lyophilized) condition requiring only the addition of the sterile liquid carrier, for example water, for injection immediately prior to use. Extemporaneous injection solutions and suspensions are

prepared from sterile powders, granules and tablets of the kind previously described. Preferred unit dosage formulations are those containing a daily dose or unit daily sub-dose, as herein above recited, or an appropriate fraction thereof, of the active ingredient.

The invention further provides veterinary compositions comprising at least one active ingredient as above defined together with a veterinary carrier therefore. Veterinary carriers are materials useful for the purpose of administering the composition and may be solid, liquid or gaseous materials which are otherwise inert or acceptable in the veterinary art and are compatible with the active ingredient. These veterinary compositions may be administered parenterally, orally or by any other desired route.

COMBINATION THERAPY

Taselisib may be employed in combination with certain chemotherapeutic agents for the treatment of a hyperproliferative disorder, including solid tumor or hematopoietic malignancy, along with pre-malignant and non-neoplastic or non-malignant hyperproliferative disorders. In certain embodiments, taselisib is combined with a chemotherapeutic agent in a single formulation as a single tablet, pill, capsule, or solution for simultaneous administration of the combination. In other embodiments, taselisib and the chemotherapeutic agent are administered according to a dosage regimen or course of therapy in separate formulations as separate tablets, pills, capsules, or solutions for sequential administration of taselisib and the chemotherapeutic agent selected from 5-FU, docetaxel, eribulin, gemcitabine, GDC-0973, GDC-0623, paclitaxel, tamoxifen, fulvestrant, dexamethasone, pertuzumab, trastuzumab emtansine, trastuzumab and letrozole. The chemotherapeutic agent has anti-hyperproliferative properties or is useful for treating the hyperproliferative disorder. The combination of taselisib and chemotherapeutic agent may have synergistic properties. The chemotherapeutic agent of the pharmaceutical combination formulation or dosing regimen preferably has complementary activities taselisib, and such that they do not adversely affect each other. Such compounds of the therapeutic combination may be administered in amounts that are effective for the purpose intended. In one embodiment, a pharmaceutical formulation of this invention comprises taselisib and a chemotherapeutic agent such as described herein. In another embodiment, the therapeutic combination is administered by a dosing regimen wherein the therapeutically effective amount of taselisib is administered in a range from twice daily to once every three weeks (q3wk), and the therapeutically effective amount of the chemotherapeutic agent is administered separately, in alternation, in a range from twice daily to once every three weeks.

Therapeutic combinations of the invention include taselisib, and a chemotherapeutic agent selected from 5-FU, docetaxel, eribulin, gemcitabine, GDC-0973, GDC-0623, paclitaxel, tamoxifen, fulvestrant, dexamethasone, pertuzumab, trastuzumab emtansine, trastuzumab and letrozole, for separate, simultaneous or sequential use in the treatment of a hyperproliferative disorder.

The combination therapy may be administered as a simultaneous or sequential regimen. When administered sequentially, the combination may be administered in two or more administrations. The combined administration includes coadministration, using separate formulations or a single pharmaceutical formulation, and consecutive administration in either order, wherein preferably there is a time period while both (or all) active agents simultaneously exert their biological activities.

Suitable dosages for any of the above coadministered agents are those presently used and may be lowered due to the combined action (synergy) of the newly identified agent and other chemotherapeutic agents or treatments, such as to increase the therapeutic index or mitigate toxicity or other side-effects or consequences.

In a particular embodiment of anti-cancer therapy, the therapeutic combination may be combined with surgical therapy and radiotherapy, as adjuvant therapy. Combination therapies according to the present invention include the administration of taselisib and one or more other cancer treatment methods or modalities. The amounts of taselisib and the chemotherapeutic agent(s) and the relative timings of administration will be selected in order to achieve the desired combined therapeutic effect.

ADMINISTRATION OF PHARMACEUTICAL COMPOSITIONS

The therapeutic combinations of the invention may be administered by any route appropriate to the condition to be treated. Suitable routes include oral, parenteral (including subcutaneous, intramuscular, intravenous, intraarterial, inhalation, intradermal, intrathecal, epidural, and infusion techniques), transdermal, rectal, nasal, topical (including buccal and sublingual), vaginal, intraperitoneal, intrapulmonary and intranasal. Topical administration can also involve the use of transdermal administration such as transdermal patches or iontophoresis devices. Formulation of drugs is discussed in Remington's Pharmaceutical Sciences, 18th Ed., (1995) Mack Publishing Co., Easton, PA. Other examples of drug formulations can be found in Liberman, H. A. and Lachman, L., Eds., Pharmaceutical Dosage Forms, Marcel Decker, Vol 3, 2nd Ed., New York, NY. For local immunosuppressive treatment, the compounds may be administered by intralesional administration, including perfusing or otherwise contacting the graft with the inhibitor before transplantation. It

will be appreciated that the preferred route may vary with for example the condition of the recipient. Where the compound is administered orally, it may be formulated as a pill, capsule, tablet, etc. with a pharmaceutically acceptable carrier, glidant, or excipient. Where the compound is administered parenterally, it may be formulated with a pharmaceutically acceptable parenteral vehicle or diluent, and in a unit dosage injectable form, as detailed below.

A dose to treat human patients may range from about 1 mg to about 1000 mg of taselisib, such as about 5 mg to about 20 mg of the compound. A dose may be administered once a day (QD), twice per day (BID), or more frequently, depending on the pharmacokinetic (PK) and pharmacodynamic (PD) properties, including absorption, distribution, metabolism, and excretion of the particular compound. In addition, toxicity factors may influence the dosage and administration dosing regimen. When administered orally, the pill, capsule, or tablet may be ingested twice daily, daily or less frequently such as weekly or once every two or three weeks for a specified period of time. The regimen may be repeated for a number of cycles of therapy.

ARTICLES OF MANUFACTURE

In another example, an article of manufacture, or "kit", containing taselisib useful for the treatment of the diseases and disorders described above is provided. In one example, the kit comprises a container comprising taselisib. The kit may further comprise a label or package insert, on or associated with the container. The term "package insert" is used to refer to instructions customarily included in commercial packages of therapeutic products, that contain information about the indications, usage, dosage, administration, contraindications and/or warnings concerning the use of such therapeutic products. Suitable containers include, for example, bottles, vials, syringes, blister pack, etc. The container may be formed from a variety of materials such as glass or plastic. The container may hold taselisib or a formulation thereof which is effective for treating the condition and may have a sterile access port (for example, the container may be an intravenous solution bag or a vial having a stopper pierceable by a hypodermic injection needle). At least one active agent in the composition is taselisib. The label or package insert indicates that the composition is used for treating the condition of choice, such as cancer. In one example, the label or package inserts indicates that the composition comprising a Formula I compound can be used to treat a disorder resulting from abnormal cell growth. The label or package insert may also indicate that the composition can be used to treat other disorders. Alternatively, or additionally, the article of manufacture may further comprise a second container comprising a pharmaceutically acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The kit may further comprise directions for the administration of taselisib and, if present, the second pharmaceutical formulation. For example, if the kit comprises a first composition comprising taselisib and a second pharmaceutical formulation, the kit may further comprise directions for the simultaneous, sequential or separate administration of the first and second pharmaceutical compositions to a patient in need thereof.

In another example, the kits are suitable for the delivery of solid oral forms of taselisib, such as tablets or capsules. Such a kit preferably includes a number of unit dosages. Such kits can include a card having the dosages oriented in the order of their intended use. An example of such a kit is a "blister pack". Blister packs are well known in the packaging industry and are widely used for packaging pharmaceutical unit dosage forms. If desired, a memory aid can be provided, for example in the form of numbers, letters, or other markings or with a calendar insert, designating the days in the treatment schedule in which the dosages can be administered.

According to one example, a kit may comprise (a) a first container with taselisib contained therein; and optionally (b) a second container with a second pharmaceutical formulation contained therein, wherein the second pharmaceutical formulation comprises a second compound with anti-hyperproliferative activity. Alternatively, or additionally, the kit may further comprise a third container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Where the kit comprises taselisib and a second therapeutic agent, i.e. the chemotherapeutic agent, the kit may comprise a container for containing the separate compositions such as a divided bottle or a divided foil packet, however, the separate compositions may also be contained within a single, undivided container. Typically, the kit comprises directions for the administration of the separate components. The kit form is particularly advantageous when the separate components are preferably administered in different dosage forms (e.g., oral and parenteral), are administered at different dosage intervals, or when titration of the individual components of the combination is desired by the prescribing physician.

EXAMPLES

Example 1 p110α, (alpha) PI3K Binding Assay

Binding Assays: Initial polarization experiments were performed on an Analyst HT 96-384 (Molecular Devices Corp, Sunnyvale, CA.). Samples for fluorescence polarization affinity measurements were prepared by addition of 1:3 serial dilutions of p110alpha PI3K (Upstate Cell Signaling Solutions, Charlottesville, VA) starting at a final concentration of 20ug/mL in polarization buffer (10 mM Tris pH 7.5, 50 mM NaCl, 4mM $MgCl_2$, 0.05% Chaps, and 1 mM DTT) to 10mM $PIP_2$ (Echelon-Inc., Salt Lake City, UT.) final concentration. After an incubation time of 30 minutes at room temperature, the reactions were stopped by the addition of GRP-1 and PIP3-TAMRA probe (Echelon-Inc., Salt Lake City, UT.) 100 nM and 5 nM final concentrations respectively. Read with standard cut-off filters for the rhodamine fluorophore (λex = 530 nm; λem = 590 nm) in 384-well black low volume Proxiplates® (PerkinElmer, Wellesley, MA.) Fluorescence polarization values were plotted as a function of the protein concentration. $EC_{50}$ values were obtained by fitting the data to a four-parameter equation using KaleidaGraph® software (Synergy software, Reading, PA). This experiment also establishes the appropriate protein concentration to use in subsequent competition experiments with inhibitors.

Inhibitor $IC_{50}$ values were determined by addition of the 0.04mg/mL p110alpha PI3K (final concentration) combined with $PIP_2$ (10mM final concentration) to wells containing 1:3 serial dilutions of the antagonists in a final concentration of 25mM ATP (Cell Signaling Technology, Inc., Danvers, MA) in the polarization buffer. After an incubation time of 30 minutes at room temperature, the reactions were stopped by the addition of GRP-1 and PIP3-TAMRA probe (Echelon-Inc., Salt Lake City, UT.) 100nM and 5nM final concentrations respectively. Read with standard cut-off filters for the rhodamine fluorophore (λex = 530 nm; λem = 590 nm) in 384-well black low volume Proxiplates® (PerkinElmer, Wellesley, MA.) Fluorescence polarization values were plotted as a function of the antagonist concentration, and the $IC_{50}$ values were obtained by fitting the data to a 4-parameter equation in Assay Explorer software (MDL, San Ramon, CA.).

Alternatively, inhibition of PI3K was determined in a radiometric assay using purified, recombinant enzyme and ATP at a concentration of 1μM (micromolar). The compound was serially diluted in 100% DMSO. The kinase reaction was incubated for 1 h at room temperature, and the reaction was terminated by the addition of PBS. $IC_{50}$ values were subsequently determined using sigmoidal dose-response curve fit (variable slope).

Example 2 *In Vitro* Cell Proliferation Assay

Cell Culture: Cell lines were grown under standard tissue culture conditions in RPMI media with 10% fetal bovine serum, 100 U/mL penicillin, and 100 μg/mL streptomycin. HCC-1954 and HDQ-P1 are breast cancer cell lines (American Type Culture Collection; Manassas, VA. HCC-1954 and HDQ-P1 cells were placed in each well of a 6-well tissue culture plate at 800,00 cells/well and incubated at 37°C overnight. Cells were incubated with the indicated concentrations of each compound for 24 hours. Following incubation, cells were washed once with cold phosphate-buffered saline (PBS) and lysed in Biosource™ Cell Extraction Buffer (Invitrogen; Carlsbad, CA) supplemented with protease inhibitors (F. Hoffman-LaRoche; Mannheim, Germany), 1 mM phenylmethylsulfonyl fluoride, and Phosphatase Inhibitor Cocktails 1 and 2 (Sigma-Aldrich; St. Louis, MO). Protein concentrations were determined using the Pierce BCA Protein Assay Kit (Thermo Fisher Scientific; Rockford, IL).

Efficacy of PI3K-binding compounds was measured by a cell proliferation assay employing the following protocol (Mendoza et al (2002) Cancer Res. 62:5485-5488).

The CellTiter-Glo® Luminescent Cell Viability Assay is a homogeneous method to determine the number of viable cells in culture based on quantitation of the ATP present, which signals the presence of metabolically active cells. The CellTiter-Glo® Assay is designed for use with multiwell plate formats, making it ideal for automated high-throughput screening (HTS), cell proliferation and cytotoxicity assays. The homogeneous assay procedure involves adding a single reagent (CellTiter-Glo® Reagent) directly to cells cultured in serum-supplemented medium. Cell washing, removal of medium or multiple pipetting steps are not required. The Cell Titer-Glo® Luminescent Cell Viability Assay, including reagents and protocol are commercially available (Promega Corp., Madison, WI, Technical Bulletin TB288).

The assay assesses the ability of compounds to enter cells and inhibit cell proliferation. The assay principle is based on the determination of the number of viable cells present by quantitating the ATP present in a homogenous assay where addition of the Cell Titer-Glo® reagent results in cell lysis and generation of a luminescent signal through the luciferase reaction. The luminescent signal is proportional to the amount of ATP present.

Procedure: Day 1 - Seed Cell Plates (384-well black, clear bottom, microclear, TC plates with lid from Falcon #353962), Harvest cells, Seed cells at 1000 cells per 54μl per well into 384 well Cell Plates for 3 days assay. Cell Culture Medium: RPMI or DMEM high glucose, 10% Fetal Bovine Serum, 2mM L-Glutamine, P/S. Incubate O/N (overnight) at 37 °C, 5% $CO_2$.

Day 2 - Add Drug to Cells, Compound Dilution, DMSO Plates (serial 1:2 for 9 points). Add 20 μl of compound at 10 mM in the 2nd column of 96 well plate. Perform serial 1:2 across the plate (10μl + 20μl 100% DMSO) for a total

of 9 points using Precision Media Plates 96-well conical bottom polypropylene plates from Nunc (cat.# 249946) (1:50 dilution). Add 147μl of Media into all wells. Transfer 3μl of DMSO + compound from each well in the DMSO Plate to each corresponding well on Media Plate using Rapidplate® (Caliper, a Perkin-Elmer Co.). For 2 drug combination studies, transfer one drug 1.5μl of DMSO + compound from each well in the DMSO Plate to each corresponding well on Media Plate using Rapidplate. Then, transfer another drug 1.5 μl to the medium plate.

Drug Addition to Cells, Cell Plate (1:10 dilution): Add 6μl of media + compound directly to cells (54 μl of media on the cells already). Incubate 3 days at 37 °C, 5% $CO_2$ in an incubator that will not be opened often.

Day 5 - Develop Plates, Thaw Cell Titer Glo Buffer at room temperature: Remove Cell Plates from 37 °C and equilibrate to room temperature for about 30 minutes. Add Cell Titer-Glo® Buffer to Cell Titer-Glo® Substrate (bottle to bottle). Add 30 μl Cell Titer-Glo® Reagent (Promega cat.# G7572) to each well of cells. Place on plate shaker for about 30 minutes. Read luminescence on Analyst HT Plate Reader (half second per well).

Cell viability assays and combination assays: Cells were seeded at 1000-2000 cells/well in 384-well plates for 16 h. On day two, nine serial 1:2 compound dilutions were made in DMSO in a 96 well plate. The compounds were further diluted into growth media using a Rapidplate® robot (Zymark Corp., Hopkinton, MA). The diluted compounds were then added to quadruplicate wells in 384-well cell plates and incubated at 37 °C and 5% $CO_2$. After 4 days, relative numbers of viable cells were measured by luminescence using Cell Titer-Glo® (Promega) according to the manufacturer's instructions and read on a Wallac Multilabel Reader® (PerkinElmer, Foster City). EC50 values were calculated using Prism® 4.0 software (GraphPad, San Diego). Drugs in combination assays were dosed starting at 4X $EC_{50}$ concentrations. If cases where the EC50 of the drug was >2.5 μM, the highest concentration used was 10 μM. The PI3K-binding compound and chemotherapeutic agents were added simultaneously or separated by 4 hours (one before the other) in all assays.

An additional exemplary in vitro cell proliferation assay includes the following steps:

1. An aliquot of 100 μl of cell culture containing about $10^4$ cells (see Table 3 for cell lines and tumor type) in medium was deposited in each well of a 384-well, opaque-walled plate.

2. Control wells were prepared containing medium and without cells.

3. The compound was added to the experimental wells and incubated for 3-5 days.

4. The plates were equilibrated to room temperature for approximately 30 minutes.

5. A volume of CellTiter-Glo® Reagent equal to the volume of cell culture medium present in each well was added.

6. The contents were mixed for 2 minutes on an orbital shaker to induce cell lysis.

7. The plate was incubated at room temperature for 10 minutes to stabilize the luminescence signal.

8. Luminescence was recorded and reported in graphs as RLU = relative luminescence units.

9. Analyze using the Chou and Talalay combination method and Dose-Effect Analysis with CalcuSyn® software (Biosoft, Cambridge, UK) in order to obtain a Combination Index.

Alternatively, cells were seeded at optimal density in a 96 well plate and incubated for 4 days in the presence of test compound. Alamar Blue™ was subsequently added to the assay medium, and cells were incubated for 6 h before reading at 544 nm excitation, 590nm emission. $EC_{50}$ values were calculated using a sigmoidal dose response curve fit.

Alternatively, Proliferation/Viability was analyzed after 48 hr of drug treatment using Cell Titer-Glo® reagent (Promega Inc., Madison, WI). DMSO treatment was used as control in all viability assays. $IC_{50}$ values were calculated using XL fit software (IDBS, Alameda, CA)

The cell lines were obtained from either ATCC (American Type Culture Collection, Manassas, VA) or DSMZ (Deutsche Sammlung von Mikroorganismen und Zellkulturen GmbH, Braunschweig, DE). Cells were cultured in RPMI 1640 medium supplemented with 10% fetal bovine serum, 100 units/ml penicillin, 2 mM L-glutamine, and 100 mg/ml streptomycin (Life Technology, Grand Island, NY) at 37 °C under 5% $CO_2$.

Example 3 SW48 Isogenic Cell Line Viability Assay

Cell culture. Cell lines were obtained from the American Type Culture Collection (ATCC, VA) or from the Deutsche Sammlung von Mikroorganismen und Zellkulturen GmbH (DMSZ, Germany). Lines were cultured in DMEM

or RPMI supplemented with 10% fetal bovine serum, 100 units/ml penicillin, and 100 μg/ml streptomycin at 37°C under 5% $CO_2$. MCF7-neo/HER2 is an in vivo selected tumor cell line developed at Genentech and derived from the parental MCF7 human breast cancer cell line. Isogenic cell lines (SW48 Parental, SW48 E545K, and SW48 H1047R) were licensed from Horizon Discovery Ltd. (Cambridge, UK) and cultured in McCoy's 5A supplemented with 10% fetal bovine serum, 100 units/ml penicillin, and 100 μg/ml streptomycin at 37°C under 5% CO2.

Cell viability assays. 384-well plates were seeded with 1000 to 2000 cells/well in a volume of 54 μl per well followed by incubation at 37°C under 5% $CO_2$ overnight (~16 hours). Compounds were diluted in DMSO to generate the desired stock concentrations then added in a volume of 6 μL per well. All treatments were tested in quadruplicate. After 4 days incubation, relative numbers of viable cells were estimated using CellTiter-Glo (Promega, Madison, WI) and total luminescence was measured on a Wallac Multilabel Reader (PerkinElmer, Foster City,CA). The concentration of drug resulting in 50% inhibition of cell viability ($IC_{50}$) or 50% maximal effective concentration ($EC_{50}$) was determined using Prism software (GraphPad, La Jolla, CA). For cell lines that failed to achieve an $IC_{50}$ the highest concentration tested (10 μM) is listed.

Example 4 *In Vivo* Mouse Tumor Xenograft Efficacy

Mice: Female severe combined immunodeficiency mice (Fox Chase SCID®, C.B-17/IcrHsd, Harlan) or nude mice (Taconic Farms, Harlan) were 8 to 9 weeks old and had a BW range of 15.1 to 21.4 grams on Day 0 of the study. The animals were fed ad libitum water (reverse osmosis, 1 ppm Cl) and NIH 31 Modified and Irradiated Lab Diet® consisting of 18.0% crude protein, 5.0% crude fat, and 5.0% crude fiber. The mice were housed on irradiated ALPHA-Dri® bed-o'cobs® Laboratory Animal Bedding in static microisolators on a 12-hour light cycle at 21-22 °C (70-72 °F) and 40-60% humidity. PRC specifically complies with the recommendations of the Guide for Care and Use of Laboratory Animals with respect to restraint, husbandry, surgical procedures, feed and fluid regulation, and veterinary care. The animal care and use program at PRC is accredited by the Association for Assessment and Accreditation of Laboratory Animal Care International (AAALAC), which assures compliance with accepted standards for the care and use of laboratory animals.

Tumor Implantation: Xenografts were initiated with cancer cells. Cells were cultured in RPMI 1640 medium supplemented with 10% fetal bovine serum, 2 mM glutamine, 100 units/mL penicillin, 100 μg/mL streptomycin sulfate and 25 μg/mL gentamicin. The cells were harvested during exponential growth and resuspended in phosphate buffered saline (PBS) at a concentration of 5 x $10^6$ or 10 x $10^6$ cells/mL depending on the doubling time of the cell line. Tumor cells were implanted subcutaneously in the right flank, and tumor growth was monitored as the average size approached the target range of 100 to 150 mm3. Twenty-one days after tumor implantation, designated as Day 0 of the study, the mice were placed into four groups each consisting of ten mice with individual tumor volumes ranging from 75-172 mm3 and group mean tumor volumes from 120-121 mm3 (see Appendix A). Volume was calculated using the formula:

$$\text{Tumor Volume (mm}^3) = (w^2 \text{ x l})/2,$$

where w = width and l = length in mm of a tumor. Tumor weight may be estimated with the assumption that 1 mg is equivalent to 1 mm3 of tumor volume.

Therapeutic Agents: A PI3K-binding compound was supplied as a dry powder in salt form, which contained 73% active agent, and was stored at room temperature protected from light. Drug doses were prepared weekly in 0.5% methylcellulose: 0.2% Tween 80 in deionized water ("Vehicle") and stored at 4°C. The salt form containing 73% active agent was accounted for in the formulation of G-033829 doses. Doses of the PI3K-binding compound were prepared on each day of dosing by diluting an aliquot of the stock with sterile saline (0.9% NaCl). All doses were formulated to deliver the stated mg/kg dosage in a volume of 0.2 mL per 20 grams of body weight (10 mL/kg).

Treatment: All doses were scaled to the body weights of the individual animals and were provided by the route indicated in each of the figures.

Endpoint: Tumor volume was measured in 2 dimensions (length and width), using Ultra Cal IV calipers (Model 54 10 111; Fred V. Fowler Company), as follows: tumor volume ($mm^3$) = (length x $width^2$) × 0.5 and analyzed using Excel version 11.2 (Microsoft Corporation). A linear mixed effect (LME) modeling approach was used to analyze the repeated measurement of tumor volumes from the same animals over time (Pinheiro J, et al. nlme: linear and nonlinear mixed effects models. R package version 3.1 92. 2009; Tan N, et al. Navitoclax enhances the efficacy of taxanes in non-small cell lung cancer models. Clin. Cancer Res. 2011;17(6):1394-1404). This approach addresses both repeated measurements and modest dropouts due to any non-treatment-related death of animals before study end. Cubic regression splines were used to fit a nonlinear profile to the time courses of log2 tumor volume at each dose level. These nonlinear profiles were then related to dose within the mixed model. Tumor growth inhibition as a percentage of vehicle control (% TGI) was calculated as the percentage of the area under the fitted curve (AUC) for the respective dose group per

day in relation to the vehicle, using the following formula: % TGI = 100 × (1 - $AUC_{dose}$/ $AUC_{veh}$). Using this formula, a TGI value of 100% indicates tumor stasis, a TGI value of > 1% but < 100% indicates tumor growth delay, and a TGI value of > 100% indicates tumor regression. Partial response (PR) for an animal was defined as a tumor regression of > 50% but < 100% of the starting tumor volume. Complete response (CR) was defined as 100% tumor regression (i.e., no measurable tumor) on any day during the study.

Toxicity: Animals were weighed daily for the first five days of the study and twice weekly thereafter. Animal body weights were measured using an Adventurer Pro® AV812 scale (Ohaus Corporation). Percent weight change was calculated as follows: body weight change (%) = [($weight_{day\ new}$ - $weight_{day\ 0}$)/$weight_{day\ 0}$] × 100. The mice were observed frequently for overt signs of any adverse, treatment- related side effects, and clinical signs of toxicity were recorded when observed. Acceptable toxicity is defined as a group mean body weight (BW) loss of less than 20% during the study and not more than one treatment-related (TR) death among ten treated animals. Any dosing regimen that results in greater toxicity is considered above the maximum tolerated dose (MTD). A death is classified as TR if attributable to treatment side effects as evidenced by clinical signs and/or necropsy, or may also be classified as TR if due to unknown causes during the dosing period or within 10 days of the last dose. A death is classified as NTR if there is no evidence that death was related to treatment side effects.

Example 5 Western Blot Analysis of p110a protein

Protein Assays: Protein concentration was determined using the Pierce BCA Protein Assay Kit (Rockford, IL). For immunoblots, equal protein amounts were separated by electrophoresis through NuPage Bis-Tris 4-12% gradient gels (Invitrogen; Carlsbad, CA); proteins were transferred onto Nitrocellulose membranes using the IBlot system and protocol from InVitrogen. Antibodies to p110alpha, and phospho-Akt (Ser473), were obtained from Cell Signaling (Danvers, MA). Antibodies to beta-actin and GAPDH were from Sigma.

For Western blots, equal amounts of protein were separated by electrophoresis through NuPage Tris-acetate 3-18% gradient gels (Invitrogen). Proteins were transferred onto nitrocellulose pore membranes using the iBlot system and protocol from Invitrogen (Carlsbad, CA). pAkt ($Ser^{473}$),and p110 alpha, and p85 antibodies were obtained from Cell Signaling Technology (Danvers, MA). Beta Actin antibody was obtained from Sigma-Aldrich (St. Louis, MO). Specific antigen-antibody interaction was detected with a horseradish peroxidase-conjugated secondary antibody IgG using enhanced chemiluminescence Western blotting detection reagents (GE Healthcare Life Sciences, Pittsburgh, PA).

Western blot analysis using polyclonal rabbit anti-PI3K p110 alpha antibody was conducted following the manufacturer's protocol (PI3 Kinase p110α Antibody #4255, PI3 Kinase p110α (C73F8) Rabbit mAb #4249, Cell Signaling Technology). Monoclonal and polyclonal PI3K p110 alpha antibodies are commercially available (Santa Cruz Biotechnology). See Popkie et al (2010) J Biol Chem.; 285(53):41337-47; Yoshioka et al (2012) Nat Med. Oct;18(10):1560-9; Biswas et al (2013) J Biol Chem. Jan 25;288(4):2325-39; Ramadani et al (2010) Sci Signal. Aug 10;3(134).

Western Blotting Protocol (Cell Signaling Technology):

For western blots, incubate membrane with diluted primary antibody in 5% w/v BSA, 1X TBS, 0.1% Tween® 20 at 4 °C with gentle shaking, overnight.

Dilutions:

Western Blotting, 1:1000

Immunoprecipitation, 1:50

Immunohistochemistry, 1:400

Supplied in 10 mM sodium HEPES (pH 7.5), 150 mM NaCl, 100 μg/ml BSA, 50% glycerol and less than 0.02% sodium azide. Store at -20 °C. Do not aliquot the antibody.

A. Solutions and Reagents

NOTE: Prepare solutions with reverse osmosis deionized (RODI) or equivalent grade water.

1. 20X Phosphate Buffered Saline (PBS): (#9808) To prepare 1 L 1X PBS: add 50 ml 20X PBS to 950 ml $dH_2O$, mix.

2. 10X Tris Buffered Saline (TBS): (#12498) To prepare 1 L 1X TBS: add 100 ml 10X to 900 ml $dH_2O$, mix.

3. 1X SDS Sample Buffer: Blue Loading Pack (#7722) or Red Loading Pack (#7723) Prepare fresh 3X reducing loading buffer by adding 1/10 volume 30X DTT to 1 volume of 3X SDS loading buffer. Dilute to 1X with $dH_2O$.

4. 10X Tris-Glycine SDS Running Buffer: (#4050) To prepare 1 L 1X running buffer: add 100 ml 10X running buffer to 900 ml $dH_2O$, mix.

5. 10X Tris-Glycine Transfer Buffer: (#12539) To prepare 1 L 1X Transfer Buffer: add 100 ml 10X Transfer Buffer to 200 ml methanol + 700 ml $dH_2O$, mix.

6. 10X Tris Buffered Saline with Tween® 20 (TBST): (#9997) To prepare 1 L 1X TBST: add 100 ml 10X TBST to 900 ml $dH_2O$, mix.

7. Nonfat Dry Milk: (#9999).

8. Blocking Buffer: 1X TBST with 5% w/v nonfat dry milk; for 150 ml, add 7.5 g nonfat dry milk to 150 ml 1X TBST and mix well.

9. Wash Buffer: (#9997) 1X TBST.

10. Bovine Serum Albumin (BSA): (#9998).

11. Primary Antibody Dilution Buffer: 1X TBST with 5% BSA; for 20 ml, add 1.0 g BSA to 20 ml 1X TBST and mix well.

12. Biotinylated Protein Ladder Detection Pack: (#7727).

13. Prestained Protein Marker, Broad Range (Premixed Format): (#7720).

14. Blotting Membrane and Paper: (Cell Signaling Technology #12369) This protocol has been optimized for nitrocellulose membranes. Pore size 0.2 μm is generally recommended.

15. Secondary Antibody Conjugated to HRP: Anti-rabbit IgG, HRP-linked Antibody (#7074).

16. Detection Reagent: SignalFire™ ECL Reagent (#6883).

B. Protein Blotting

A general protocol for sample preparation.

1. Treat cells by adding fresh media containing regulator for desired time.

2. Aspirate media from cultures; wash cells with 1X PBS; aspirate.

3. Lyse cells by adding 1X SDS sample buffer (100 μl per well of 6-well plate or 500 μl for a 10 cm diameter plate). Immediately scrape the cells off the plate and transfer the extract to a microcentrifuge tube. Keep on ice.

4. Sonicate for 10-15 sec to complete cell lysis and shear DNA (to reduce sample viscosity).

5. Heat a 20 μl sample to 95-100°C for 5 min; cool on ice.

6. Microcentrifuge for 5 min.

7. Load 20 μl onto SDS-PAGE gel (10 cm x 10 cm).
NOTE: Loading of prestained molecular weight markers (#7720, 10 μl/lane) to verify electrotransfer and biotinylated protein ladder (#7727, 10 μl/lane) to determine molecular weights are recommended.

8. Electrotransfer to nitrocellulose membrane (#12369).

C. Membrane Blocking and Antibody Incubations

NOTE: Volumes are for 10 cm x 10 cm (100 $cm^2$) of membrane; for different sized membranes, adjust volumes accordingly.

I. Membrane Blocking

1. (Optional) After transfer, wash nitrocellulose membrane with 25 ml TBS for 5 min at room temperature.

2. Incubate membrane in 25 ml of blocking buffer for 1 hr at room temperature.

3. Wash three times for 5 min each with 15 ml of TBST.

II. Primary Antibody Incubation

1. Incubate membrane and primary antibody (at the appropriate dilution and diluent as recommended in the product datasheet) in 10 ml primary antibody dilution buffer with gentle agitation overnight at 4°C.

2. Wash three times for 5 min each with 15 ml of TBST.

3. Incubate membrane with Anti-rabbit IgG, HRP-linked Antibody (#7074 at 1:2000) and anti-biotin, HRP-linked Antibody (#7075 at 1:1000-1:3000) to detect biotinylated protein markers in 10 ml of blocking buffer with gentle agitation for 1 hr at room temperature.

4. Wash three times for 5 min each with 15 ml of TBST.

5. Proceed with detection (Section D).

D. Detection of Proteins

Directions for Use:

1. Wash membrane-bound HRP (antibody conjugate) three times for 5 minutes in TBST.

2. Prepare 1X SignalFire™ ECL Reagent (#6883) by diluting one part 2X Reagent A and one part 2X Reagent B (e.g. for 10 ml, add 5 ml Reagent A and 5 ml Reagent B). Mix well.

3. Incubate substrate with membrane for 1 minute, remove excess solution (membrane remains wet), wrap in plastic and expose to X-ray film.
* Avoid repeated exposure to skin.

Western Blot Reprobing Protocol

Reprobing of an existing membrane is a convenient means to immunoblot for multiple proteins independently when only a limited amount of sample is available. It should be noted that for the best possible results a fresh blot is always recommended. Reprobing can be a valuable method but with each reprobing of a blot there is potential for increased background signal. Additionally, it is recommended that you verify the removal of the first antibody complex prior to reprobing so that signal attributed to binding of the new antibody is not leftover signal from the first immunoblotting experiment. This can be done by re-exposing the blot to ECL reagents and making sure there is no signal prior to adding the next primary antibody.

A. Solutions and Reagents

NOTE: Prepare solutions with reverse osmosis deionized (RODI) or equivalently purified water.

1. Wash Buffer: Tris Buffered Saline with Tween® 20 (TBST-10X) (#9997)

2. Stripping Buffer: To prepare 100 ml, mix 0.76 g Tris base, 2 g SDS and 700 μl β-mercaptoethanol. Bring to 100 ml with deionized $H_2O$. Adjust pH to 6.8 with HCl.

B. Protocol

1. After film exposure, wash membrane four times for 5 min each in TBST. Best results are obtained if the membrane is not allowed to dry.

2. Incubate membrane for 30 min at 50°C in stripping buffer (with slight agitation).

3. Wash membrane six times for 5 min each in TBST.

4. (Optional) To assure that the original signal is removed, wash membrane twice for 5 min each with 10 ml of TBST. Incubate membrane with LumiGLO® with gentle agitation for 1 min at room temperature. Drain membrane of excess developing solution. Do not let dry. Wrap in plastic wrap and expose to x-ray film.

5. Wash membrane again four times for 5 min each in TBST.

6. The membrane is now ready to reuse. Start detection at the "Membrane Blocking and Antibody Incubations" step in the Western Immunoblotting Protocol.

Example 6 Immunohistochemistry (IHC) detection of mutant p110 alpha

To determine mutant p110 alpha levels in patient tumor biopsy samples, various diagnostic assays are available. In one embodiment, mutant p110 alpha levels may be analyzed by immunohistochemistry (IHC). Paraffin-embedded tissue sections from a tumor biopsy may be subjected to the IHC assay and accorded a staining intensity criteria as follows:

Score 0 - no staining is observed or membrane staining is observed in less than 10% of tumor cells.

Score 1+ - a faint/barely perceptible membrane staining is detected in more than 10% of the tumor cells. The cells are only stained in part of their membrane.

Score 2+ - a weak to moderate complete membrane staining is observed in more than 10% of the tumor cells.

Score 3+ - a moderate to strong complete membrane staining is observed in more than 10% of the tumor cells.

Tumor samples may be characterized according to their scores.

In some embodiments, the mutant p110 alpha biomarker is detected using an anti-mutant p110 alpha antibody. In some embodiments, the mutant p110 alpha biomarker is detected as a weak staining intensity by IHC. In some embodiments, the mutant p110 alpha biomarker is detected as a moderate staining intensity by IHC. In some embodiments, the mutant p110 alpha biomarker is detected as a strong staining intensity by IHC.

The Ventana Benchmark XT® or Benchmark Ultra® system may be used to perform IHC staining.

Example 7 Mass spectrometry analysis of p110 alpha cell lines

Liquid chromatography-tandem mass spectrometry (LC-MS/MS) analysis was performed on p110alpha (PIK3CA) protein immunoprecipitated from three cell lines each treated for 24 hr with either DMSO (vehicle) or GDC-0032 (500 nM): HCC1954, HCC202 and HDQP1. Each experiment was performed beginning with 4-6 mg protein lysate per cell line/treatment (total of 6 samples/experiment) for n=4 biological replicates. One gel region per sample, corresponding to the expected migration of PIK3CA was excised based on the migration of purified p110alpha protein in an adjacent lane. Gel pieces were diced into ~1 $mm^3$ pieces and subjected to in-gel digestion as follows. Gel pieces were destained with 50mM ammonium bicarbonate/50% acetonitrile and dehydrated with 100% acetonitrile prior to reduction and alkylation using 50mM dithiothreitol (30 min, 50 °C) and 50mM iodoacetamide (20 min, room temperature), respectively. Gel pieces were again dehydrated, then allowed to reswell in a 20ng/ul trypsin in 50mM ammonium bicarbonate/5%

acetonitrile digestion buffer on ice for 2 hours, and then transferred to a 37°C oven for overnight incubation. Digested peptides were transferred to microcentrifuge tubes and gel pieces were extracted twice, once with 50% acetonitrile/0.5% trifluoroacetic acid, and a second round with 100% acetonitrile. Extracts were combined with digested peptides and speed-vac dried to completion. Samples were reconstituted in 5% formic acid/0.1% heptafluorobutyric acid/0.01% hydrogen peroxide 30 minutes prior to LC-MS/MS analysis.

Samples were analyzed by LC-MS/MS with duplicate injection (with the exception of the first replicate where samples were injected once) on a Thermo LTQ Orbitrap Elite coupled to a Waters nanoAcquity UPLC. Peptides were loaded onto a 0.1mm X 100mm Waters Symmetry C18 column packed with 1.7um BEH-130 resin and separated via a two-stage linear gradient where solvent B (98% acetonitrile, 2% water) was ramped from 5% to 25% over 20 minutes and then from 25% to 50% over 2 minutes. Data was acquired in data dependent mode with Orbitrap full MS scans collected at 60,000 resolution and the top 15 most intense precursors selected for CID MS/MS fragmentation in the ion trap. MS2 spectra were searched using Mascot, both against a concatenated target-decoy Uniprot database of human proteins as well as against a small database containing wild type, E545K and H1047R mutant PIK3CA sequences in order to identify mutant peptides. Spectral matches for the Uniprot search were filtered at a permissive false discovery rate of 10% using linear discriminant analysis prior to manual inspection.

Extracted ion chromatograms and peak area integration for PIK3CA peptides were generated with 10 ppm mass tolerances using in-house software (MSPlorer). Peak area data for each of 14 peptides were normalized to the most abundant peak area among the six samples on a per block basis. In cases where duplicate injections (technical replicates) were available, normalized data for the two replicates were averaged to generate a single normalized peak area per peptide-condition-experiment (i.e. quantified feature) to generate the protein sequence plots.

For statistical analysis, the original, non-normalized peak areas across the four biological replicates were consolidated in R using linear mixed effects modeling (1me4 package) to determine the relative ratio and p-value for the comparison of DMSO (vehicle) versus GDC-0032 (500 nM) treatments per cell line for each of total PIK3CA, wild-type PIK3CA, and mutant PIK3CA. Total PIK3CA was determined based on the data generated from the following peptides:

| | | |
|---|---|---|
| EATLITIK | (residues 39-46; 444.77481 m/z) | (SEQ ID NO.:9) |
| DLNSPHSR | (155-162; 463.22945 m/z) | (SEQ ID NO.:10) |
| LCVLEYQGK | (241-249) | (SEQ ID NO.:11) |
| VCGCDEYFLEK | (254-264; 710.30246 m/z) | (SEQ ID NO.:12) |
| VPCSNPR | (376-382) | (SEQ ID NO.:13) |
| EAGFSYSHAGLSNR | (503-516; 748.35281 m/z) | (SEQ ID NO.:14) |
| YEQYLDNLLVR | (641-651; 713.37540 m/z) | (SEQ ID NO.:15) |
| FGLLLESYCR | (684-693; 629.32042 m/z) | (SEQ ID NO.:16) |
| LINLTDILK | (712-720; 521.83039 m/z). | (SEQ ID NO.:17) |
| QMNDAR | (1042-1047; 375.66360 m/z) | (SEQ ID NO.:18) |
| DPLSEITEQEK | (538-548; 644.81917 m/z) | (SEQ ID NO.:19) |
| DPLSEITK | (538-545; 451.74627 m/z) | (SEQ ID NO.:20) |

For cell line(s) bearing the H1047R mutation (i.e. HCC-1954), wild-type PIK3CA was determined based on the QMNDAHHGGWTTK (1042-1054; 749.82824 m/z) peptide (SEQ ID NO.:7) and mutant PIK3CA based on QMNDAR (1042-1047; 375.66360 m/z) (SEQ ID NO.:18) and HGGWTTK (1048-1054; 393.6983 m/z) (SEQ ID NO.:8) peptides covering the 1047-locus (Figures 24A and 24B). For cell line(s) bearing the E545K mutation (i.e. HCC-202), wild-type PIK3CA was determined based on the DPLSEITEQEK (538-548; 644.81917 m/z) (SEQ ID NO.:19) peptide and mutant PIK3CA based on DPLSEITK (538-545; 451.74627 m/z) (SEQ ID NO.:20) peptide covering the 545-locus. Peptides from these two loci were not considered when determining total PIK3CA. Whenever applicable, cysteine residues within PIK3CA peptides were analyzed in their carbamidomethylated form (+57.021 Da) and methionine residues quantified based on their singly oxidized (Met-sulfoxide) form (+15.9949 Da). $Log_2$ ratios (GDC-0032/DMSO) and the corresponding p-values for total PIK3CA, wild-type PIK3CA, and mutant PIK3CA were used to determine the relative abundances (and associated 95% confidence intervals) of wild-type and mutant PIK3CA in each condition and cell line by applying the conservation of mass principle. Explicitly:

$$\mathrm{WT_PI3KCA_DMSO} + \mathrm{MUT_PI3KCA_DMSO} = \mathrm{total_PI3KCA_DMSO}$$

$$WT_PI3KCA_GDC + MUT_PI3KCA_GDC = total_PI3KCA_GDC$$

$$\text{proportion } WT_PI3KCA = p = WT_PI3KCA / total_PI3KCA$$

$$\text{proportion } MUT_PI3KCA = 1\text{-}p = MUT_PI3KCA / total_PI3KCA$$

Data are plotted as relative intensity values where total_PI3KCA_DMSO is normalized to 1.0 and error bars represent the 95% confidence intervals for each measurement based on the linear effects model.

Although the foregoing invention has been described in some detail by way of illustration and example for purposes of clarity of understanding, the descriptions and examples should not be construed as limiting the scope of the invention. The scope of protection is defined by the appended claims.

## Claims

1. A process of selecting a patient for treatment with taselisib comprising:

(a) treating a biological sample obtained from the patient with taselisib; and
(b) detecting the depletion of p110 alpha protein;

wherein

(i) the depletion of p110 alpha protein in the biological sample identifies a patient who will respond to treatment with taselisib;
(ii) depletion of p110 alpha protein indicates therapeutic responsiveness by a patient to the compound; and
(iii) depletion of p110 alpha protein is measured by binding to an anti-pl 10 alpha antibody or depletion of p110 alpha protein is detected by mass spectroscopy.

**2.** The process of claim 1 wherein binding of the anti-pl 10 alpha antibody to the p110 alpha protein in the sample is determined by western blot analysis, enzyme-linked immunosorbent assay (ELISA), radioimmunoassay (RIA), immunohistochemistry (IHC), fluorescence-activated cell sorting (FACS), or reverse-phase protein array.

**3.** A process of selecting patients with a PIK3CA mutation for treatment with taselisib comprising:

(a) detecting a PIK3CA mutation in a biological sample obtained from the patient; and
(b) comparing the level of p110 alpha in a biological sample obtained from the patient prior to administration of taselisib with the level of p110 alpha in the biological sample obtained from the patient after administration of taselisib,

wherein a depletion in the level of p110 alpha in the biological sample obtained from the patient after administration of taselisib identifies a patient who will respond to treatment with taselisib.

**4.** An in vitro process of selecting a treatment regimen for a patient diagnosed as having cancer, the process comprising contacting a cancer cell of the patient with an effective amount of taselisib, and detecting the level of p110 alpha in response to taselisib, wherein detection of depletion of p110 alpha indicates that the cancer is susceptible to treatment with taselisib, and wherein the treatment regimen comprises administering taselisib to the patient if the cancer is determined to be susceptible to treatment with taselisib.

**5.** The process of claim 4 wherein the cancer cell is a PIK3CA mutant cancer cell.

**6.** Taselisib for use in the treatment of cancer, wherein the patients have been selected according to any one of the methods of claims 1-3.

## Patentansprüche

**1.** Verfahren zum Auswählen eines Patienten für eine Behandlung mit Taselisib, umfassend:

(a) Behandeln einer von dem Patienten erhaltenen biologischen Probe mit Taselisib; und
(b) Nachweisen der Depletion von p110-Alpha-Protein;

wobei

(i) die Depletion von p110-Alpha-Protein in der biologischen Probe einen Patienten identifiziert, der auf eine Behandlung mit Taselisib ansprechen wird;
(ii) die Depletion von p110-Alpha-Protein auf ein therapeutisches Ansprechen eines Patienten auf die Verbindung hindeutet; und
(iii) die Depletion von p110-Alpha-Protein durch die Bindung an einen Anti-p110-Alpha-Antikörper gemessen wird oder die Depletion von p110-Alpha-Protein durch Massenspektrometrie nachgewiesen wird.

**2.** Verfahren nach Anspruch 1, wobei die Bindung des Anti-p110-Alpha-Antikörpers an das p110-Alpha-Protein in der Probe durch Western-Blot-Analyse, enzymgekoppelten Immunadsorptionstest (ELISA), Radioimmuntest (RIA), Immunhistochemie (IHC), fluoreszenzaktivierte Zellsortierung (FACS) oder Umkehrphasen-Proteinanordnung bestimmt wird.

3. Verfahren zum Auswählen von Patienten mit einer PIK3CA-Mutation für eine Behandlung mit Taselisib, umfassend:

(a) Nachweisen einer PIK3CA-Mutation in einer von dem Patienten erhaltenen biologischen Probe; und
(b) Vergleichen der Menge von p110-Alpha in einer von dem Patienten vor der Verabreichung von Taselisib erhaltenen biologischen Probe mit der Menge von p110-Alpha in der von dem Patienten nach der Verabreichung von Taselisib erhaltenen biologischen Probe,

wobei eine Depletion der Menge von p110-Alpha in der von dem Patienten nach der Verabreichung von Taselisib erhaltenen biologischen Probe einen Patienten identifiziert, der auf die Behandlung mit Taselisib ansprechen wird.

4. *In-vitro*-Verfahren zum Auswählen eines Behandlungsschemas für einen Patienten, bei dem Krebs diagnostiziert worden ist, wobei das Verfahren das Inkontaktbringen einer Krebszelle des Patienten mit einer wirksamen Menge von Taselisib und das Nachweisen der Menge von p110-Alpha als Reaktion auf Taselisib umfasst, wobei der Nachweis einer Depletion von p110-Alpha darauf hindeutet, dass der Krebs gegenüber der Behandlung mit Taselisib empfindlich ist, und wobei das Behandlungsschema das Verabreichen von Taselisib an den Patienten umfasst, wenn bestimmt wird, dass der Krebs gegenüber der Behandlung mit Taselisib empfindlich ist.

5. Verfahren nach Anspruch 4, wobei die Krebszelle eine Krebszelle mit einer PIK3CA-Mutation ist.

6. Taselisib zur Verwendung bei der Behandlung von Krebs, wobei die Patienten nach einem der Verfahren der Ansprüche I bis 3 ausgewählt worden sind.

## Revendications

1. Procédé de sélection d'un patient pour un traitement avec du tasélisib comprenant :

(a) le traitement d'un échantillon biologique obtenu du patient avec du tasélisib ; et
(b) la détection de la déplétion de la protéine p110 alpha ;

dans lequel

(i) la déplétion de la protéine p110 alpha dans l'échantillon biologique identifie un patient qui répondra au traitement avec le tasélisib ;
(ii) la déplétion de la protéine p110 alpha indique la sensibilité thérapeutique d'un patient au composé ; et
(iii) la déplétion de la protéine p110 alpha est mesurée par liaison à un anticorps anti-p110 alpha ou la déplétion de la protéine p110 alpha est détectée par spectroscopie de masse.

2. Procédé selon la revendication 1, dans lequel la liaison de l'anticorps anti-p110 alpha à la protéine p110 alpha dans l'échantillon est déterminée par une analyse par transfert de Western, un dosage d'immunoabsorption par enzyme liée (ELISA), un dosage radio-immunologique (RIA), une immunohistochimie (IHC), un tri de cellules activées par fluorescence (FACS) ou une puce à protéines en phase inverse.

3. Procédé de sélection de patients présentant une mutation de PIK3CA pour un traitement avec du tasélisib comprenant :

(a) la détection d'une mutation de PIK3CA dans un échantillon biologique obtenu du patient ; et
(b) la comparaison du taux de p110 alpha dans un échantillon biologique obtenu du patient avant l'administration du tasélisib avec le taux de p110 alpha dans l'échantillon biologique obtenu du patient après l'administration du tasélisib,

dans lequel une déplétion du taux de p110 alpha dans l'échantillon biologique obtenu du patient après l'administration du tasélisib identifie un patient qui répondra au traitement avec le tasélisib.

4. Procédé *in vitro* de sélection d'un schéma de traitement pour un patient diagnostiqué comme ayant un cancer, le procédé comprenant la mise en contact d'une cellule cancéreuse du patient avec une quantité efficace de tasélisib, et la détection du taux de p110 alpha en réponse au tasélisib, dans lequel la détection d'une déplétion de p110 alpha indique que le cancer est susceptible d'être traité avec le tasélisib, et dans lequel le schéma de traitement

comprend l'administration du tasélisib au patient si le cancer est déterminé comme étant susceptible d'être traité avec le tasélisib.

**5.** Procédé selon la revendication 4, dans lequel la cellule cancéreuse est une cellule cancéreuse présentant une mutation de PIK3CA.

**6.** Tasélisib pour une utilisation dans le traitement d'un cancer, dans lequel les patients ont été sélectionnés conformément à l'une quelconque des méthodes selon les revendications 1 à 3.

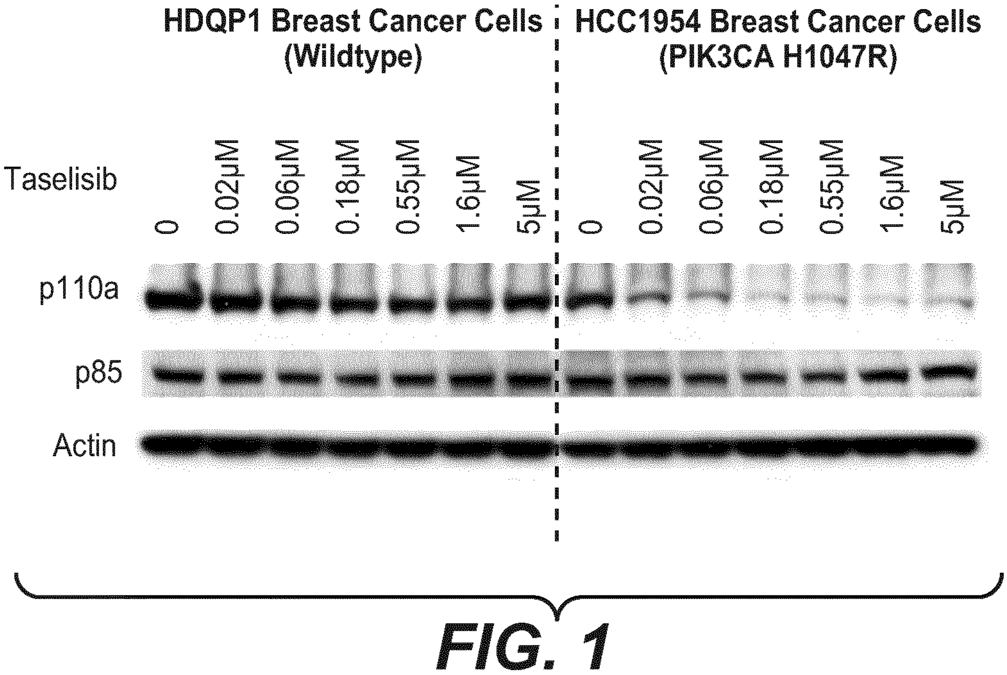
HDQP1 Breast Cancer Cells
(Wildtype)
HCC1954 Breast Cancer Cells
(PIK3CA H1047R)
Taselisib
0
0.02μM
0.06μM
0.18μM
0.55μM
1.6μM
5μM
0
0.02μM
0.06μM
0.18μM
0.55μM
1.6μM
5μM
p110a
p85
Actin

*FIG. 1*

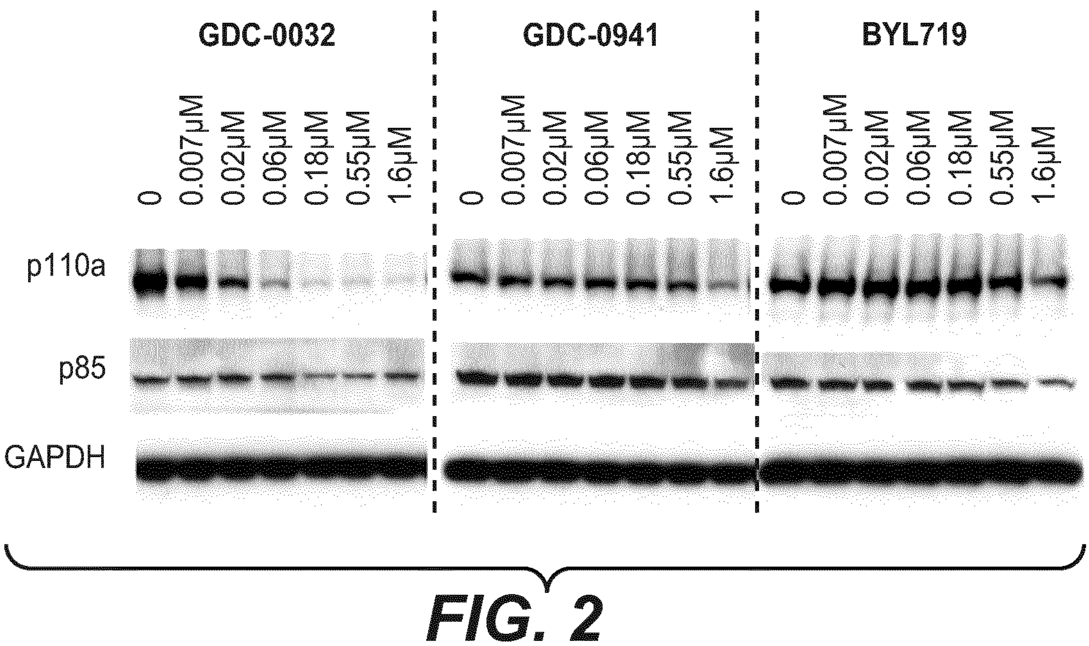
GDC-0032
GDC-0941
BYL719
0
0.007μM
0.02μM
0.06μM
0.18μM
0.55μM
1.6μM
0
0.007μM
0.02μM
0.06μM
0.18μM
0.55μM
1.6μM
0
0.007μM
0.02μM
0.06μM
0.18μM
0.55μM
1.6μM
p110a
p85
GAPDH

*FIG. 2*

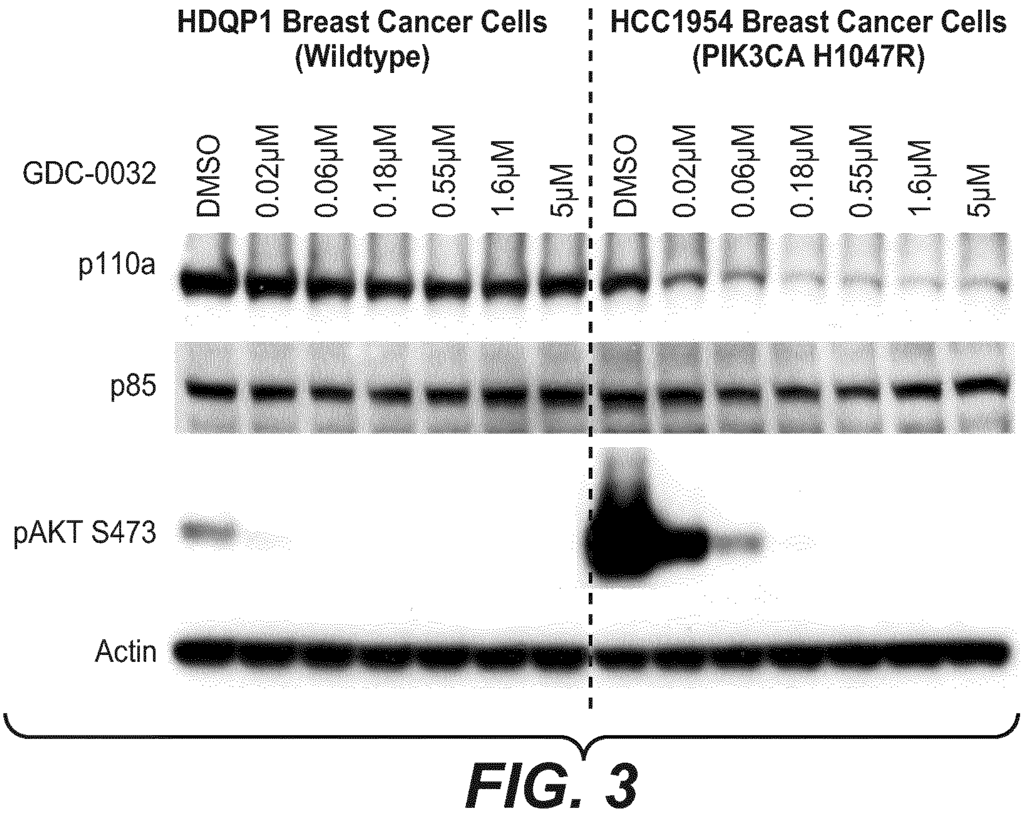
HDQP1 Breast Cancer Cells
(Wildtype)
HCC1954 Breast Cancer Cells
(PIK3CA H1047R)
GDC-0032
DMSO
0.02μM
0.06μM
0.18μM
0.55μM
1.6μM
5μM
DMSO
0.02μM
0.06μM
0.18μM
0.55μM
1.6μM
5μM
p110a
p85
pAKT S473
Actin

FIG. 3

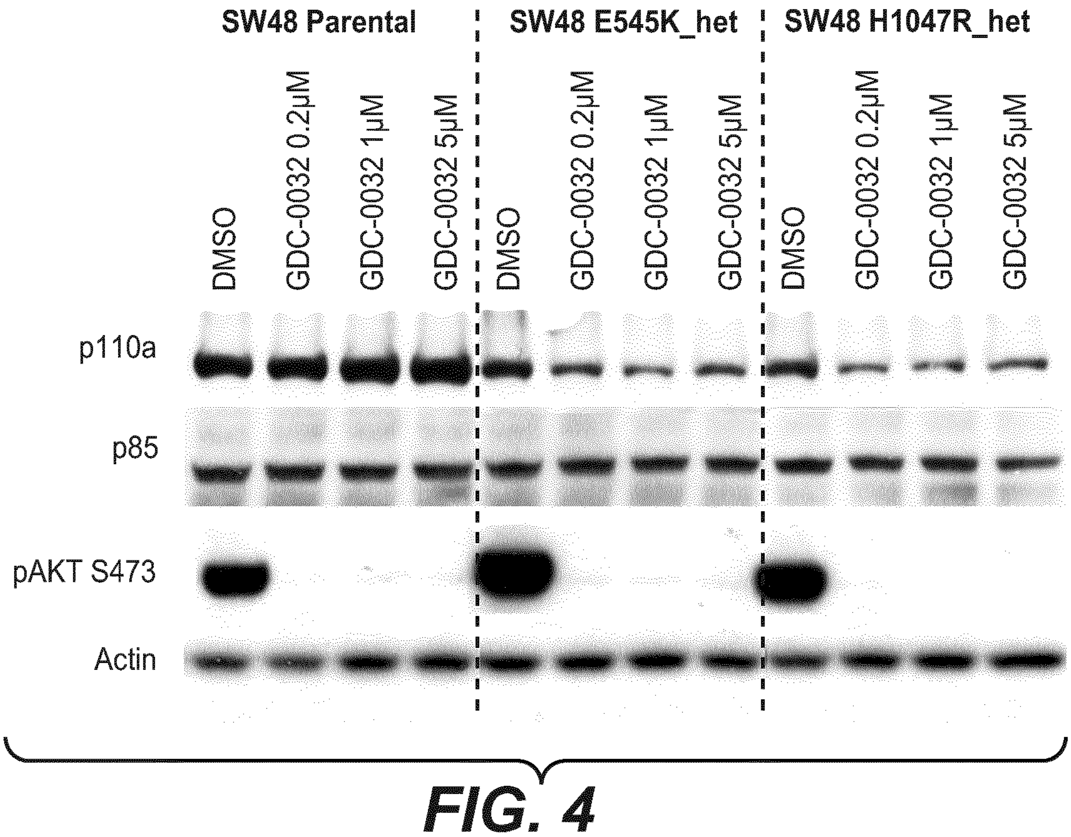
SW48 Parental
SW48 E545K_het
SW48 H1047R_het
DMSO
GDC-0032 0.2μM
GDC-0032 1μM
GDC-0032 5μM
DMSO
GDC-0032 0.2μM
GDC-0032 1μM
GDC-0032 5μM
DMSO
GDC-0032 0.2μM
GDC-0032 1μM
GDC-0032 5μM
p110a
p85
pAKT S473
Actin

FIG. 4

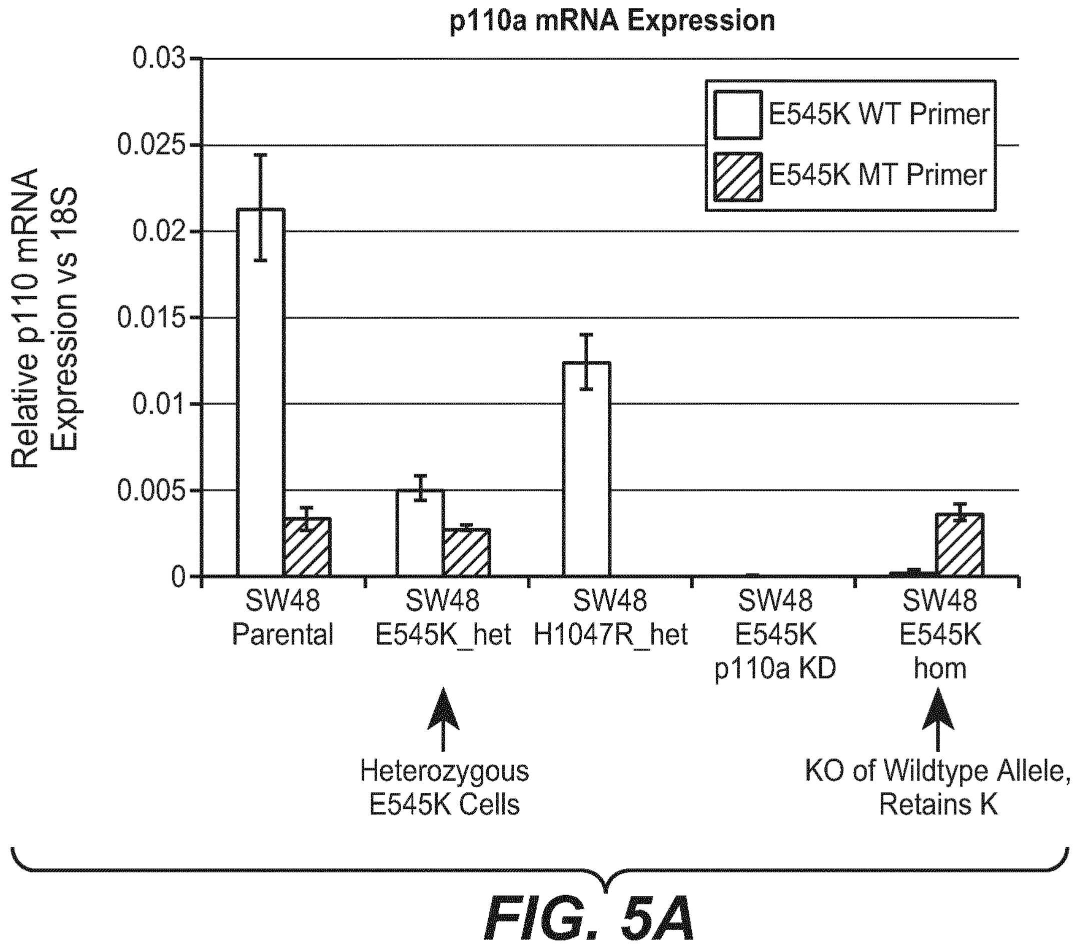
p110a mRNA Expression
0.03
0.025
0.02
0.015
0.01
0.005
0
Relative p110 mRNA Expression vs 18S
E545K WT Primer
E545K MT Primer
SW48 Parental
SW48 E545K_het
SW48 H1047R_het
SW48 E545K p110a KD
SW48 E545K hom
Heterozygous E545K Cells
KO of Wildtype Allele, Retains K

FIG. 5A

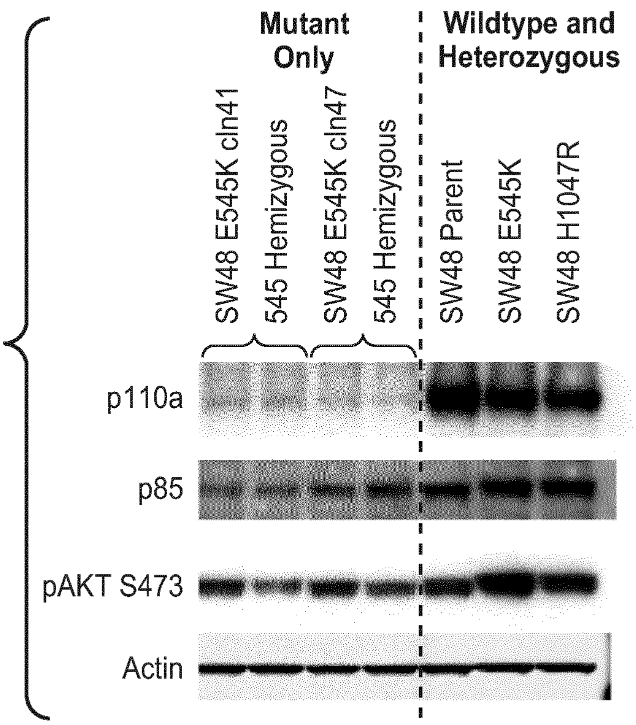
Mutant Only
Wildtype and Heterozygous
SW48 E545K cln41
545 Hemizygous
SW48 E545K cln47
545 Hemizygous
SW48 Parent
SW48 E545K
SW48 H1047R
p110a
p85
pAKT S473
Actin

FIG. 5B

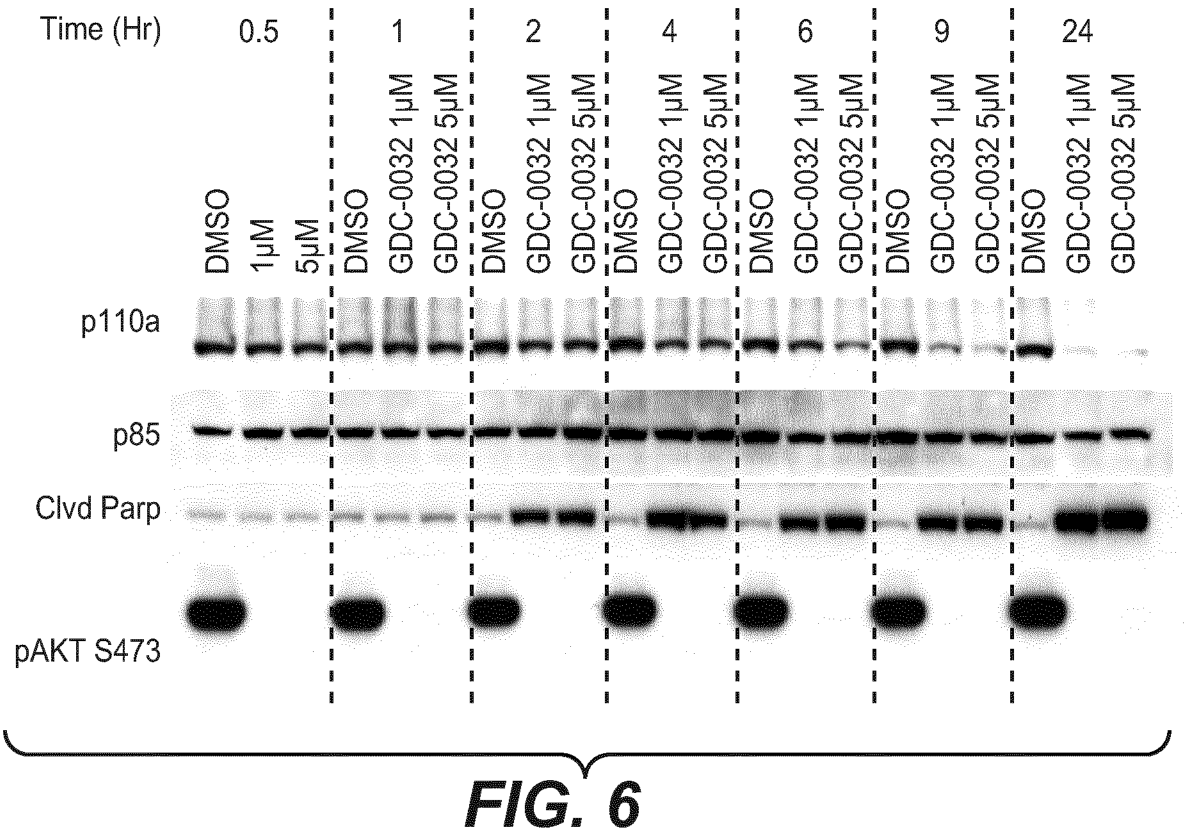
Time (Hr)
0.5
1
2
4
6
9
24
DMSO
1μM
5μM
DMSO
GDC-0032 1μM
GDC-0032 5μM
DMSO
GDC-0032 1μM
GDC-0032 5μM
DMSO
GDC-0032 1μM
GDC-0032 5μM
DMSO
GDC-0032 1μM
GDC-0032 5μM
DMSO
GDC-0032 1μM
GDC-0032 5μM
DMSO
GDC-0032 1μM
GDC-0032 5μM
p110a
p85
Clvd Parp
pAKT S473

FIG. 6

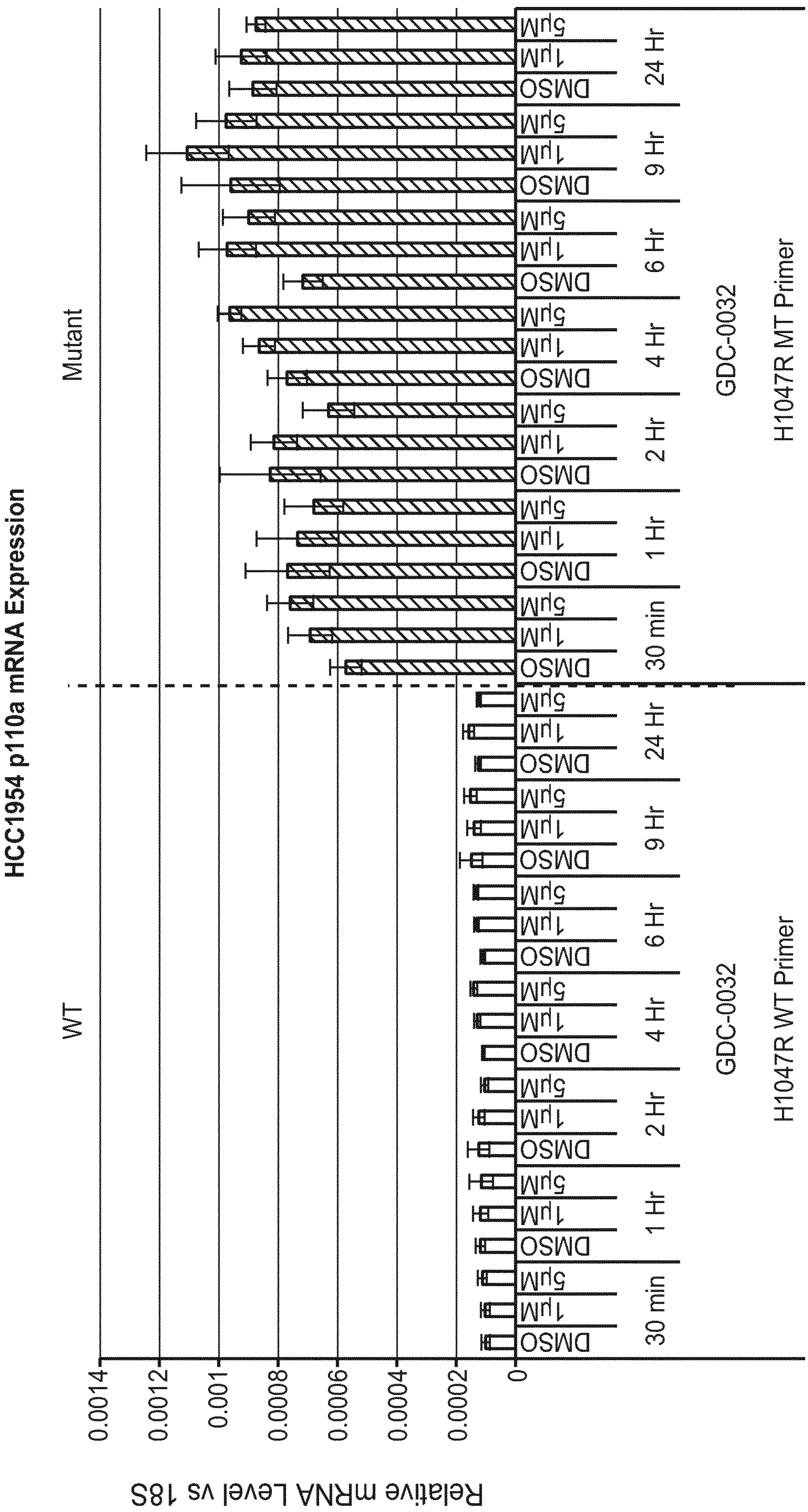
HCC1954 p110a mRNA Expression
WT
Mutant
Relative mRNA Level vs 18S
0.0014
0.0012
0.001
0.0008
0.0006
0.0004
0.0002
0
DMSO
1μM
5μM
30 min
1 Hr
2 Hr
4 Hr
6 Hr
9 Hr
24 Hr
GDC-0032
H1047R WT Primer
H1047R MT Primer

FIG. 7A

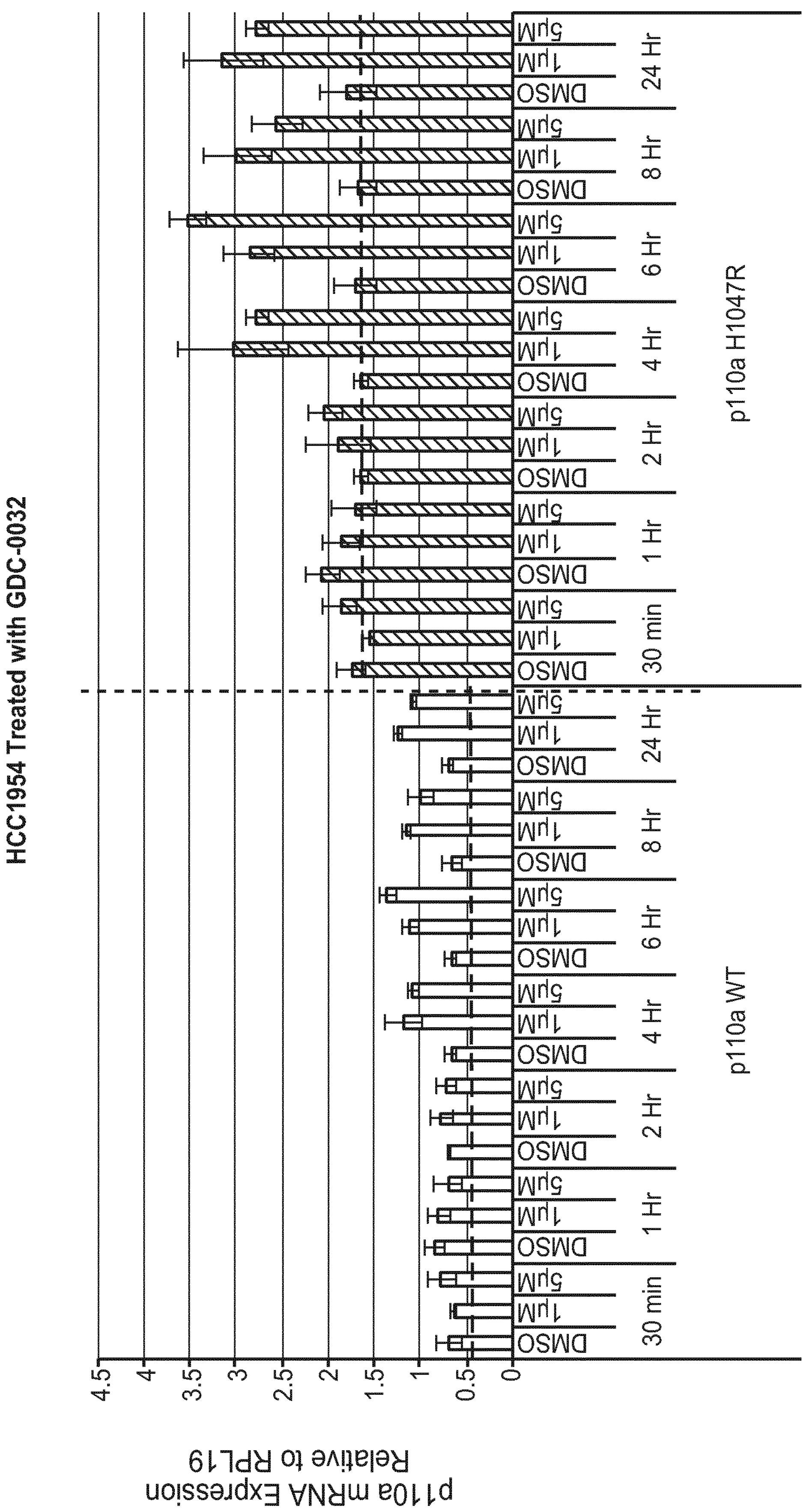
HCC1954 Treated with GDC-0032
p110a mRNA Expression
Relative to RPL19
0
0.5
1
1.5
2
2.5
3
3.5
4
4.5
DMSO
1µM
5µM
30 min
1 Hr
2 Hr
4 Hr
6 Hr
8 Hr
24 Hr
p110a WT
p110a H1047R

*FIG. 7B*

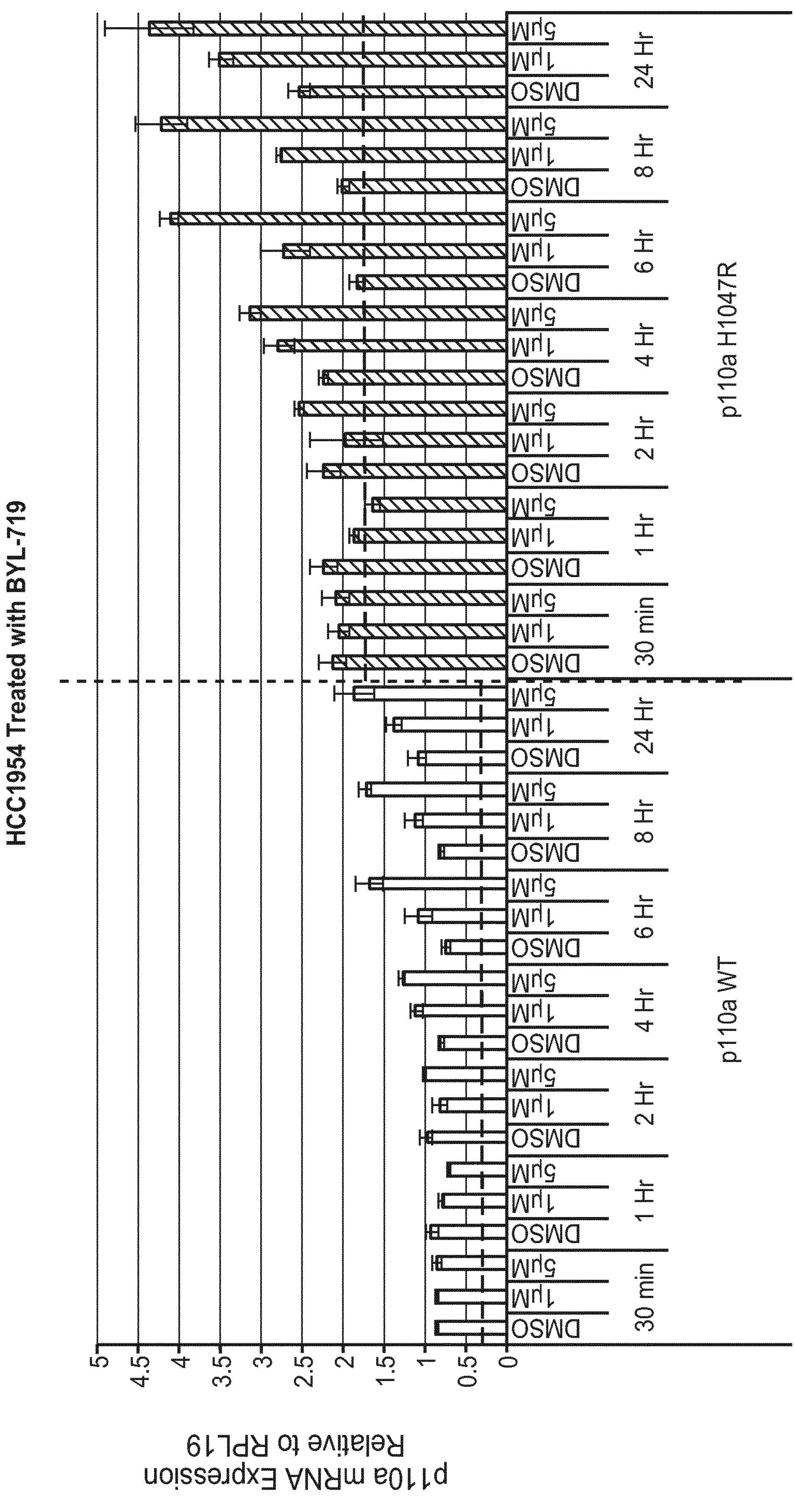
HCC1954 Treated with BYL-719
p110a mRNA Expression Relative to RPL19
0
0.5
1
1.5
2
2.5
3
3.5
4
4.5
5
DMSO
1μM
5μM
30 min
1 Hr
2 Hr
4 Hr
6 Hr
8 Hr
24 Hr
p110a WT
p110a H1047R

FIG. 7C

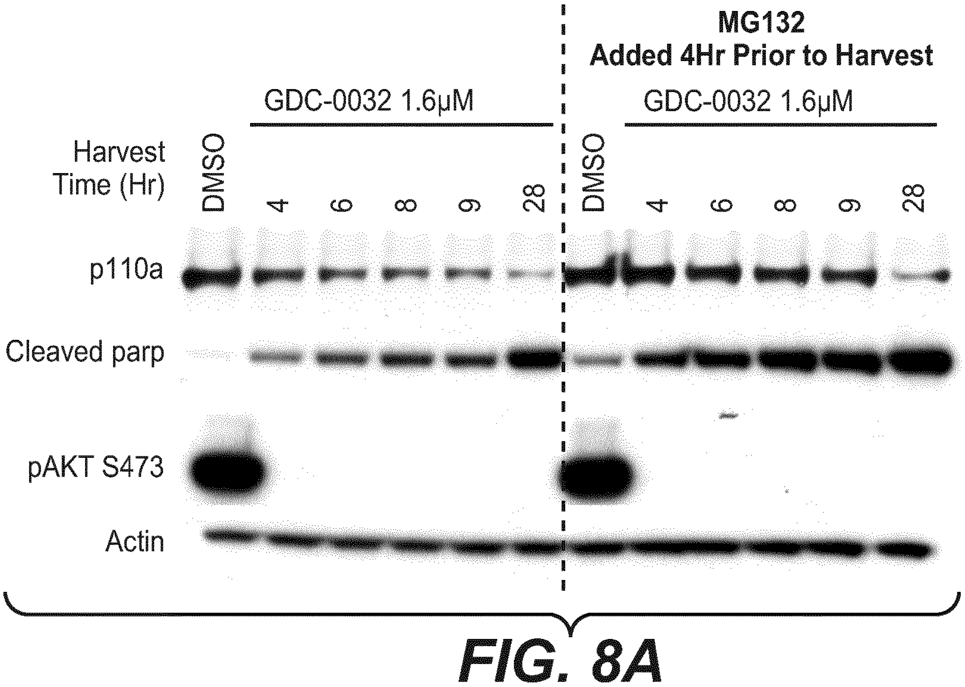
MG132
Added 4Hr Prior to Harvest
GDC-0032 1.6μM
GDC-0032 1.6μM
Harvest Time (Hr)
DMSO
4
6
8
9
28
DMSO
4
6
8
9
28
p110a
Cleaved parp
pAKT S473
Actin

FIG. 8A

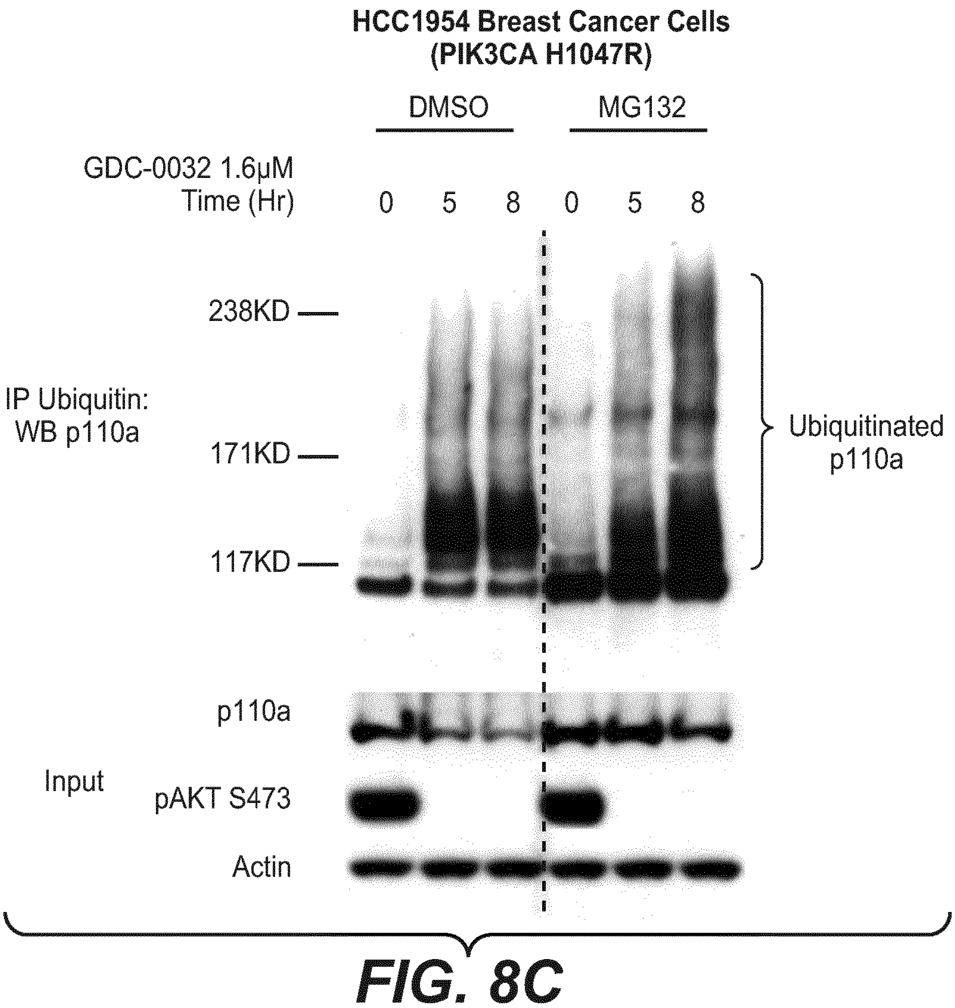
HCC1954 Breast Cancer Cells
(PIK3CA H1047R)
DMSO
MG132
GDC-0032 1.6μM
Time (Hr)
0
5
8
0
5
8
238KD
171KD
117KD
IP Ubiquitin:
WB p110a
Ubiquitinated p110a
p110a
Input
pAKT S473
Actin

FIG. 8C

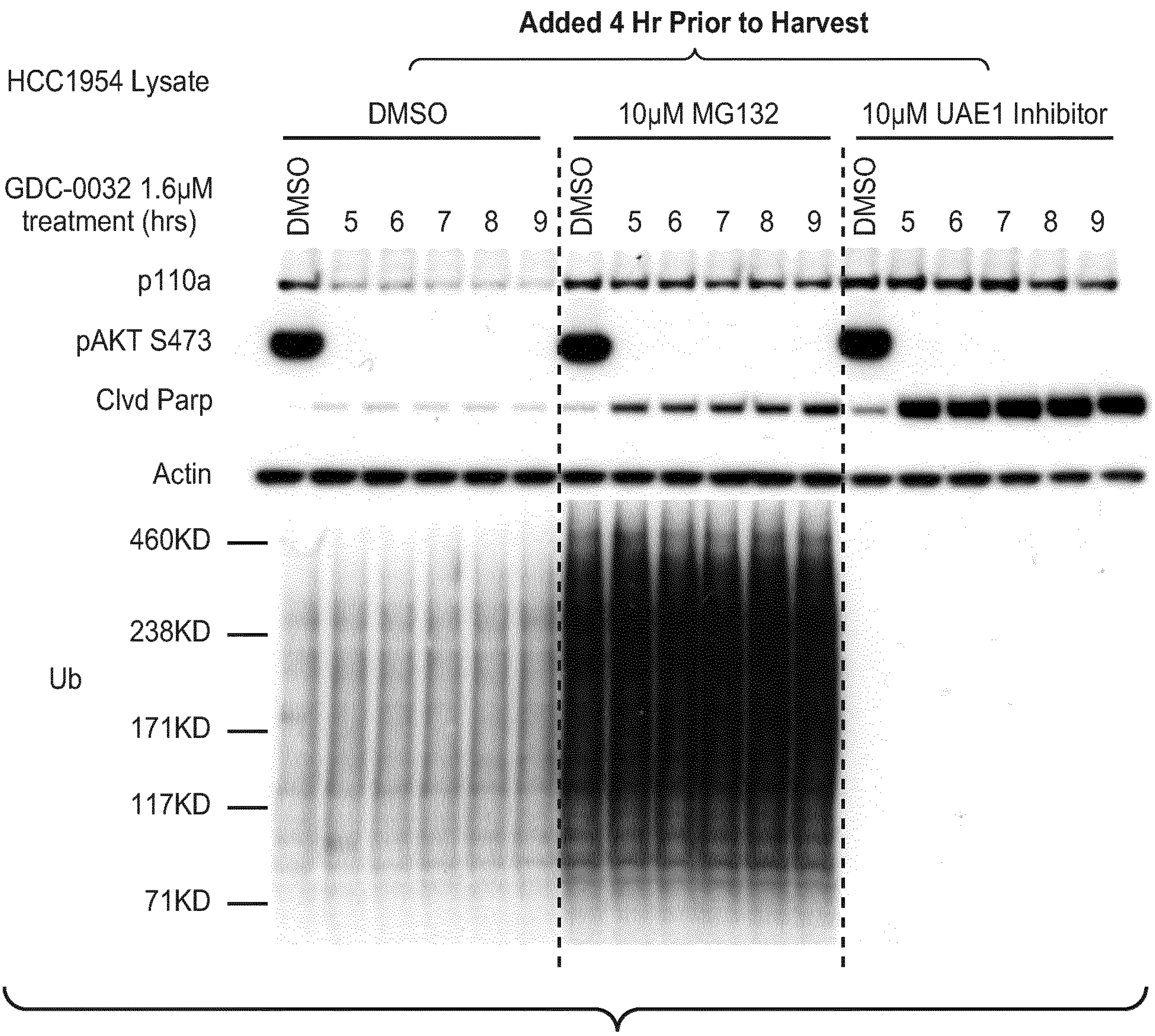
Added 4 Hr Prior to Harvest
HCC1954 Lysate
DMSO
10μM MG132
10μM UAE1 Inhibitor
GDC-0032 1.6μM treatment (hrs)
DMSO 5 6 7 8 9
DMSO 5 6 7 8 9
DMSO 5 6 7 8 9
p110a
pAKT S473
Clvd Parp
Actin
460KD
238KD
Ub
171KD
117KD
71KD

FIG. 8B

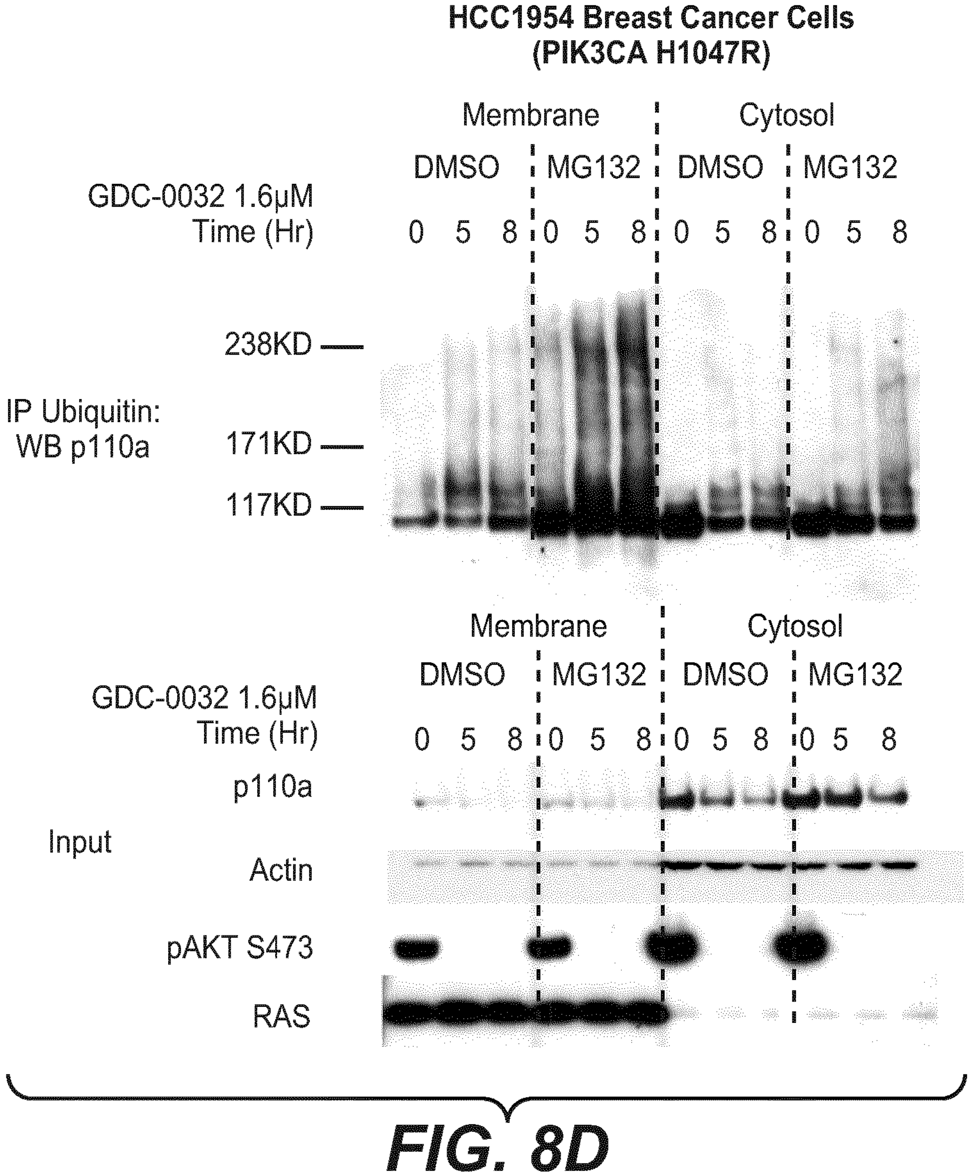
HCC1954 Breast Cancer Cells
(PIK3CA H1047R)
Membrane
Cytosol
DMSO
MG132
DMSO
MG132
GDC-0032 1.6μM
Time (Hr)
0 5 8 0 5 8 0 5 8 0 5 8
238KD
IP Ubiquitin:
WB p110a
171KD
117KD
Membrane
Cytosol
DMSO
MG132
DMSO
MG132
GDC-0032 1.6μM
Time (Hr)
0 5 8 0 5 8 0 5 8 0 5 8
p110a
Input
Actin
pAKT S473
RAS

FIG. 8D

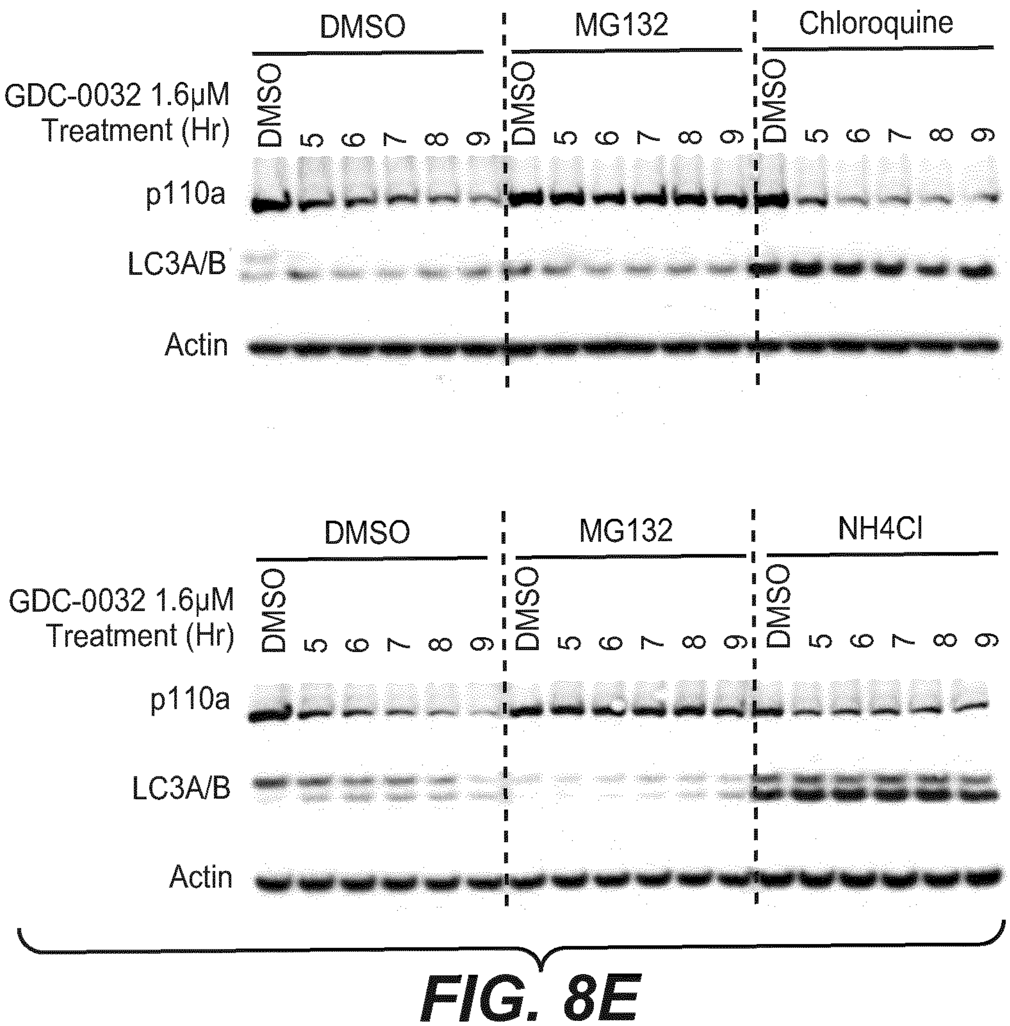
DMSO
MG132
Chloroquine
GDC-0032 1.6μM
Treatment (Hr)
DMSO 5 6 7 8 9 DMSO 5 6 7 8 9 DMSO 5 6 7 8 9
p110a
LC3A/B
Actin
DMSO
MG132
NH4Cl
GDC-0032 1.6μM
Treatment (Hr)
DMSO 5 6 7 8 9 DMSO 5 6 7 8 9 DMSO 5 6 7 8 9
p110a
LC3A/B
Actin

FIG. 8E

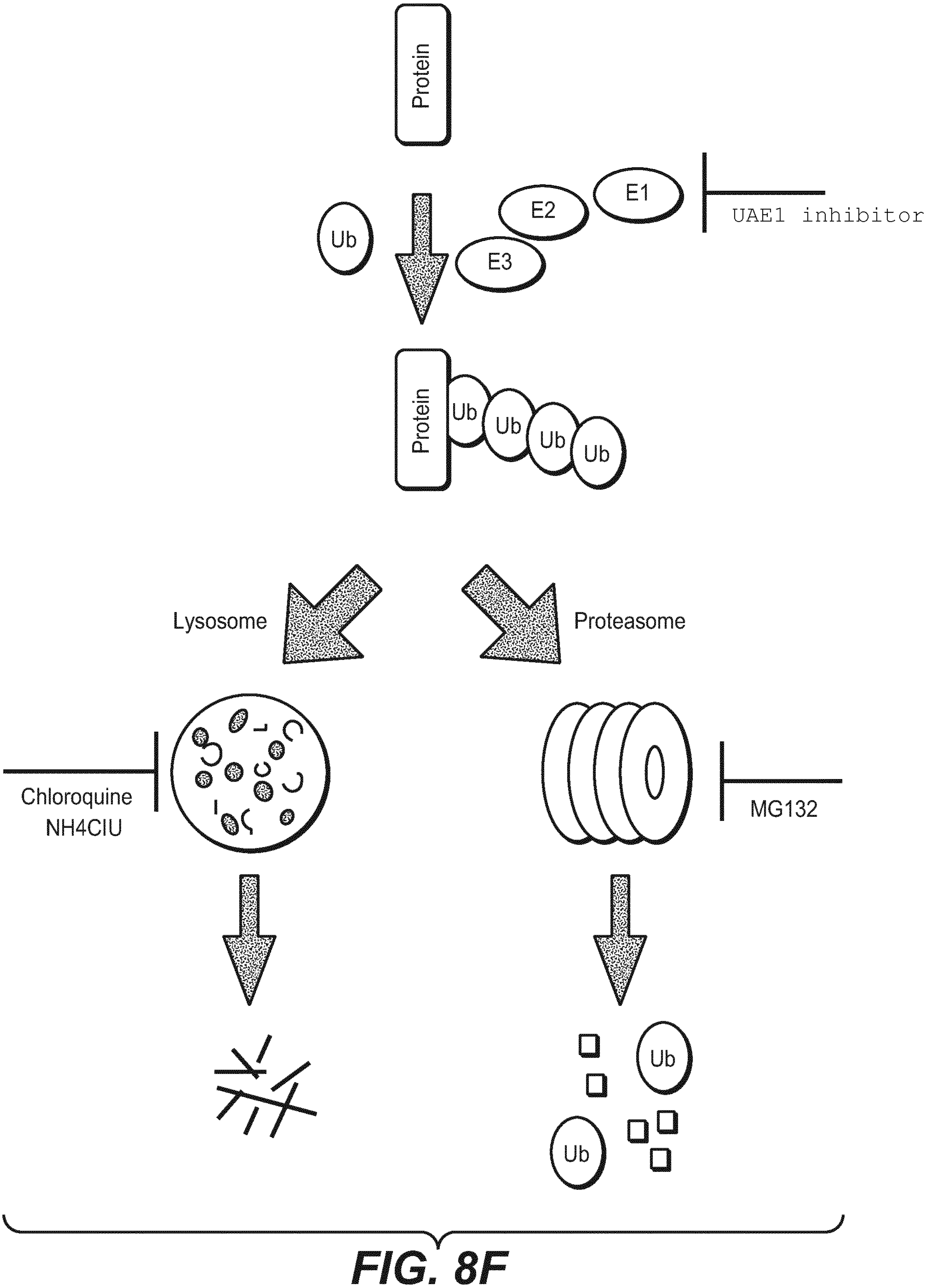
Protein
UAE1 inhibitor
E1
E2
E3
Ub
Protein
Ub
Ub
Ub
Ub
Lysosome
Proteasome
Chloroquine
NH4ClU
MG132
Ub
Ub

FIG. 8F

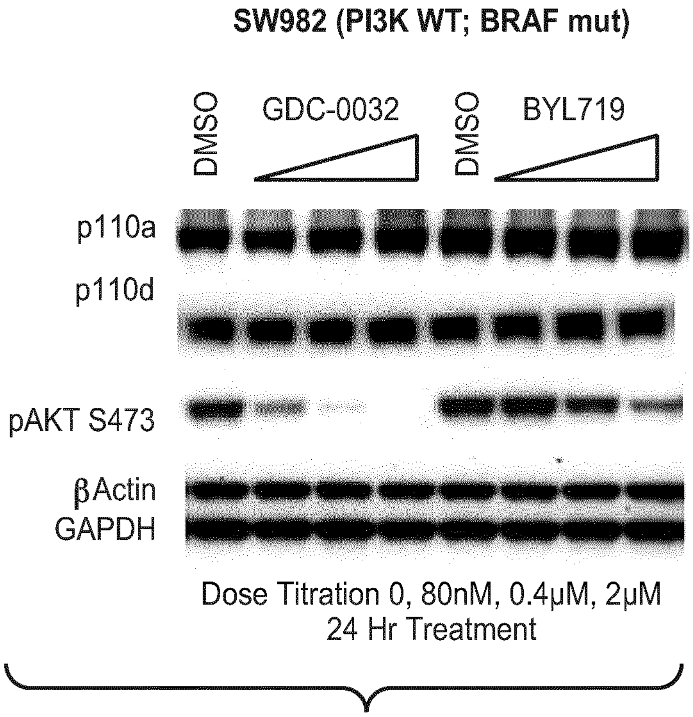
SW982 (PI3K WT; BRAF mut)
DMSO
GDC-0032
DMSO
BYL719
p110a
p110d
pAKT S473
βActin
GAPDH
Dose Titration 0, 80nM, 0.4μM, 2μM
24 Hr Treatment

FIG. 9A

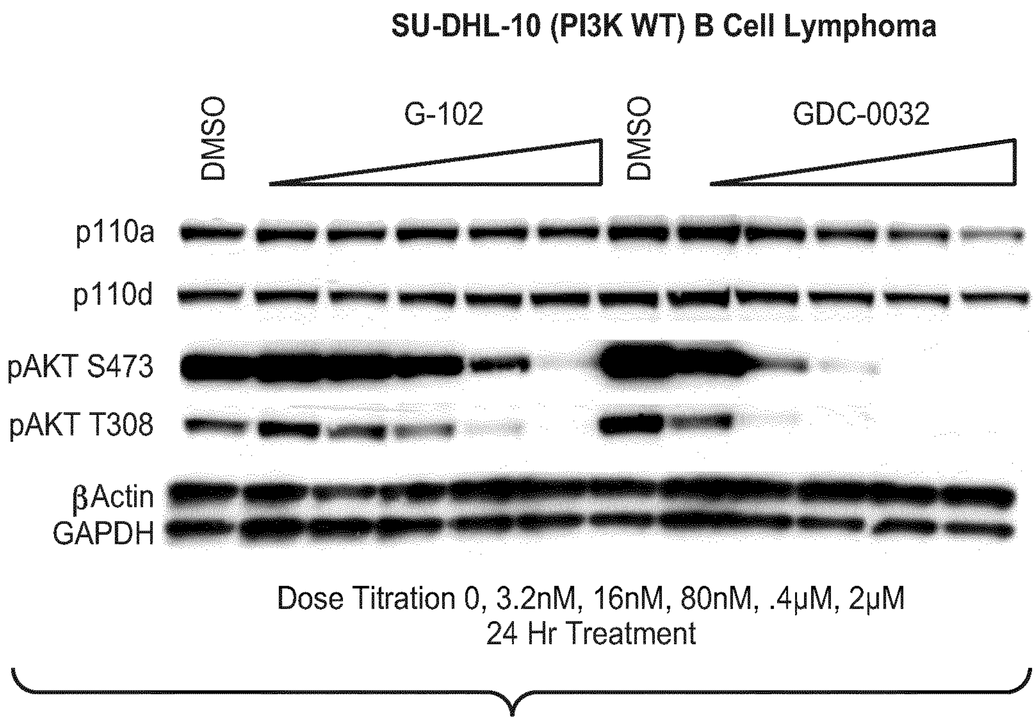
SU-DHL-10 (PI3K WT) B Cell Lymphoma
DMSO
G-102
DMSO
GDC-0032
p110a
p110d
pAKT S473
pAKT T308
βActin
GAPDH
Dose Titration 0, 3.2nM, 16nM, 80nM, .4μM, 2μM
24 Hr Treatment

FIG. 9B

FIG. 10

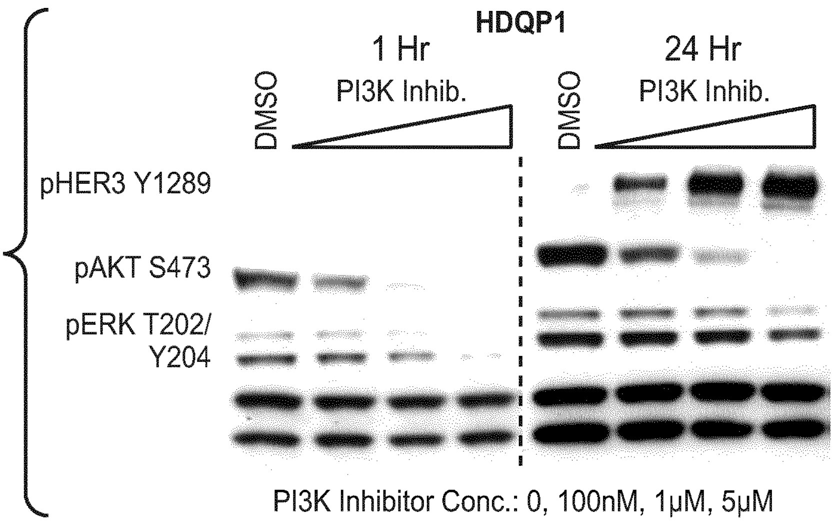


FIG. 11A

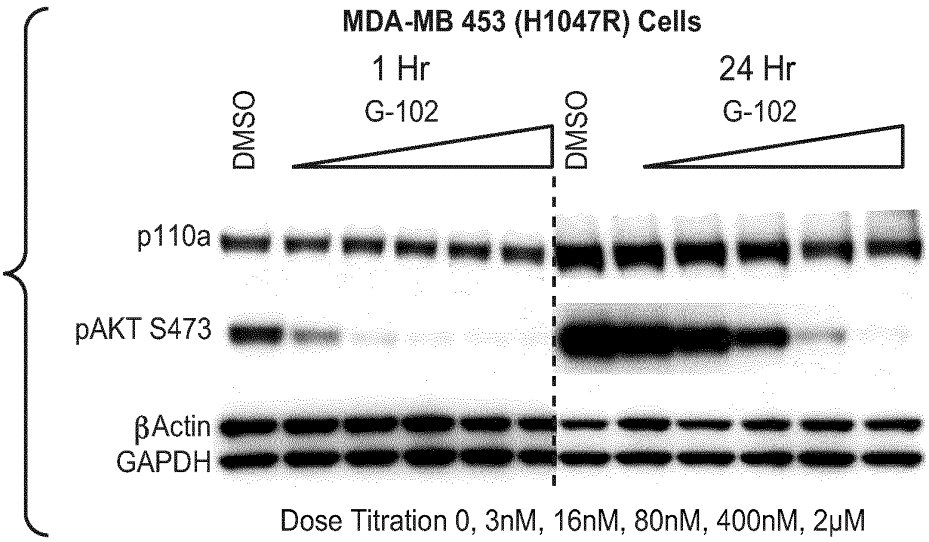


FIG. 11B

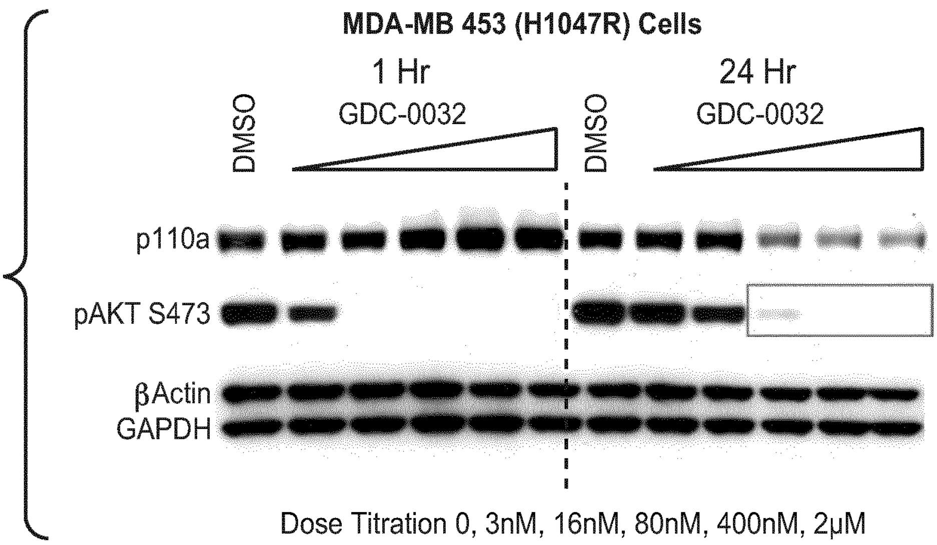

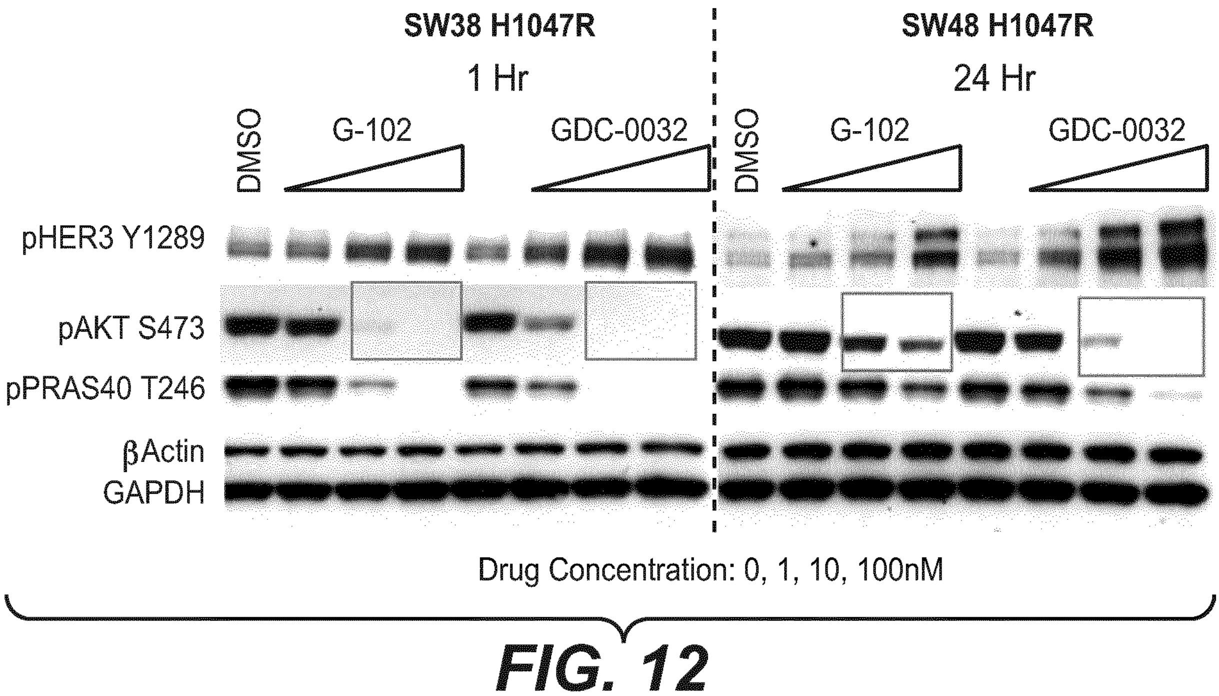
SW38 H1047R
1 Hr
SW48 H1047R
24 Hr
DMSO
G-102
GDC-0032
DMSO
G-102
GDC-0032
pHER3 Y1289
pAKT S473
pPRAS40 T246
βActin
GAPDH
Drug Concentration: 0, 1, 10, 100nM

FIG. 12

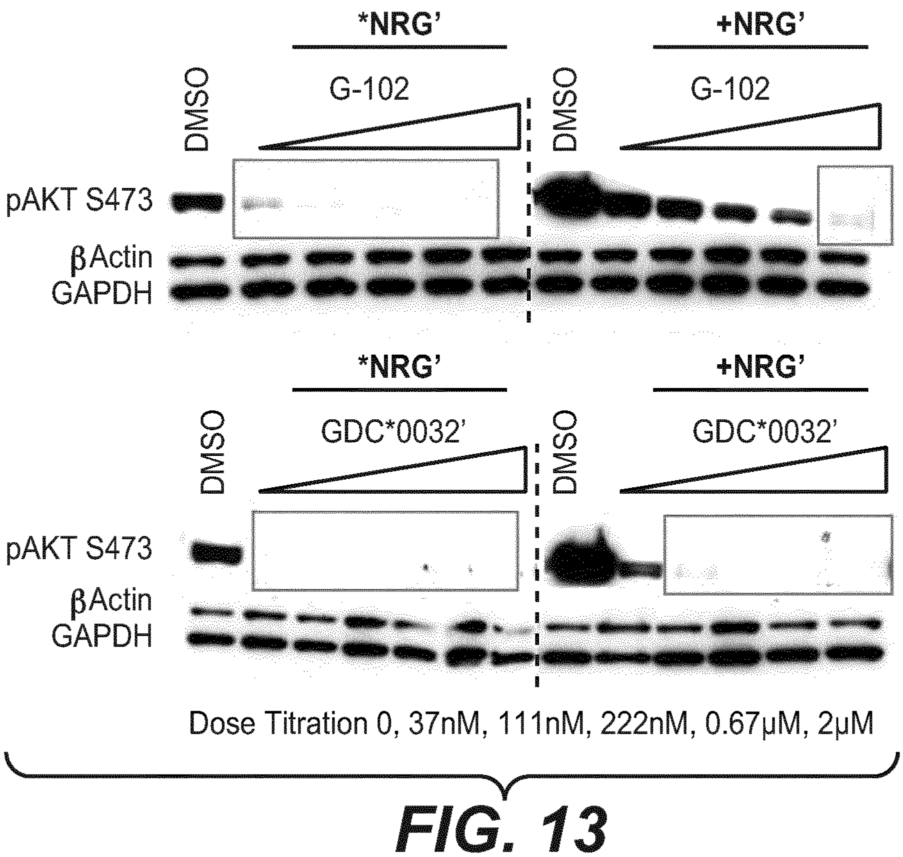
*NRG'
+NRG'
DMSO
G-102
DMSO
G-102
pAKT S473
βActin
GAPDH
*NRG'
+NRG'
DMSO
GDC*0032'
DMSO
GDC*0032'
pAKT S473
βActin
GAPDH
Dose Titration 0, 37nM, 111nM, 222nM, 0.67μM, 2μM

FIG. 13

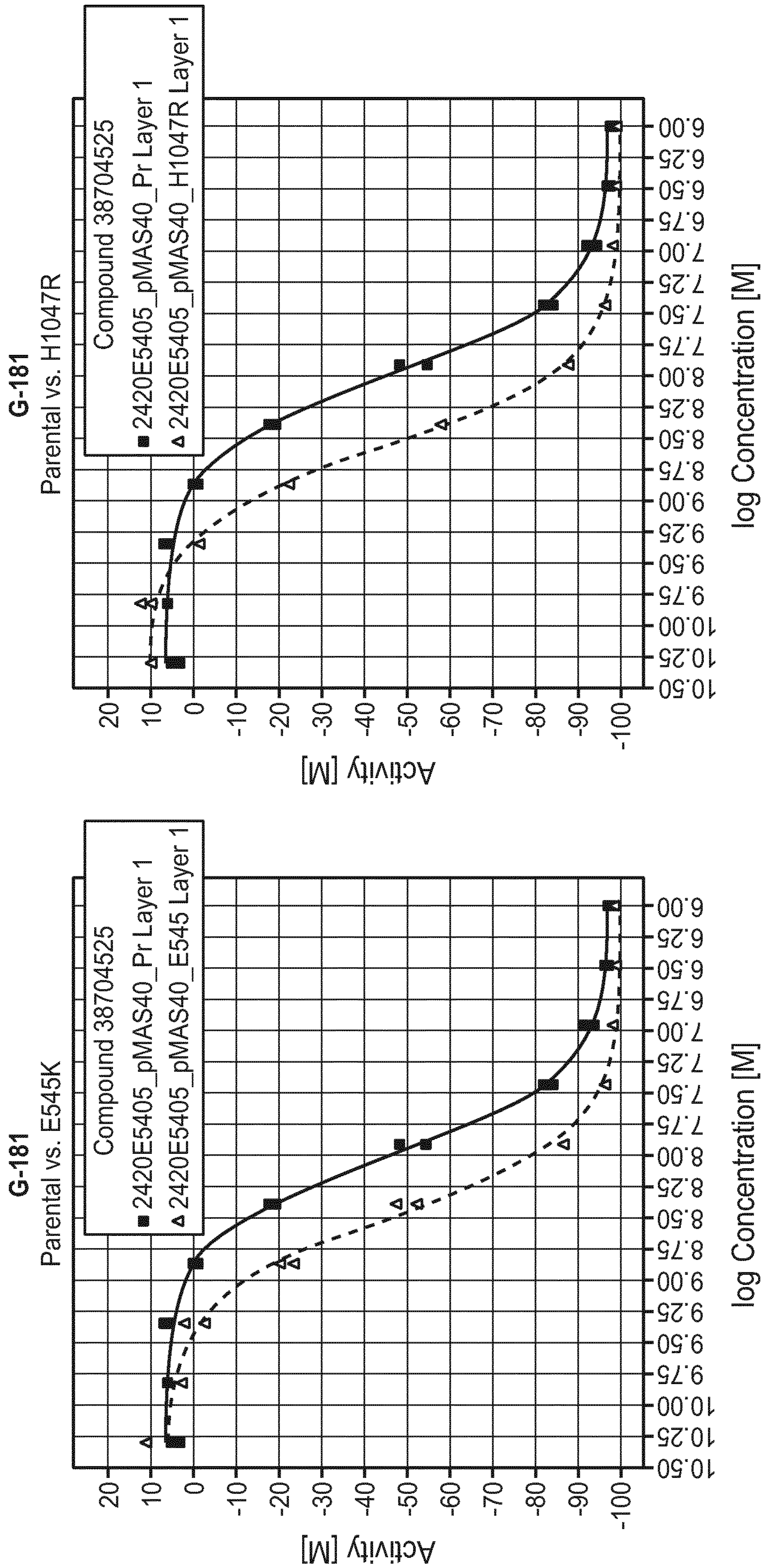


| | EC50 (nM) | Parental / Mutant |
|---|---|---|
| Parental | 10.2 | -- |
| E545K | 3.4 | 3.0 |
| H1047R | 2.6 | 3.9 |

FIG. 14A

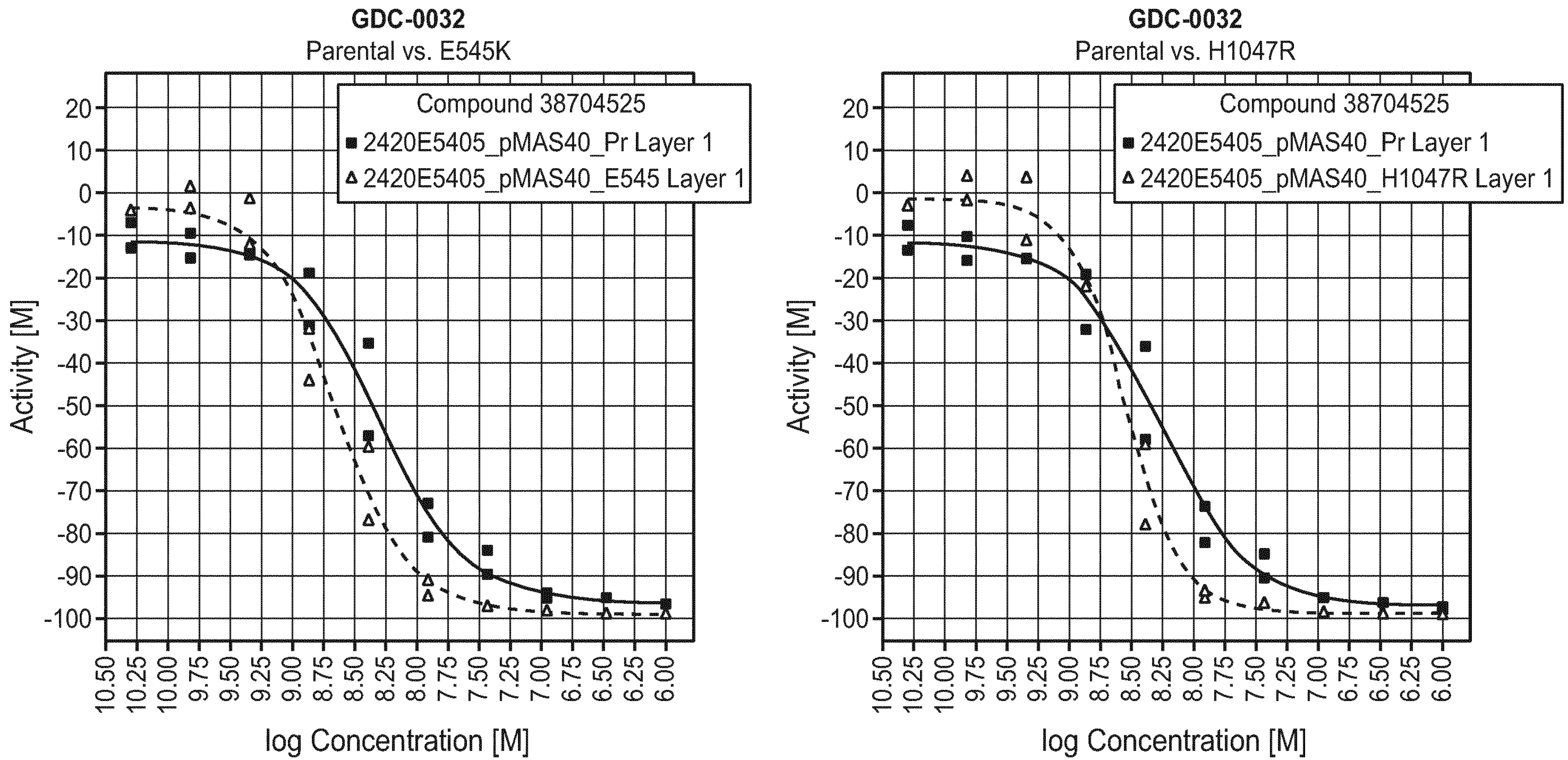


| | EC50 (nM) | Parental / Mutant |
|---|---|---|
| Parental | 5.1 | -- |
| E545K | 2.3 | 2.2 |
| H1047R | 2.7 | 1.9 |

FIG. 14B

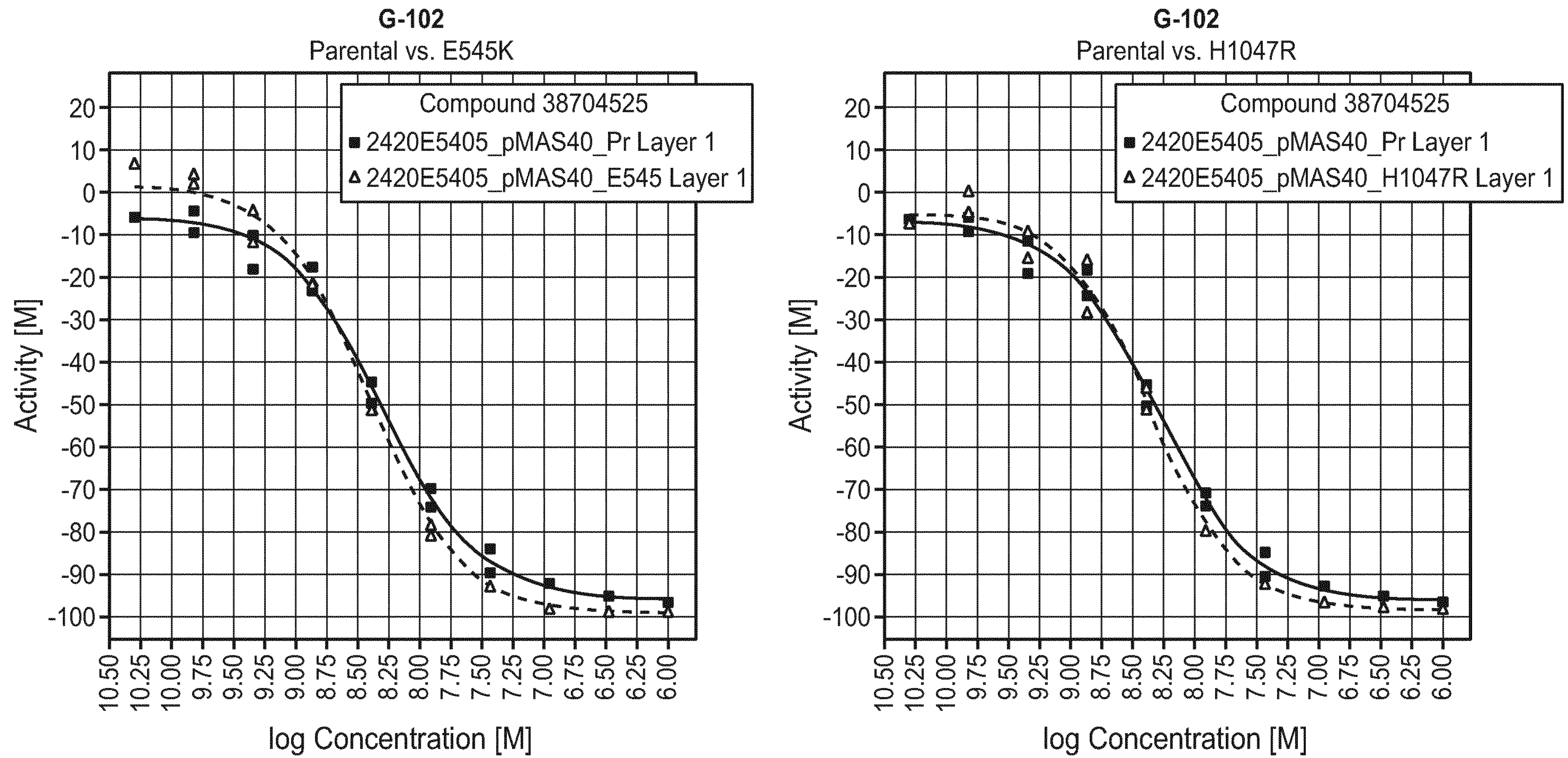


| | EC50 (nM) | Parental / Mutant |
|---|---|---|
| Parental | 4.9 | -- |
| E545K | 3.9 | 1.3 |
| H1047R | 4.4 | 1.1 |

FIG. 14C

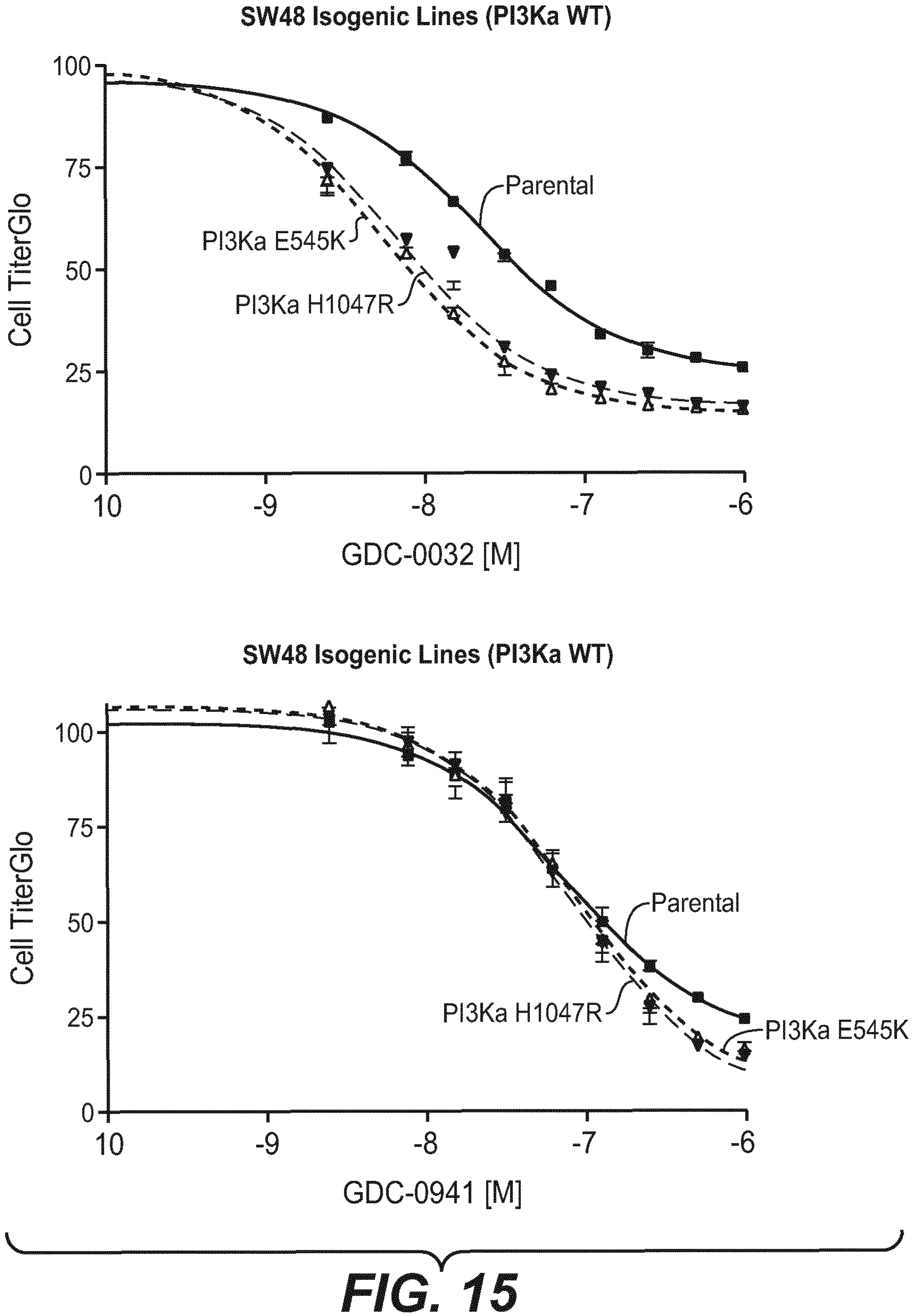
SW48 Isogenic Lines (PI3Ka WT)
Cell TiterGlo
100
75
50
25
0
10
-9
-8
-7
-6
GDC-0032 [M]
Parental
PI3Ka E545K
PI3Ka H1047R
SW48 Isogenic Lines (PI3Ka WT)
Cell TiterGlo
100
75
50
25
0
10
-9
-8
-7
-6
GDC-0941 [M]
Parental
PI3Ka H1047R
PI3Ka E545K

FIG. 15

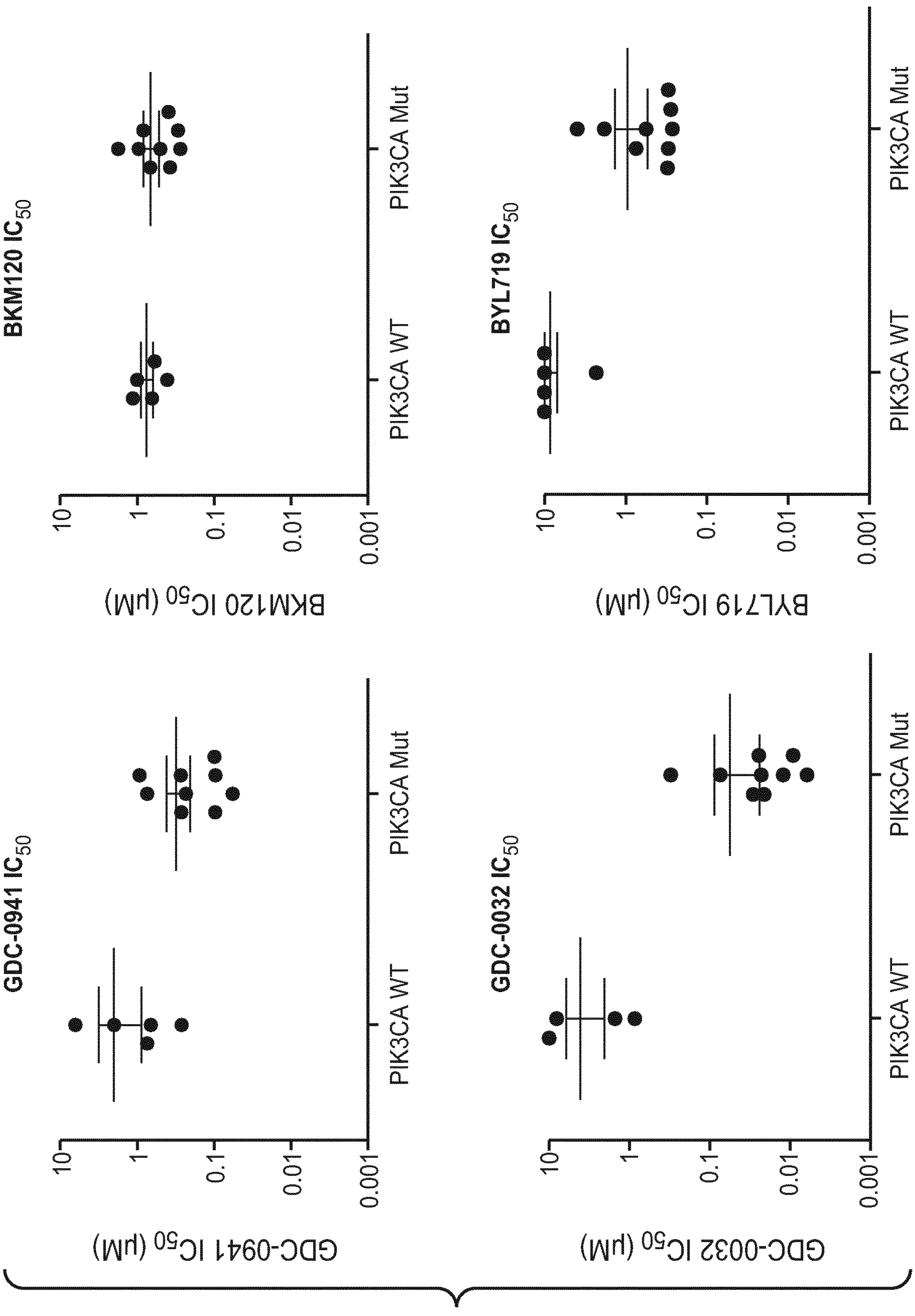
GDC-0941 IC50
GDC-0941 IC50 (µM)
10
1
0.1
0.01
0.001
PIK3CA WT
PIK3CA Mut
BKM120 IC50
BKM120 IC50 (µM)
GDC-0032 IC50
GDC-0032 IC50 (µM)
BYL719 IC50
BYL719 IC50 (µM)

*FIG. 16*

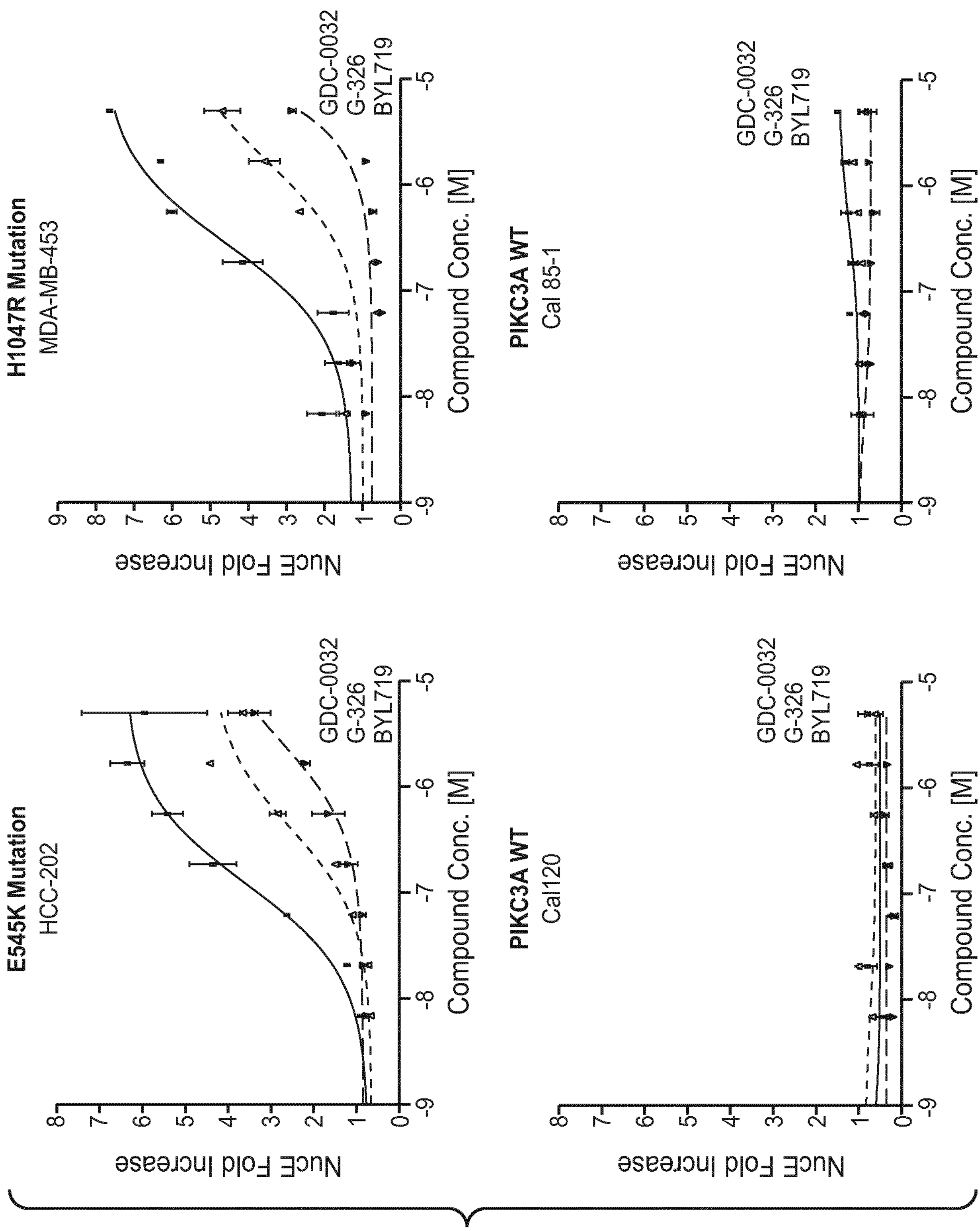
E545K Mutation
HCC-202
H1047R Mutation
MDA-MB-453
PIKC3A WT
Cal120
PIKC3A WT
Cal 85-1
NucE Fold Increase
Compound Conc. [M]
GDC-0032
G-326
BYL719

FIG. 17

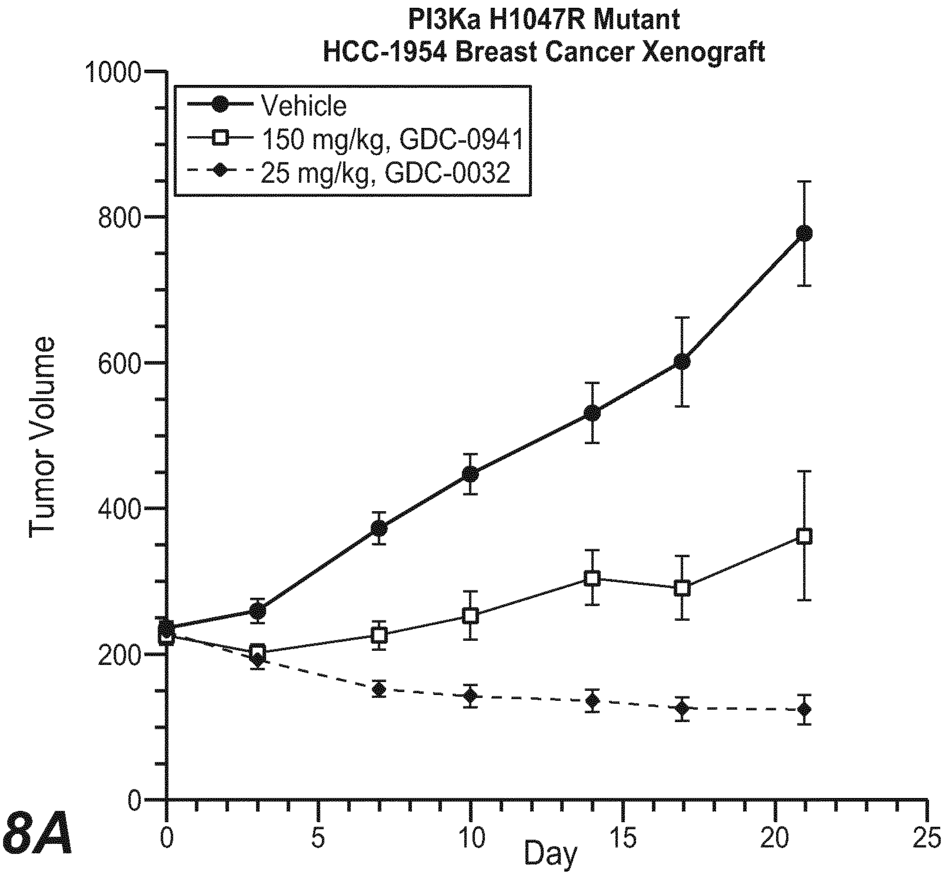


FIG. 18A

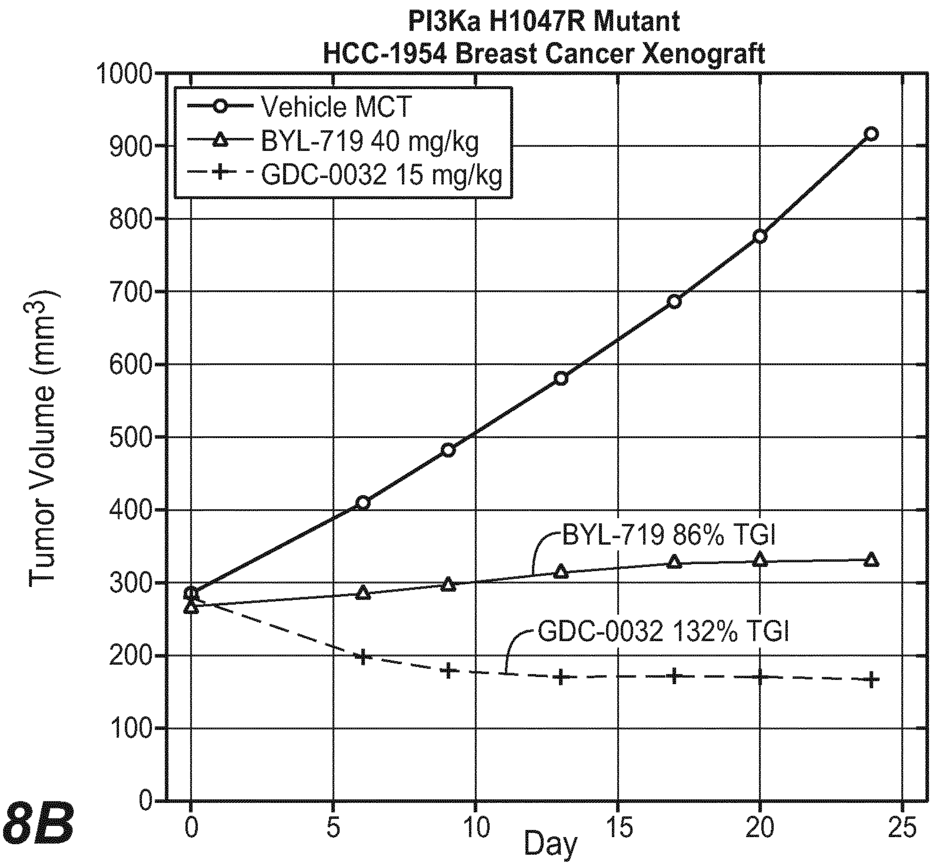


FIG. 18B

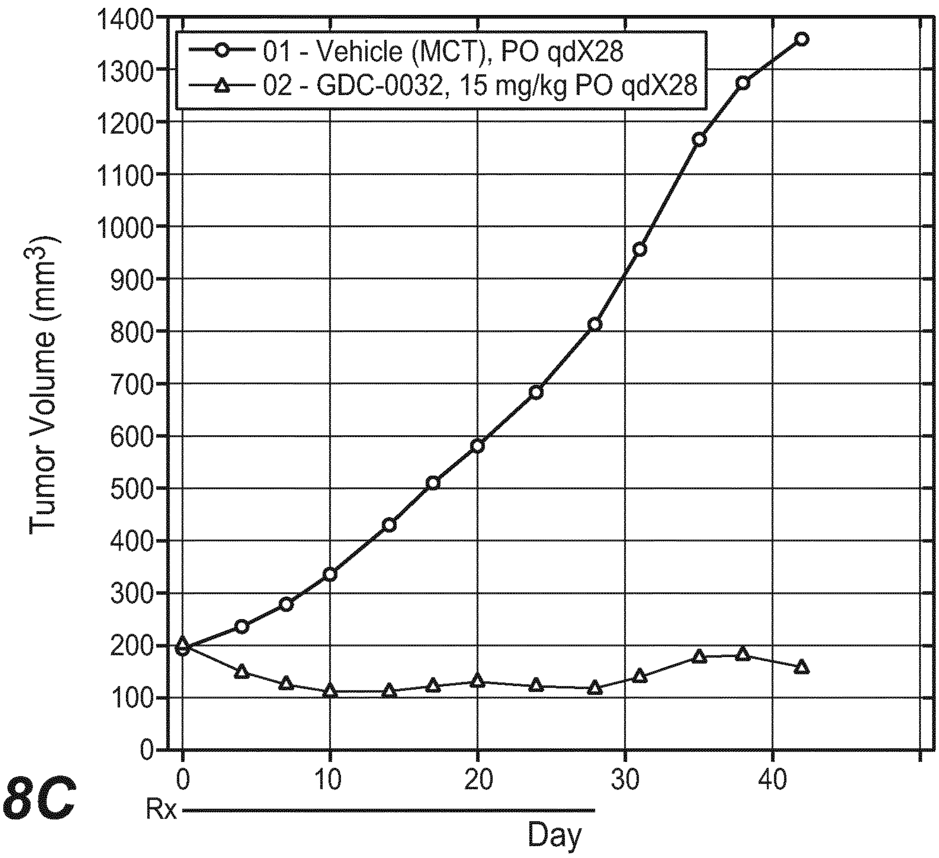


FIG. 18C

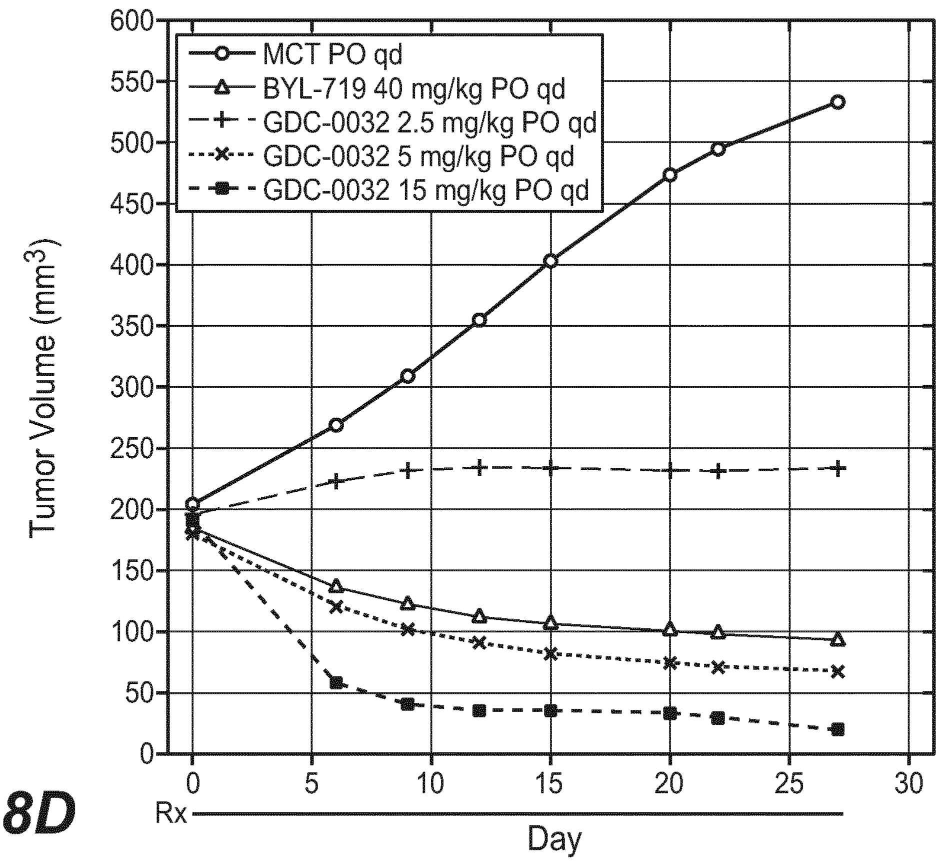


FIG. 18D

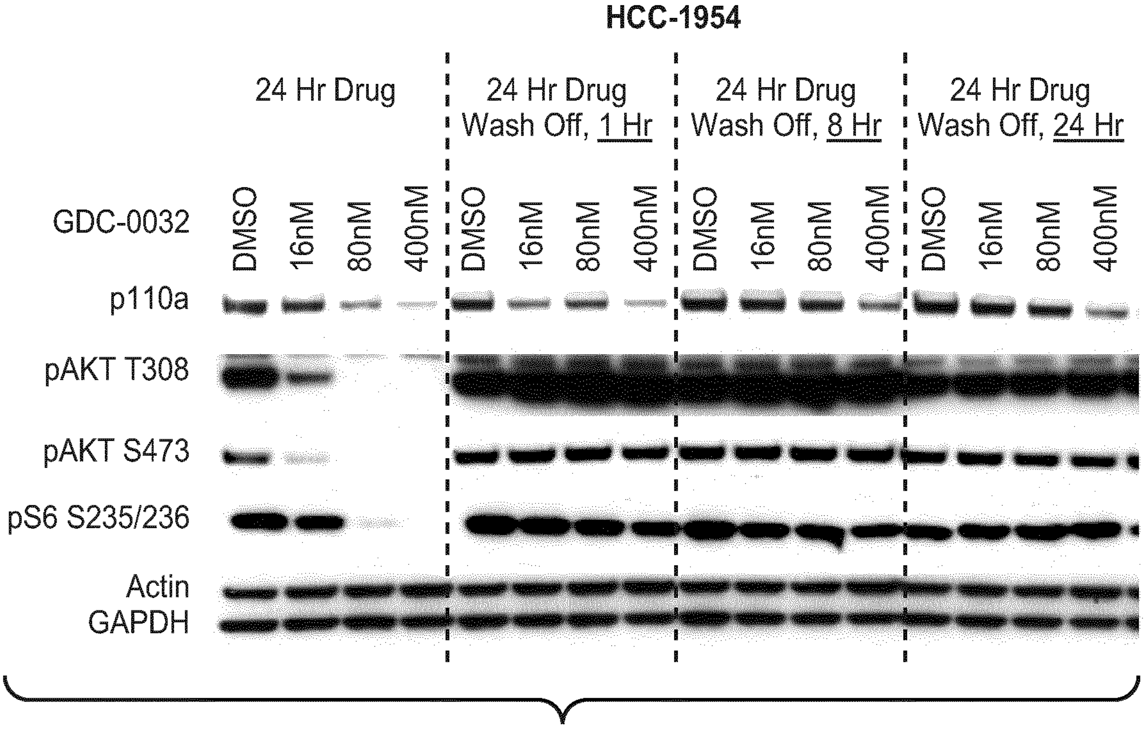
HCC-1954
24 Hr Drug
24 Hr Drug Wash Off, 1 Hr
24 Hr Drug Wash Off, 8 Hr
24 Hr Drug Wash Off, 24 Hr
GDC-0032
DMSO
16nM
80nM
400nM
p110a
pAKT T308
pAKT S473
pS6 S235/236
Actin
GAPDH

FIG. 19

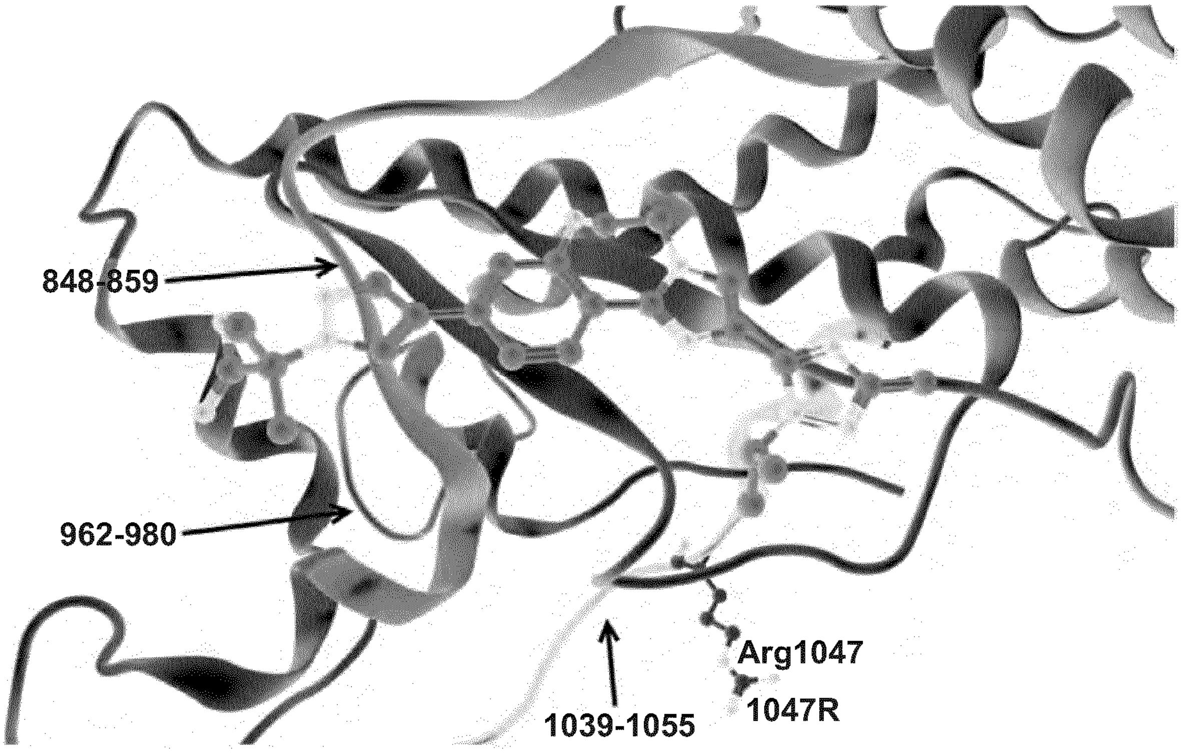
848-859
962-980
1039-1055
Arg1047
1047R

FIG. 20

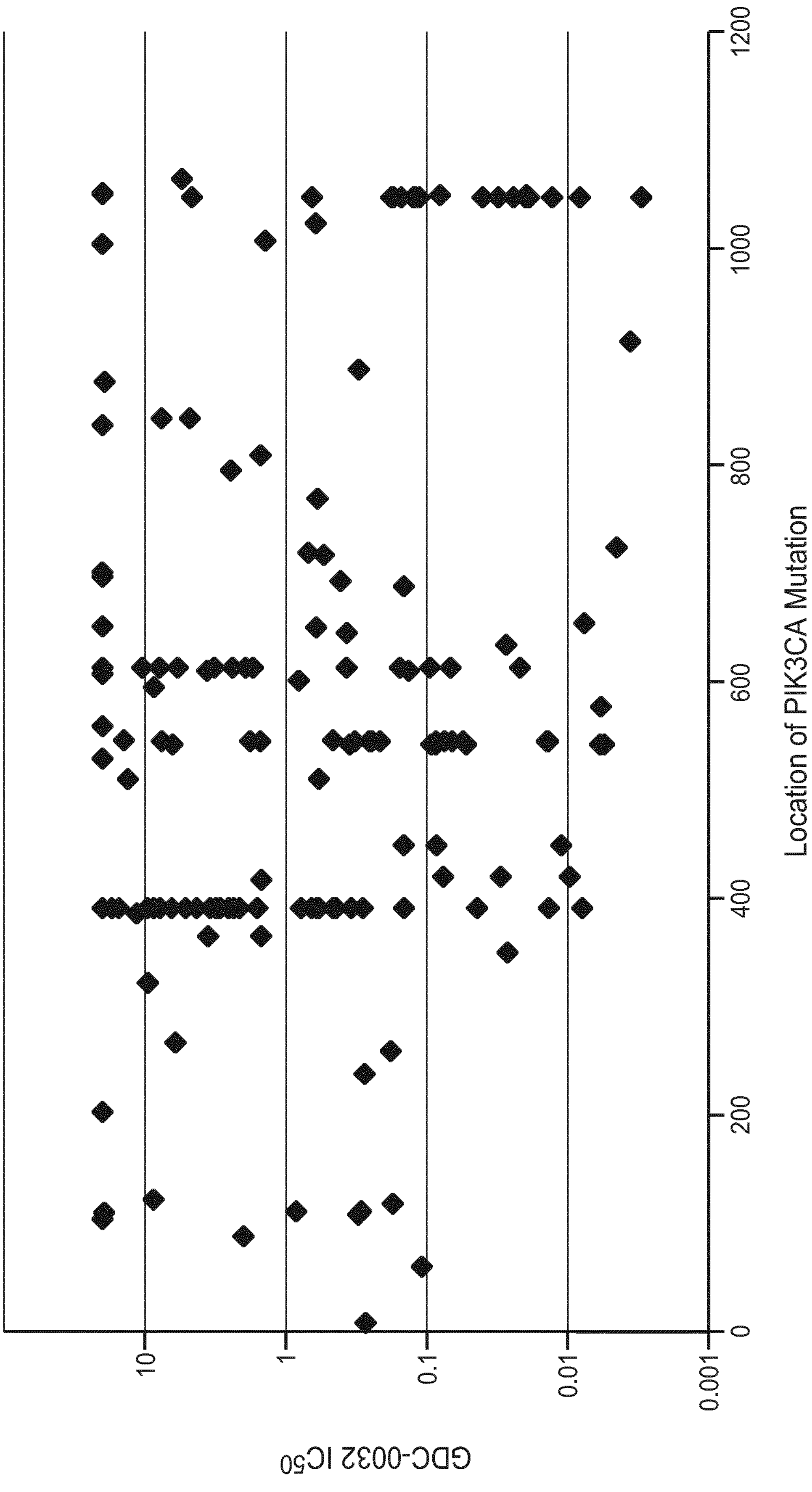
GDC-0032 IC50
10
1
0.1
0.01
0.001
0
200
400
600
800
1000
1200
Location of PIK3CA Mutation

*FIG. 21*

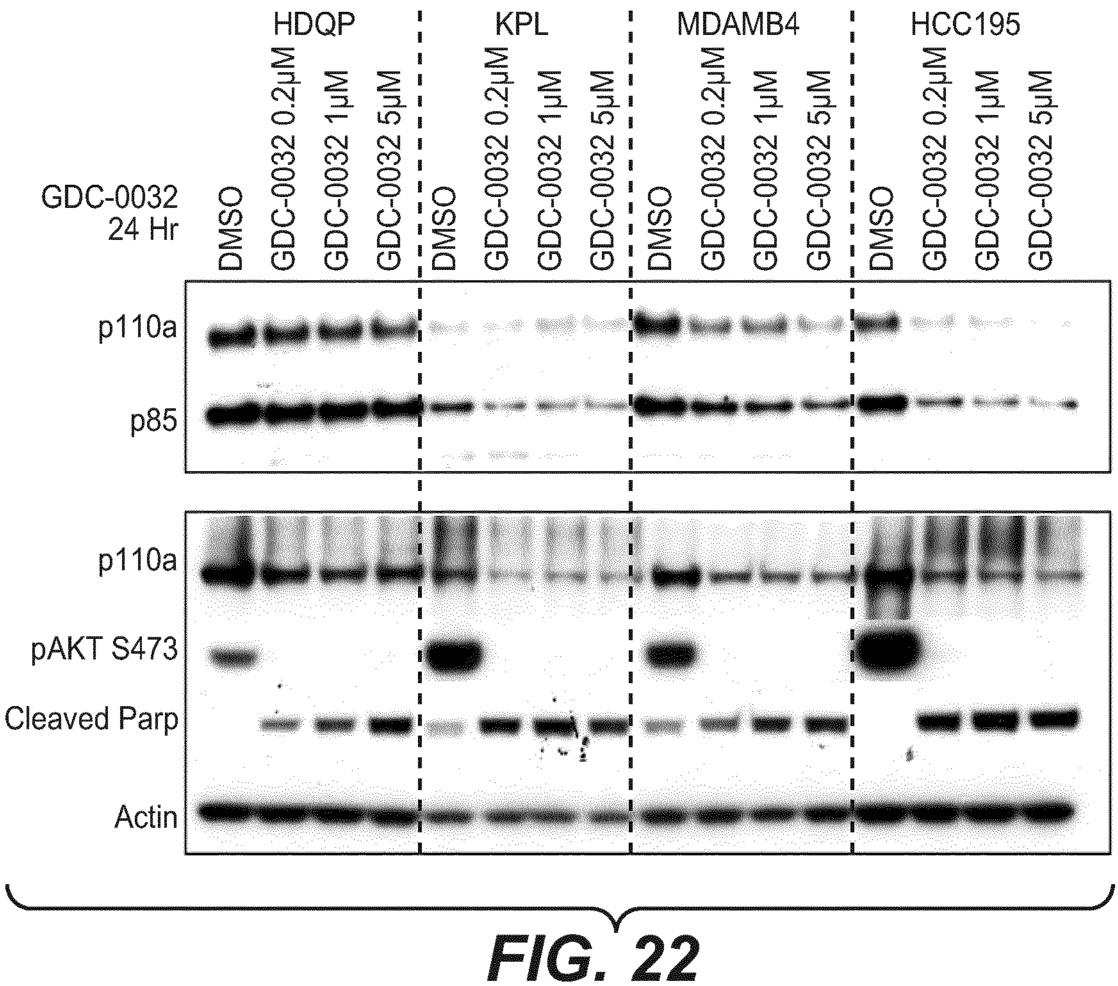
HDQP
KPL
MDAMB4
HCC195
GDC-0032
24 Hr
DMSO
GDC-0032 0.2μM
GDC-0032 1μM
GDC-0032 5μM
p110a
p85
p110a
pAKT S473
Cleaved Parp
Actin

*FIG. 22*

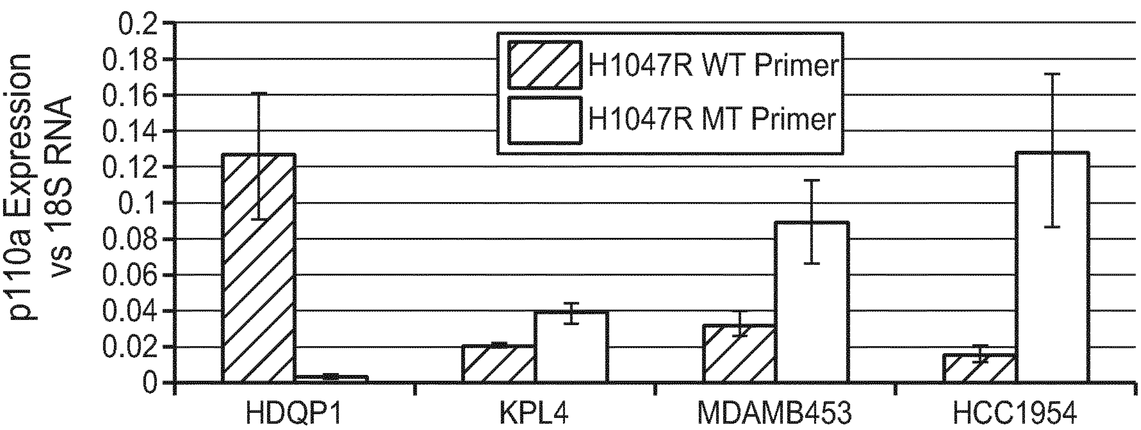
p110a Expression vs 18S RNA
0.2
0.18
0.16
0.14
0.12
0.1
0.08
0.06
0.04
0.02
0
H1047R WT Primer
H1047R MT Primer
HDQP1
KPL4
MDAMB453
HCC1954

*FIG. 23*

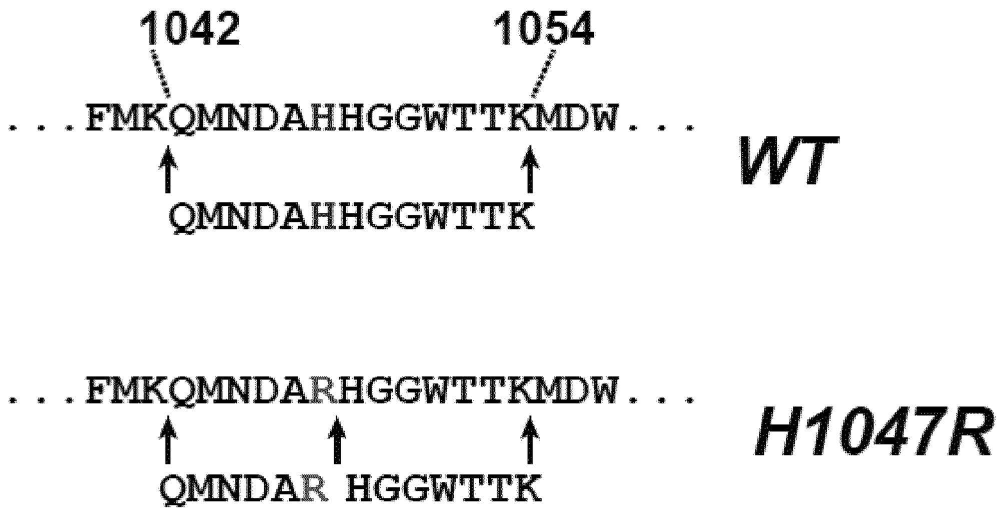


*FIG. 24A*

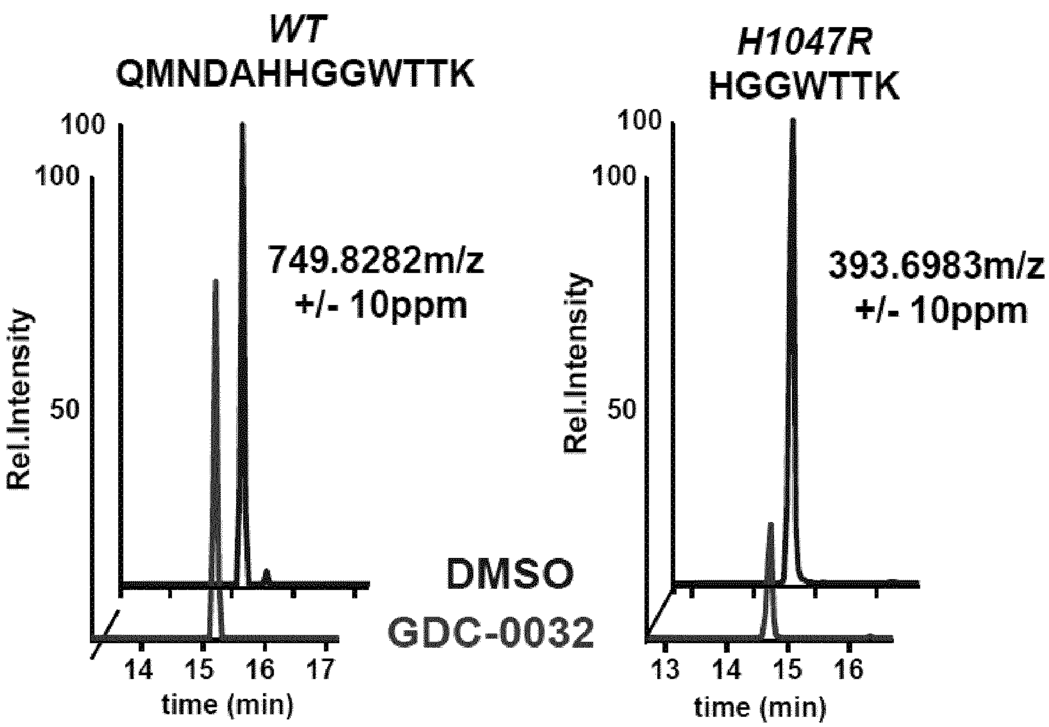


*FIG. 24B*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 62186236 A
- US 5824492 A
- US 5846824 A
- US 6274327 B
- US 20080207611 A
- US 7846929 B
- US 7781433 B
- US 20080076758 A
- US 7888352 B
- US 20080269210 A
- US 8324206 B
- US 8247397 B
- US 8604014 B
- US 8536161 B
- WO 2011036280 A
- US 8242104 B
- US 8343955 B
- US 8586574 B
- US 20140044706 A
- US 8227462 B
- US 8476268 B
- US 8710085 B
- US 4275149 A
- US 4318980 A
- US 4737456 A
- US 4016043 A
- US 4424279 A
- US 4018653 A
- US 3773919 A
- US 62186236 B


### Non-patent literature cited in the description


- **YUAN ; CANTLEY.** *Oncogene,* 2008, vol. 27, 5497-510
- **OSAKA et al.** *Apoptosis,* 2004, vol. 9, 667-76
- **SAMUELS et al.** *Science,* 2004, vol. 304, 554
- **HENNESSY et al.** *Nat. Rev. Drug Discov.,* 2005, vol. 4, 988-1004
- **ZHANG ; YU.** *Clin. Cancer Res.,* 2010, vol. 16, 4325-30
- **OPEL et al.** *Cancer Res.,* 2007, vol. 67, 735-45
- **RAZIS et al.** *Breast Cancer Res. Treat.,* 2011, vol. 128, 447-56
- **BACHMAN KE et al.** *Cancer Biol Ther.,* 2004, vol. 3, 772-775
- **STEMKE-HALE K et al.** *Cancer Res.,* 2008, vol. 68, 6084-6091
- **KOBOLDT DC et al.** *Nature,* 2012, vol. 490, 61-70
- **SAMUELS Y et al.** *Science,* 2004, vol. 304, 554
- **ARTHUR LM et al.** *Breast Cancer Res Treat.,* 2014, vol. 147, 211
- **ABUBAKER.** *Leukemia,* 2007, vol. 21, 2368-2370
- **RAMEH et al.** *J. Biol Chem.,* 1999, vol. 274, 8347-8350
- **PANAYOTOU et al.** *Trends Cell Biol,* 1992, vol. 2, 358-60
- **WHITMAN et al.** *Nature,* 1988, vol. 332, 664
- **VIVANCO et al.** *Nature Rev. Cancer,* 2002, vol. 2, 489
- **PHILLIPS et al.** *Cancer,* 1998, vol. 83, 41
- **WORKMAN P.** *Biochem Soc Trans,* 2004, vol. 32, 393-396
- **PATEL et al.** *Proc. Am. Assoc. of Cancer Res. (Abstract LB-247) 95th Annual Meeting,* 27 March 2004
- Phosphoinositide 3-Kinase: Function and Mechanisms. **AHMADI K ; WATERFIELD MD.** Encyclopedia of Biological Chemistry. Elsevier/Academic Press, 2004
- **STECK PA et al.** *Nat. Genet.,* 1997, vol. 15 (4), 356-62
- **CHU EC et al.** *Med. Sci. Monit.,* 2004, vol. 10 (10), RA235-41
- **OTSU et al.** *Cell,* 1991, vol. 65, 91-104
- **HILES et al.** *Cell,* 1992, vol. 70, 419-29
- **RAMEH et al.** *Cell,* 1995, vol. 83, 821-30
- **VOLINIA et al.** *Oncogene,* 1992, vol. 7, 789-93
- **MEYER et al.** *Cancer Res,* 2011, vol. 71 (13), 4344-51
- **CHAKRABARTY et al.** *Oncogene,* 2010, vol. 29 (37), 5193-5203
- **WALLIN et al.** *Mol. Can. Ther.,* 2011, vol. 10 (12), 2426-2436

- **SUTHERLIN et al.** *Jour. Med. Chem.,* 2011, vol. 54, 7579-7587
- **FOLKES et al.** *Jour. of Med. Chem.,* 2008, vol. 51 (18), 5522-5532
- **BELVIN et al.** *American Association for Cancer Research Annual Meeting,* 15 April 2008
- **FOLKES et al.** *American Association for Cancer Research Annual Meeting,* 14 April 2008
- **FRIEDMAN et al.** *American Association for Cancer Research Annual Meeting,* 14 April 2008
- **WALLIN et al.** *Clin. Cancer Res.,* 2012, vol. 18, 3901-3911
- **YUAN et al.** *Oncogene,* 2013, vol. 32, 318-326
- **O'BRIEN et al.** *Clin Cancer Res.,* 2010, vol. 16, 3670-3683
- **SALPHATI et al.** *Drug Metab. and Disp.,* 2010, vol. 38 (9), 1436-1442
- **EDGAR et al.** *Cancer Res.,* 2010, vol. 70, 1164-1172
- *CHEMICAL ABSTRACTS,* 1282512-48-4
- **NDUBAKU et al.** *Jour. Med. Chem.,* 2013, vol. 56 (11), 4597-4610
- **STABEN et al.** *Bioorg. Med. Chem. Lett.,* 2013, vol. 23, 2606-2613
- **KANG et al.** *Cell Cycle,* 2005, vol. 4 (4), 578-581
- **ZHAO ; VOGT.** *Oncogene,* 2008, vol. 27 (41), 5486-5496
- **BURKE et al.** *Proc. Natl. Acad. Sci.,* 2012, vol. 109 (38), 15259-15264
- **OLIVERO A et al.** *American Association for Cancer Research annual meeting,* 06 April 2013
- **WALLIN J et al.** *36th San Antonio Breast Cancer Symposium,* 10 December 2013
- **SAMPATH D et al.** *36th San Antonio Breast Cancer Symposium (SABCS),* 10 December 2013
- Handbook of Pharmaceutical Salts. Properties, Selection and Use. Wiley-VCH, 2002
- **S. BERGE et al.** *Journal of Pharmaceutical Sciences,* 1977, vol. 66 (1), 1-19
- **P. GOULD.** *International J. of Pharmaceutics,* 1986, vol. 33, 201-217
- **ANDERSON et al.** The Practice of Medicinal Chemistry. Academic Press, 1996
- Remington's Pharmaceutical Sciences. Mack Publishing Co, 1995
- **CHOU ; TALALAY.** *Adv. Enzyme Regul.,* 1984, vol. 22, 27-55
- **LEHÁR et al.** *Molecular Systems Biology,* 2007, vol. 3, 80
- **ENGVALL E ; PERLMAN P.** Enzyme-linked immunosorbent assay (ELISA). Quantitative assay of immunoglobulin G. *Immunochemistry,* 1971, vol. 8 (9), 871-4
- **VAN WEEMEN BK ; SCHUURS AH.** Immunoassay using antigen-enzyme conjugates. *FEBS Letters,* 1971, vol. 15 (3), 232-236
- **BARDELLI A et al.** *Science,* 2003, vol. 300 (5621), 949
- **CER RZ et al.** *Nucl. Acids Res.,* 2009, vol. 37, W441-W445
- **YU et al.** *Mol. Cell Bio.,* 1998, vol. 18, 1379-1387
- **WU et al.** *Proc. Natl. Acad. Sci.,* 2009, vol. 106 (48), 20258-20263
- **JADHAV, T. et al.** Defining an Embedded Code for Protein Ubiquitination. *J. Proteomics Bioinform,* 2009, vol. 2 (7), 316-333
- **WANG, G. et al.** K63-linked ubiquitination in kinase activation and cancer. *Frontiers in Oncology,* 2012, vol. 2 (5), 1-13
- **FURET, P. et al.** *Bioorg. Med. Chem. Lett.,* 2013, vol. 23, 3741-3748
- **OLIVERO ; JURIC.** *AACR,* 2013
- **BURKE et al.** *Proc. Natl. Acad. Sci.,* 2012, vol. 109, 15259-15264
- **ZHANG, X. et al.** *Mol. Cell,* 2011, vol. 41, 567-5789
- **HUANG, C.-H. et al.** *Science,* 2007, vol. 318, 1744
- **BERNDT, A. et al.** *Nat. Chem. Biol.,* 2010, vol. 6, 11
- **WALKER, E.H. et al.** Structural determinants of phosphoinositide 3-kinase inhibition by wortmannin, LY294002, quercetin, myricetin, and staurosporine. *Mol.Cell,* 2000, vol. 6, 909
- **SAMANTA, U. et al.** *Biol. Crystallogr.,* 1999, vol. D55, 1421
- *CHEMICAL ABSTRACTS,* 934660-93-2
- *CHEMICAL ABSTRACTS,* 1168091-68-6
- Remington's Pharmaceutical Sciences. Mack Publ. Co, 1995
- Pharmaceutical Dosage Forms. Marcel Decker, vol. 3
- **MENDOZA et al.** *Cancer Res.,* 2002, vol. 62, 5485-5488
- **PINHEIRO J et al.** nlme: linear and nonlinear mixed effects models. *R package version 3.1 92.,* 2009
- **TAN N et al.** Navitoclax enhances the efficacy of taxanes in non-small cell lung cancer models. *Clin. Cancer Res.,* 2011, vol. 17 (6), 1394-1404
- **POPKIE et al.** *J Biol Chem.,* 2010, vol. 285 (53), 41337-47
- **YOSHIOKA et al.** *Nat Med.,* October 2013, vol. 18 (10), 1560-9
- **BISWAS et al.** *J Biol Chem.,* 25 January 2013, vol. 288 (4), 2325-25
- **RAMADANI et al.** *Sci Signal,* 10 August 2010, vol. 3 (134